# EUROPEAN PATENT SPECIFICATION

(11) **EP 1 796 598 B1**
(45) Date of publication and mention of the grant of the patent: **13.01.2010**
(21) Application number: 05794197.3
(22) Date of filing: 31.08.2005
(51) Int. Cl.: A61F 2/30, A61L 27/34, A61L 27/54

(54) **A POLYMERIC WRAP FOR IN VIVO DELIVERY OF OSTEOINDUCTIVE FORMULATIONS**
POLYMERWICKEL FÜR DIE IN-VIVO-ABGABE VON OSTEOINDUKTIVEN FORMULIERUNGEN
EMBALLAGE POLYMERE POUR LA DELIVRANCE IN VIVO DE FORMULATIONS OSTEOINDUCTRICES

(30) Priority: 01.09.2004 US 931198
(43) Date of publication of application: 20.06.2007
(73) Proprietor: Warsaw Orthopedic, Inc., Warsaw, IN 46581 (US)
(72) Inventor: SERBOUSEK, Jon, C., Memphis, TN 38132 (US)
(74) Representative: O'Connell, Maura
(86) International application number: PCT/US2005/030976
(87) International publication number: WO 2006/028848

(56) References cited:
- WO-A-95/28973
- US-A- 5 947 893
- US-A1- 2003 045 942
- US-A1- 2004 158 329
- US-B1- 6 261 586
- US-B1- 6 648 916

### SEQUENCE LISTING

<110> Serbousek, Jon
<120> A Polymeric Wrap for In Vivo Delivery of Osteoinductive Formulations
<130> 64118.000083
<160> 84
<170> PatentIn version 3.2
<210> 1
   <211> 2487
   <212> DNA
   <213> Homo sapiens
<400> 1
   gccgcttccc tcgccgccgc cccgccagca tgcccggcgt ggcccgcctg ccgctgctgc     60
   tcgggctgct gctgctcccg cgtcccggcc ggccgctgga cttggccgac tacacctatg    120
   acctggcgga ggaggacgac tcggagcccc tcaactacaa agacccctgc aaggcggctg    180
   cctttcttgg ggacattgcc ctggacgaag aggacctgag ggccttccag gtacagcagg    240
   ctgtggatct cagacggcac acagctcgta agtcctccat caaagctgca gttccaggaa    300
   acacttctac ccccagctgc cagagcacca acgggcagcc tcagagggga gcctgtggga    360
   gatggagagg tagatcccgt agccggcggg cggcgacgtc ccgaccagag cgtgtgtggc    420
   ccgatggggt catccccttt gtcattgggg gaaacttcac tggtagccag agggcagtct    480
   tccggcaggc catgaggcac tgggagaagc acacctgtgt caccttcctg gagcgcactg    540
   acgaggacag ctatattgtg ttcacctatc gaccttgcgg gtgctgctcc tacgtgggtc    600
   gccgcggcgg gggcccccag gccatctcca tcggcaagaa ctgtgacaag ttcggcattg    660
   tggtccacga gctgggccac gtcgtcggct tctggcacga acacactcgg ccagaccggg    720
   accgccacgt ttccatcgtt cgtgagaaca tccagccagg gcaggagtat aacttcctga    780
   agatggagcc tcaggaggtg gagtccctgg gggagaccta tgacttcgac agcatcatgc    840
   attacgctcg gaacacattc tccaggggca tcttcctgga taccattgtc cccaagtatg    900
   aggtgaacgg ggtgaaacct cccattggcc aaaggacacg gctcagcaag ggggacattg    960
   cccaagcccg caagctttac aagtgcccag cctgtggaga gaccctgcaa gacagcacag   1020
   gcaacttctc ctcccctgaa taccccaatg gctactctgc tcacatgcac tgcgtgtggc   1080
   gcatctctgt cacacccggg gagaagatca tcctgaactt cacgtccctg gacctgtacc    1140
   gcagccgcct gtgctggtac gactatgtgg aggtccgaga tggcttctgg aggaaggcgc    1200
   ccctccgagg ccgcttctgc gggtccaaac tccctgagcc tatcgtctcc actgacagcc    1260
   gcctctgggt tgaattccgc agcagcagca attgggttgg aaagggcttc tttgcagtct    1320
   acgaagccat ctgcgggggt gatgtgaaaa aggactatgg ccacattcaa tcgcccaact    1380
   acccagacga ttaccggccc agcaaagtct gcatctggcg gatccaggtg tctgagggct    1440
   tccacgtggg cctcacattc cagtcctttg agattgagcg ccacgacagc tgtgcctacg    1500
   actatctgga ggtgcgcgac gggcacagtg agagcagcac cctcatcggg cgctactgtg    1560
   gctatgagaa gcctgatgac atcaagagca cgtccagccg cctctggctc aagttcgtct    1620
   ctgacgggtc cattaacaaa gcgggctttg ccgtcaactt tttcaaagag gtggacgagt    1680
   gctctcggcc caaccgcggg ggctgtgagc agcggtgcct caacaccctg ggcagctaca    1740
   agtgcagctg tgaccccggg tacgagctgg ccccagacaa gcgccgctgt gaggctgctt    1800
   gtggcggatt cctcaccaag ctcaacggct ccatcaccag cccgggctgg cccaaggagt    1860
   acccccccaa caagaactgc atctggcagc tggtggcccc cacccagtac cgcatctccc    1920
   tgcagtttga cttctttgag acagagggca atgatgtgtg caagtacgac ttcgtggagg    1980
   tgcgcagtgg actcacagct gactccaagc tgcatggcaa gttctgtggt tctgagaagc    2040
   ccgaggtcat cacctcccag tacaacaaca tgcgcgtgga gttcaagtcc gacaacaccg    2100
   tgtccaaaaa gggcttcaag gcccacttct tctcagaaaa gaggccagct ctgcagcccc    2160
   ctcggggacg cccccaccag ctcaaattcc gagtgcagaa aagaaaccgg accccccagt    2220
   gaggcctgcc aggcctcccg gaccccttgt tactcaggaa cctcaccttg gacggaatgg    2280
   gatgggggct tcggtgccca ccaacccccc acctccactc tgccattccg gcccacctcc    2340
   ctctggccgg acagaactgg tgctctcttc tccccactgt gcccgtccgc ggaccgggga    2400
   cccttccccg tgccctaccc cctcccattt tgatggtgtc tgtgacattt cctgttgtga    2460
   agtaaaagag ggacccctgc gtcctgc                                        2487
<210> 2
   <211> 730
   <212> PRT
   <213> Homo sapiens
<400> 2
   Met Pro Gly Val Ala Arg Leu Pro Leu Leu Leu Gly Leu Leu Leu Leu
   1 5 10 15
   Pro Arg Pro Gly Arg Pro Leu Asp Leu Ala Asp Tyr Thr Tyr Asp Leu
   20 25 30
   Ala Glu Glu Asp Asp Ser Glu Pro Leu Asn Tyr Lys Asp Pro Cys Lys
   35 40 45
   Ala Ala Ala Phe Leu Gly Asp Ile Ala Leu Asp Glu Glu Asp Leu Arg
   50 55 60
   Ala Phe Gln Val Gln Gln Ala Val Asp Leu Arg Arg His Thr Ala Arg
   65 70 75 80
   Lys Ser Ser Ile Lys Ala Ala Val Pro Gly Asn Thr Ser Thr Pro Ser
   85 90 95
   Cys Gln Ser Thr Asn Gly Gln Pro Gln Arg Gly Ala Cys Gly Arg Trp
   100 105 110
   Arg Gly Arg Ser Arg Ser Arg Arg Ala Ala Thr Ser Arg Pro Glu Arg
   115 120 125
   Val Trp Pro Asp Gly Val Ile Pro Phe Val Ile Gly Gly Asn Phe Thr
   130 135 140
   Gly Ser Gln Arg Ala Val Phe Arg Gln Ala Met Arg His Trp Glu Lys
   145 150 155 160
   His Thr Cys Val Thr Phe Leu Glu Arg Thr Asp Glu Asp Ser Tyr Ile
   165 170 175
   Val Phe Thr Tyr Arg Pro Cys Gly Cys Cys Ser Tyr Val Gly Arg Arg
   180 185 190
   Gly Gly Gly Pro Gln Ala Ile Ser Ile Gly Lys Asn Cys Asp Lys Phe
   195 200 205
   Gly Ile Val Val His Glu Leu Gly His Val Val Gly Phe Trp His Glu
   210 215 220
   His Thr Arg Pro Asp Arg Asp Arg His Val Ser Ile Val Arg Glu Asn
   225 230 235 240
   Ile Gln Pro Gly Gln Glu Tyr Asn Phe Leu Lys Met Glu Pro Gln Glu
   245 250 255
   Val Glu Ser Leu Gly Glu Thr Tyr Asp Phe Asp Ser Ile Met His Tyr
   260 265 270
   Ala Arg Asn Thr Phe Ser Arg Gly Ile Phe Leu Asp Thr Ile Val Pro
   275 280 285
   Lys Tyr Glu Val Asn Gly Val Lys Pro Pro Ile Gly Gln Arg Thr Arg
   290 295 300
   Leu Ser Lys Gly Asp Ile Ala Gln Ala Arg Lys Leu Tyr Lys Cys Pro
   305 310 315 320
   Ala Cys Gly Glu Thr Leu Gln Asp Ser Thr Gly Asn Phe Ser Ser Pro
   325 330 335
   Glu Tyr Pro Asn Gly Tyr Ser Ala His Met His Cys Val Trp Arg Ile
   340 345 350
   Ser Val Thr Pro Gly Glu Lys Ile Ile Leu Asn Phe Thr Ser Leu Asp
   355 360 365
   Leu Tyr Arg Ser Arg Leu Cys Trp Tyr Asp Tyr Val Glu Val Arg Asp
   370 375 380
   Gly Phe Trp Arg Lys Ala Pro Leu Arg Gly Arg Phe Cys Gly Ser Lys
   385 390 395 400
   Leu Pro Glu Pro Ile Val Ser Thr Asp Ser Arg Leu Trp Val Glu Phe
   405 410 415
   Arg Ser Ser Ser Asn Trp Val Gly Lys Gly Phe Phe Ala Val Tyr Glu
   420 425 430
   Ala Ile Cys Gly Gly Asp Val Lys Lys Asp Tyr Gly His Ile Gln Ser
           435                 440                 445
   Pro Asn Tyr Pro Asp Asp Tyr Arg Pro Ser Lys Val Cys Ile Trp Arg
       450                 455                 460
   Ile Gln Val Ser Glu Gly Phe His Val Gly Leu Thr Phe Gln Ser Phe
   465                 470                 475                 480
   Glu Ile Glu Arg His Asp Ser Cys Ala Tyr Asp Tyr Leu Glu Val Arg
                   485                 490                 495
   Asp Gly His Ser Glu Ser Ser Thr Leu Ile Gly Arg Tyr Cys Gly Tyr
               500                 505                 510
   Glu Lys Pro Asp Asp Ile Lys Ser Thr Ser Ser Arg Leu Trp Leu Lys
           515                 520                 525
   Phe Val Ser Asp Gly Ser Ile Asn Lys Ala Gly Phe Ala Val Asn Phe
       530                 535                 540
   Phe Lys Glu Val Asp Glu Cys Ser Arg Pro Asn Arg Gly Gly Cys Glu
   545                 550                 555                 560
   Gln Arg Cys Leu Asn Thr Leu Gly Ser Tyr Lys Cys Ser Cys Asp Pro
                   565                 570                 575
   Gly Tyr Glu Leu Ala Pro Asp Lys Arg Arg Cys Glu Ala Ala Cys Gly
               580                 585                 590
   Gly Phe Leu Thr Lys Leu Asn Gly Ser Ile Thr Ser Pro Gly Trp Pro
           595                 600                 605
   Lys Glu Tyr Pro Pro Asn Lys Asn Cys Ile Trp Gln Leu Val Ala Pro
       610                 615                 620
   Thr Gln Tyr Arg Ile Ser Leu Gln Phe Asp Phe Phe Glu Thr Glu Gly
   625                 630                 635                 640
   Asn Asp Val Cys Lys Tyr Asp Phe Val Glu Val Arg Ser Gly Leu Thr
                   645                 650                 655
   Ala Asp Ser Lys Leu His Gly Lys Phe Cys Gly Ser Glu Lys Pro Glu
   660 665 670
   Val Ile Thr Ser Gln Tyr Asn Asn Met Arg Val Glu Phe Lys Ser Asp
   675 680 685
   Asn Thr Val Ser Lys Lys Gly Phe Lys Ala His Phe Phe Ser Glu Lys
   690 695 700
   Arg Pro Ala Leu Gln Pro Pro Arg Gly Arg Pro His Gln Leu Lys Phe
   705 710 715 720
   Arg Val Gln Lys Arg Asn Arg Thr Pro Gln
   725 730
<210> 3
   <211> 1821
   <212> DNA
   <213> Homo sapiens
<400> 3
   tcccgaccag agcgtgtgtg gcccgatggg gtcatcccct ttgtcattgg gggaaacttc 60
   actggtagcc agagggcagt cttccggcag gccatgaggc actgggagaa gcacacctgt 120
   gtcaccttcc tggagcgcac tgacgaggac agctatattg tgttcaccta tcgaccttgc 180
   gggtgctgct cctacgtggg tcgccgcggc gggggccccc aggccatctc catcggcaag 240
   aactgtgaca agttcggcat tgtggtccac gagctgggcc acgtcgtcgg cttctggcac 300
   gaacacactc ggccagaccg ggaccgccac gtttccatcg ttcgtgagaa catccagcca 360
   gggcaggagt ataacttcct gaagatggag cctcaggagg tggagtccct gggggagacc 420
   tatgacttcg acagcatcat gcattacgct cggaacacat tctccagggg catcttcctg 480
   gataccattg tccccaagta tgaggtgaac ggggtgaaac ctcccattgg ccaaaggaca 540
   cggctcagca agggggacat tgcccaagcc cgcaagcttt acaagtgccc agcctgtgga 600
   gagaccctgc aagacagcac aggcaacttc tcctcccctg aataccccaa tggctactct 660
   gctcacatgc actgcgtgtg gcgcatctct gtcacacccg gggagaagat catcctgaac 720
   ttcacgtccc tggacctgta ccgcagccgc ctgtgctggt acgactatgt ggaggtccga 780
   gatggcttct ggaggaaggc gcccctccga ggccgcttct gcgggtccaa actccctgag 840
   cctatcgtct ccactgacag ccgcctctgg gttgaattcc gcagcagcag caattgggtt 900
   ggaaagggct tctttgcagt ctacgaagcc atctgcgggg gtgatgtgaa aaaggactat 960
   ggccacattc aatcgcccaa ctacccagac gattaccggc ccagcaaagt ctgcatctgg 1020
   cggatccagg tgtctgaggg cttccacgtg ggcctcacat tccagtcctt tgagattgag 1080
   cgccacgaca gctgtgccta cgactatctg gaggtgcgcg acgggcacag tgagagcagc 1140
   accctcatcg ggcgctactg tggctatgag aagcctgatg acatcaagag cacgtccagc 1200
   cgcctctggc tcaagttcgt ctctgacggg tccattaaca aagcgggctt tgccgtcaac 1260
   tttttcaaag aggtggacga gtgctctcgg cccaaccgcg ggggctgtga gcagcggtgc 1320
   ctcaacaccc tgggcagcta caagtgcagc tgtgaccccg ggtacgagct ggccccagac 1380
   aagcgccgct gtgaggctgc ttgtggcgga ttcctcacca agctcaacgg ctccatcacc 1440
   agcccgggct ggcccaagga gtaccccccc aacaagaact gcatctggca gctggtggcc 1500
   cccacccagt accgcatctc cctgcagttt gacttctttg agacagaggg caatgatgtg 1560
   tgcaagtacg acttcgtgga ggtgcgcagt ggactcacag ctgactccaa gctgcatggc 1620
   aagttctgtg gttctgagaa gcccgaggtc atcacctccc agtacaacaa catgcgcgtg 1680
   gagttcaagt ccgacaacac cgtgtccaaa aagggcttca aggcccactt cttctcagaa 1740
   aagaggccag ctctgcagcc ccctcgggga cgcccccacc agctcaaatt ccgagtgcag 1800
   aaaagaaacc ggacccccca g 1821
<210> 4
   <211> 610
   <212> PRT
   <213> Homo sapiens
<400> 4
   Ala Ala Thr Ser Arg Pro Glu Arg Val Trp Pro Asp Gly Val Ile Pro
   1 5 10 15
   Phe Val Ile Gly Gly Asn Phe Thr Gly Ser Gln Arg Ala Val Phe Arg
   20 25 30
   Gln Ala Met Arg His Trp Glu Lys His Thr Cys Val Thr Phe Leu Glu
   35 40 45
   Arg Thr Asp Glu Asp Ser Tyr Ile Val Phe Thr Tyr Arg Pro Cys Gly
               50                  55                  60
   Cys Cys Ser Tyr Val Gly Arg Arg Gly Gly Gly Pro Gln Ala Ile Ser
   65                  70                  75                  80
   Ile Gly Lys Asn Cys Asp Lys Phe Gly Ile Val Val His Glu Leu Gly
                   85                  90                  95
   His Val Val Gly Phe Trp His Glu His Thr Arg Pro Asp Arg Asp Arg
               100                 105                 110
   His Val Ser Ile Val Arg Glu Asn Ile Gln Pro Gly Gln Glu Tyr Asn
           115                 120                 125
   Phe Leu Lys Met Glu Pro Gln Glu Val Glu Ser Leu Gly Glu Thr Tyr
       130                 135                 140
   Asp Phe Asp Ser Ile Met His Tyr Ala Arg Asn Thr Phe Ser Arg Gly
   145                 150                 155                 160
   Ile Phe Leu Asp Thr Ile Val Pro Lys Tyr Glu Val Asn Gly Val Lys
                   165                 170                 175
   Pro Pro Ile Gly Gln Arg Thr Arg Leu Ser Lys Gly Asp Ile Ala Gln
               180                 185                 190
   Ala Arg Lys Leu Tyr Lys Cys Pro Ala Cys Gly Glu Thr Leu Gln Asp
           195                 200                 205
   Ser Thr Gly Asn Phe Ser Ser Pro Glu Tyr Pro Asn Gly Tyr Ser Ala
       210                 215                 220
   His Met His Cys Val Trp Arg Ile Ser Val Thr Pro Gly Glu Lys Ile
   225                 230                 235                 240
   Ile Leu Asn Phe Thr Ser Leu Asp Leu Tyr Arg Ser Arg Leu Cys Trp
                   245                 250                 255
   Tyr Asp Tyr Val Glu Val Arg Asp Gly Phe Trp Arg Lys Ala Pro Leu
               260                 265                 270
   Arg Gly Arg Phe Cys Gly Ser Lys Leu Pro Glu Pro Ile Val Ser Thr
   275 280 285
   Asp Ser Arg Leu Trp Val Glu Phe Arg Ser Ser Ser Asn Trp Val Gly
   290 295 300
   Lys Gly Phe Phe Ala Val Tyr Glu Ala Ile Cys Gly Gly Asp Val Lys
   305 310 315 320
   Lys Asp Tyr Gly His Ile Gln Ser Pro Asn Tyr Pro Asp Asp Tyr Arg
   325 330 335
   Pro Ser Lys Val Cys Ile Trp Arg Ile Gln Val Ser Glu Gly Phe His
   340 345 350
   Val Gly Leu Thr Phe Gln Ser Phe Glu Ile Glu Arg His Asp Ser Cys
   355 360 365
   Ala Tyr Asp Tyr Leu Glu Val Arg Asp Gly His Ser Glu Ser Ser Thr
   370 375 380
   Leu Ile Gly Arg Tyr Cys Gly Tyr Glu Lys Pro Asp Asp Ile Lys Ser
   385 390 395 400
   Thr Ser Ser Arg Leu Trp Leu Lys Phe Val Ser Asp Gly Ser Ile Asn
   405 410 415
   Lys Ala Gly Phe Ala Val Asn Phe Phe Lys Glu Val Asp Glu Cys Ser
   420 425 430
   Arg Pro Asn Arg Gly Gly Cys Glu Gln Arg Cys Leu Asn Thr Leu Gly
   435 440 445
   Ser Tyr Lys Cys Ser Cys Asp Pro Gly Tyr Glu Leu Ala Pro Asp Lys
   450 455 460
   Arg Arg Cys Glu Ala Ala Cys Gly Gly Phe Leu Thr Lys Leu Asn Gly
   465 470 475 480
   Ser Ile Thr Ser Pro Gly Trp Pro Lys Glu Tyr Pro Pro Asn Lys Asn
   485 490 495
   Cys Ile Trp Gln Leu Val Ala Pro Thr Gln Tyr Arg Ile Ser Leu Gln
   500 505 510
   Phe Asp Phe Phe Glu Thr Glu Gly Asn Asp Val Cys Lys Tyr Asp Phe
   515 520 525
   Val Glu Val Arg Ser Gly Leu Thr Ala Asp Ser Lys Leu His Gly Lys
   530 535 540
   Phe Cys Gly Ser Glu Lys Pro Glu Val Ile Thr Ser Gln Tyr Asn Asn
   545 550 555 560
   Met Arg Val Glu Phe Lys Ser Asp Asn Thr Val Ser Lys Lys Gly Phe
   565 570 575
   Lys Ala His Phe Phe Ser Glu Lys Arg Pro Ala Leu Gln Pro Pro Arg
   580 585 590
   Gly Arg Pro His Gln Leu Lys Phe Arg Val Gln Lys Arg Asn Arg Thr
   595 600 605
   Pro Gln
   610
<210> 5
   <211> 1547
   <212> DNA
   <213> Homo sapiens
<400> 5
   ggggacttct tgaacttgca gggagaataa cttgcgcacc ccactttgcg ccggtgcctt 60
   tgccccagcg gagcctgctt cgccatctcc gagccccacc gcccctccac tcctcggcct 120
   tgcccgacac tgagacgctg ttcccagcgt gaaaagagag actgcgcggc cggcacccgg 180
   gagaaggagg aggcaaagaa aaggaacgga cattcggtcc ttgcgccagg tcctttgacc 240
   agagtttttc catgtggacg ctctttcaat ggacgtgtcc ccgcgtgctt cttagacgga 300
   ctgcggtctc ctaaaggtcg accatggtgg ccgggacccg ctgtcttcta gcgttgctgc 360
   ttccccaggt cctcctgggc ggcgcggctg gcctcgttcc ggagctgggc cgcaggaagt 420
   tcgcggcggc gtcgtcgggc cgcccctcat cccagccctc tgacgaggtc ctgagcgagt 480
   tcgagttgcg gctgctcagc atgttcggcc tgaaacagag acccaccccc agcagggacg     540
   ccgtggtgcc cccctacatg ctagacctgt atcgcaggca ctcaggtcag ccgggctcac     600
   ccgccccaga ccaccggttg gagagggcag ccagccgagc caacactgtg cgcagcttcc     660
   accatgaaga atctttggaa gaactaccag aaacgagtgg gaaaacaacc cggagattct     720
   tctttaattt aagttctatc cccacggagg agtttatcac ctcagcagag cttcaggttt     780
   tccgagaaca gatgcaagat gctttaggaa acaatagcag tttccatcac cgaattaata     840
   tttatgaaat cataaaacct gcaacagcca actcgaaatt ccccgtgacc agacttttgg     900
   acaccaggtt ggtgaatcag aatgcaagca ggtgggaaag ttttgatgtc acccccgctg     960
   tgatgcggtg gactgcacag ggacacgcca accatggatt cgtggtggaa gtggcccact    1020
   tggaggagaa acaaggtgtc tccaagagac atgttaggat aagcaggtct ttgcaccaag    1080
   atgaacacag ctggtcacag ataaggccat tgctagtaac ttttggccat gatggaaaag    1140
   ggcatcctct ccacaaaaga gaaaaacgtc aagccaaaca caaacagcgg aaacgcctta    1200
   agtccagctg taagagacac cctttgtacg tggacttcag tgacgtgggg tggaatgact    1260
   ggattgtggc tcccccgggg tatcacgcct tttactgcca cggagaatgc ccttttcctc    1320
   tggctgatca tctgaactcc actaatcatg ccattgttca gacgttggtc aactctgtta    1380
   actctaagat tcctaaggca tgctgtgtcc cgacagaact cagtgctatc tcgatgctgt    1440
   accttgacga gaatgaaaag gttgtattaa agaactatca ggacatggtt gtggagggtt    1500
   gtgggtgtcg ctagtacagc aaaattaaat acataaatat atatata                  1547
<210> 6
   <211> 396
   <212> PRT
   <213> Homo sapiens
<400> 6
   Met Val Ala Gly Thr Arg Cys Leu Leu Ala Leu Leu Leu Pro Gln Val
   1               5                   10                  15
   Leu Leu Gly Gly Ala Ala Gly Leu Val Pro Glu Leu Gly Arg Arg Lys
               20                  25                  30
   Phe Ala Ala Ala Ser Ser Gly Arg Pro Ser Ser Gln Pro Ser Asp Glu
           35                  40                  45
   Val Leu Ser Glu Phe Glu Leu Arg Leu Leu Ser Met Phe Gly Leu Lys
   50 55 60
   Gln Arg Pro Thr Pro Ser Arg Asp Ala Val Val Pro Pro Tyr Met Leu
   65 70 75 80
   Asp Leu Tyr Arg Arg His Ser Gly Gln Pro Gly Ser Pro Ala Pro Asp
   85 90 95
   His Arg Leu Glu Arg Ala Ala Ser Arg Ala Asn Thr Val Arg Ser Phe
   100 105 110
   His His Glu Glu Ser Leu Glu Glu Leu Pro Glu Thr Ser Gly Lys Thr
   115 120 125
   Thr Arg Arg Phe Phe Phe Asn Leu Ser Ser Ile Pro Thr Glu Glu Phe
   130 135 140
   Ile Thr Ser Ala Glu Leu Gln Val Phe Arg Glu Gln Met Gln Asp Ala
   145 150 155 160
   Leu Gly Asn Asn Ser Ser Phe His His Arg Ile Asn Ile Tyr Glu Ile
   165 170 175
   Ile Lys Pro Ala Thr Ala Asn Ser Lys Phe Pro Val Thr Arg Leu Leu
   180 185 190
   Asp Thr Arg Leu Val Asn Gln Asn Ala Ser Arg Trp Glu Ser Phe Asp
   195 200 205
   Val Thr Pro Ala Val Met Arg Trp Thr Ala Gln Gly His Ala Asn His
   210 215 220
   Gly Phe Val Val Glu Val Ala His Leu Glu Glu Lys Gln Gly Val Ser
   225 230 235 240
   Lys Arg His Val Arg Ile Ser Arg Ser Leu His Gln Asp Glu His Ser
   245 250 255
   Trp Ser Gln Ile Arg Pro Leu Leu Val Thr Phe Gly His Asp Gly Lys
   260 265 270
   Gly His Pro Leu His Lys Arg Glu Lys Arg Gln Ala Lys His Lys Gln
   275 280 285
   Arg Lys Arg Leu Lys Ser Ser Cys Lys Arg His Pro Leu Tyr Val Asp
   290 295 300
   Phe Ser Asp Val Gly Trp Asn Asp Trp Ile Val Ala Pro Pro Gly Tyr
   305 310 315 320
   His Ala Phe Tyr Cys His Gly Glu Cys Pro Phe Pro Leu Ala Asp His
   325 330 335
   Leu Asn Ser Thr Asn His Ala Ile Val Gln Thr Leu Val Asn Ser Val
   340 345 350
   Asn Ser Lys Ile Pro Lys Ala Cys Cys Val Pro Thr Glu Leu Ser Ala
   355 360 365
   Ile Ser Met Leu Tyr Leu Asp Glu Asn Glu Lys Val Val Leu Lys Asn
   370 375 380
   Tyr Gln Asp Met Val Val Glu Gly Cys Gly Cys Arg
   385 390 395
<210> 7
   <211> 1794
   <212> DNA
   <213> Homo sapiens
<400> 7
   agatcttgaa aacacccggg ccacacacgc cgcgacccac agctctttct cagcgttgga 60
   gtggagacgg cgcccgcagc gccctgcgcg ggtgaggtcc gcgcagctgc tggggaagag 120
   cccacctgtc aggctgcgct gggtcagcgc agcaagtggg gctggccgct atctcgctgc 180
   acccggccgc gtcccgggct ccgtgcgccc tcgccccagc tggtttggag ttcaaccctc 240
   ggctccgccg ccggctcctt gcgccttcgg agtgtcccgc agcgacgccg ggagccgacg 300
   cgccgcgcgg gtacctagcc atggctgggg cgagcaggct gctgtttctg tggctgggct 360
   gcttctgcgt gagcctggcg cagggagaga gaccgaagcc acctttcccg gagctccgca 420
   aagctgtgcc aggtgaccgc acggcaggtg ctggcccgga ctccgagctg gagccgcaag 480
   acaaggtctc tgaacacatg ctgcggctct atgacaggta cagcacggtc caggcggccc     540
   ggacaccggg ctccctggag ggaggctcgg agccctggcg ccctcggctc ctgcgcgaag     600
   gcaacacggt tcgcagcttt cgggcggcag cagcagaaac tcttgaaaga aaaggactgt     660
   atatcttcaa tctgacatcg ctaaccaagt ctgaaaacat tttgtctgcc acactgtatt     720
   tctgtattgg agagctagga aacatcagcc tgagttgtcc agtgtctgga ggatgctccc     780
   atcatgctca gaggaaacac attcagattg atctttctgc atggaccctc aaattcagca     840
   gaaaccaaag tcaactcctt ggccatctgt cagtggatat ggccaaatct catcgagata     900
   ttatgtcctg gctgtctaaa gatatcactc aattcttgag gaaggccaaa gaaaaagaag     960
   agttcctcat aggatttaac attacgtcca agggacgcca gctgccaaag aggaggttac    1020
   cttttccaga gccttatatc ttggtatatg ccaatgatgc cgccatttct gagccagaaa    1080
   gtgtggtatc aagcttacag ggacaccgga attttcccac tggaactgtt cccaaatggg    1140
   atagccacat cagagctgcc ctttccattg agcggaggaa gaagcgctct actgggctct    1200
   tgctgcctct gcagaacaac gagcttcctg gggcagaata ccagtataaa aaggatgagg    1260
   tgtgggagga gagaaagcct tacaagaccc ttcaggctca ggcccctgaa aagagtaaga    1320
   ataaaaagaa acagagaaag gggcctcatc ggaagagcca gaggctccaa tttgatgagc    1380
   agaccctgaa aaaggcaagg agaaagcagt ggattgaacc tcggaattgc gccaggagat    1440
   acctcaaggt agactttgca gatattggct ggagtgaatg gattatctcc cccaagtcct    1500
   ttgatgccta ttattgctct ggagcatgcc agttccccat gccaaagtct ttgaagccat    1560
   caaatcatgc taccatccag agtatagtga gagctgtggg ggtcgttcct gggattcctg    1620
   agccttgctg tgtaccagaa aagatgtcct cactcagtat tttattcttt gatgaaaata    1680
   agaatgtagt gcttaaagta taccctaaca tgacagtaga gtcttgcgct tgcagataac    1740
   ctggcaaaga actcatttga atgcttaatt caatctctag agtcgacgga attc          1794
<210> 8
   <211> 472
   <212> PRT
   <213> Homo sapiens
<400> 8
   Met Ala Gly Ala Ser Arg Leu Leu Phe Leu Trp Leu Gly Cys Phe Cys
   1               5                   10                  15
   Val Ser Leu Ala Gln Gly Glu Arg Pro Lys Pro Pro Phe Pro Glu Leu
   20 25 30
   Arg Lys Ala Val Pro Gly Asp Arg Thr Ala Gly Ala Gly Pro Asp Ser
   35 40 45
   Glu Leu Glu Pro Gln Asp Lys Val Ser Glu His Met Leu Arg Leu Tyr
   50 55 60
   Asp Arg Tyr Ser Thr Val Gln Ala Ala Arg Thr Pro Gly Ser Leu Glu
   65 70 75 80
   Gly Gly Ser Glu Pro Trp Arg Pro Arg Leu Leu Arg Glu Gly Asn Thr
   85 90 95
   Val Arg Ser Phe Arg Ala Ala Ala Ala Glu Thr Leu Glu Arg Lys Gly
   100 105 110
   Leu Tyr Ile Phe Asn Leu Thr Ser Leu Thr Lys Ser Glu Asn Ile Leu
   115 120 125
   Ser Ala Thr Leu Tyr Phe Cys Ile Gly Glu Leu Gly Asn Ile Ser Leu
   130 135 140
   Ser Cys Pro Val Ser Gly Gly Cys Ser His His Ala Gln Arg Lys His
   145 150 155 160
   Ile Gln Ile Asp Leu Ser Ala Trp Thr Leu Lys Phe Ser Arg Asn Gln
   165 170 175
   Ser Gln Leu Leu Gly His Leu Ser Val Asp Met Ala Lys Ser His Arg
   180 185 190
   Asp Ile Met Ser Trp Leu Ser Lys Asp Ile Thr Gln Phe Leu Arg Lys
   195 200 205
   Ala Lys Glu Lys Glu Glu Phe Leu Ile Gly Phe Asn Ile Thr Ser Lys
   210 215 220
   Gly Arg Gln Leu Pro Lys Arg Arg Leu Pro Phe Pro Glu Pro Tyr Ile
   225                 230                 235                 240
   Leu Val Tyr Ala Asn Asp Ala Ala Ile Ser Glu Pro Glu Ser Val Val
                   245                 250                 255
   Ser Ser Leu Gln Gly His Arg Asn Phe Pro Thr Gly Thr Val Pro Lys
               260                 265                 270
   Trp Asp Ser His Ile Arg Ala Ala Leu Ser Ile Glu Arg Arg Lys Lys
           275                 280                 285
   Arg Ser Thr Gly Leu Leu Leu Pro Leu Gln Asn Asn Glu Leu Pro Gly
       290                 295                 300
   Ala Glu Tyr Gln Tyr Lys Lys Asp Glu Val Trp Glu Glu Arg Lys Pro
   305                 310                 315                 320
   Tyr Lys Thr Leu Gln Ala Gln Ala Pro Glu Lys Ser Lys Asn Lys Lys
                   325                 330                 335
   Lys Gln Arg Lys Gly Pro His Arg Lys Ser Gln Arg Leu Gln Phe Asp
               340                 345                 350
   Glu Gln Thr Leu Lys Lys Ala Arg Arg Lys Gln Trp Ile Glu Pro Arg
           355                 360                 365
   Asn Cys Ala Arg Arg Tyr Leu Lys Val Asp Phe Ala Asp Ile Gly Trp
       370                 375                 380
   Ser Glu Trp Ile Ile Ser Pro Lys Ser Phe Asp Ala Tyr Tyr Cys Ser
   385                 390                 395                 400
   Gly Ala Cys Gln Phe Pro Met Pro Lys Ser Leu Lys Pro Ser Asn His
                   405                 410                 415
   Ala Thr Ile Gln Ser Ile Val Arg Ala Val Gly Val Val Pro Gly Ile
               420                 425                 430
   Pro Glu Pro Cys Cys Val Pro Glu Lys Met Ser Ser Leu Ser Ile Leu
           435                 440                 445
   Phe Phe Asp Glu Asn Lys Asn Val Val Leu Lys Val Tyr Pro Asn Met
   450 455 460
   Thr Val Glu Ser Cys Ala Cys Arg
   465 470
<210> 9
   <211> 1999
   <212> DNA
   <213> Homo sapiens
<400> 9
   gagggagggg ccgccgggga agaggaggag gaaggaaaga aagaaagcga gggagggaaa 60
   gaggaggaag gaagatgcga gaaggcagag gaggagggag ggagggaagg agcgcggagc 120
   ccggcccgga agctaggtga gtgtggcatc cgagctgagg gacgcgagcc tgagacgccg 180
   ctgctgctcc ggctgagtat ctagcttgtc tccccgatgg gattcccgtc caagctatct 240
   cgagcctgca gcgccacagt ccccggccct cgcccaggtt cactgcaacc gttcagaggt 300
   ccccaggagc tgctgctggc gagcccgcta ctgcagggac ctatggagcc attccgtagt 360
   gccatcccga gcaacgcact gctgcagctt ccctgagcct ttccagcaag tttgttcaag 420
   attggctgtc aagaatcatg gactgttatt atatgccttg ttttctgtca agacaccatg 480
   attcctggta accgaatgct gatggtcgtt ttattatgcc aagtcctgct aggaggcgcg 540
   agccatgcta gtttgatacc tgagacgggg aagaaaaaag tcgccgagat tcagggccac 600
   gcgggaggac gccgctcagg gcagagccat gagctcctgc gggacttcga ggcgacactt 660
   ctgcagatgt ttgggctgcg ccgccgcccg cagcctagca agagtgccgt cattccggac 720
   tacatgcggg atctttaccg gcttcagtct ggggaggagg aggaagagca gatccacagc 780
   actggtcttg agtatcctga gcgcccggcc agccgggcca acaccgtgag gagcttccac 840
   cacgaagaac atctggagaa catcccaggg accagtgaaa actctgcttt tcgtttcctc 900
   tttaacctca gcagcatccc tgagaacgag gcgatctcct ctgcagagct tcggctcttc 960
   cgggagcagg tggaccaggg ccctgattgg gaaaggggct tccaccgtat aaacatttat 1020
   gaggttatga agcccccagc agaagtggtg cctgggcacc tcatcacacg actactggac 1080
   acgagactgg tccaccacaa tgtgacacgg tgggaaactt ttgatgtgag ccctgcggtc 1140
   cttcgctgga cccgggagaa gcagccaaac tatgggctag ccattgaggt gactcacctc 1200
   catcagactc ggacccacca gggccagcat gtcaggatta gccgatcgtt acctcaaggg 1260
   agtgggaatt gggcccagct ccggcccctc ctggtcacct ttggccatga tggccggggc    1320
   catgccttga cccgacgccg gagggccaag cgtagcccta agcatcactc acagcgggcc    1380
   aggaagaaga ataagaactg ccggcgccac tcgctctatg tggacttcag cgatgtgggc    1440
   tggaatgact ggattgtggc cccaccaggc taccaggcct tctactgcca tggggactgc    1500
   ccctttccac tggctgacca cctcaactca accaaccatg ccattgtgca gaccctggtc    1560
   aattctgtca attccagtat ccccaaagcc tgttgtgtgc ccactgaact gagtgccatc    1620
   tccatgctgt acctggatga gtatgataag gtggtactga aaaattatca ggagatggta    1680
   gtagagggat gtgggtgccg ctgagatcag gcagtccttg aggatagaca gatatacaca    1740
   ccacacacac acaccacata caccacacac acacgttccc atccactcac ccacacacta    1800
   cacagactgc ttccttatag ctggactttt atttaaaaaa aaaaaaaaaa aaatggaaaa    1860
   aatccctaaa cattcacctt gaccttattt atgactttac gtgcaaatgt tttgaccata    1920
   ttgatcatat attttgacaa aatatattta taactacgta ttaaaagaaa aaaataaaat    1980
   gagtcattat tttaaaggt                                                 1999
<210> 10
   <211> 408
   <212> PRT
   <213> Homo sapiens
<400> 10
   Met Ile Pro Gly Asn Arg Met Leu Met Val Val Leu Leu Cys Gln Val
   1               5                   10                  15
   Leu Leu Gly Gly Ala Ser His Ala Ser Leu Ile Pro Glu Thr Gly Lys
               20                  25                  30
   Lys Lys Val Ala Glu Ile Gln Gly His Ala Gly Gly Arg Arg Ser Gly
           35                  40                  45
   Gln Ser His Glu Leu Leu Arg Asp Phe Glu Ala Thr Leu Leu Gln Met
       50                  55                  60
   Phe Gly Leu Arg Arg Arg Pro Gln Pro Ser Lys Ser Ala Val Ile Pro
   65                  70                  75                  80
   Asp Tyr Met Arg Asp Leu Tyr Arg Leu Gln Ser Gly Glu Glu Glu Glu
                   85                  90                  95
   Glu Gln Ile His Ser Thr Gly Leu Glu Tyr Pro Glu Arg Pro Ala Ser
               100                 105                 110
   Arg Ala Asn Thr Val Arg Ser Phe His His Glu Glu His Leu Glu Asn
           115                 120                 125
   Ile Pro Gly Thr Ser Glu Asn Ser Ala Phe Arg Phe Leu Phe Asn Leu
       130                 135                 140
   Ser Ser Ile Pro Glu Asn Glu Ala Ile Ser Ser Ala Glu Leu Arg Leu
   145                 150                 155                 160
   Phe Arg Glu Gln Val Asp Gln Gly Pro Asp Trp Glu Arg Gly Phe His
                   165                 170                 175
   Arg Ile Asn Ile Tyr Glu Val Met Lys Pro Pro Ala Glu Val Val Pro
               180                 185                 190
   Gly His Leu Ile Thr Arg Leu Leu Asp Thr Arg Leu Val His His Asn
           195                 200                 205
   Val Thr Arg Trp Glu Thr Phe Asp Val Ser Pro Ala Val Leu Arg Trp
       210                 215                 220
   Thr Arg Glu Lys Gln Pro Asn Tyr Gly Leu Ala Ile Glu Val Thr His
   225                 230                 235                 240
   Leu His Gln Thr Arg Thr His Gln Gly Gln His Val Arg Ile Ser Arg
                   245                 250                 255
   Ser Leu Pro Gln Gly Ser Gly Asn Trp Ala Gln Leu Arg Pro Leu Leu
               260                 265                 270
   Val Thr Phe Gly His Asp Gly Arg Gly His Ala Leu Thr Arg Arg Arg
           275                 280                 285
   Arg Ala Lys Arg Ser Pro Lys His His Ser Gln Arg Ala Arg Lys Lys
       290                 295                 300
   Asn Lys Asn Cys Arg Arg His Ser Leu Tyr Val Asp Phe Ser Asp Val
   305 310 315 320
   Gly Trp Asn Asp Trp Ile Val Ala Pro Pro Gly Tyr Gln Ala Phe Tyr
   325 330 335
   Cys His Gly Asp Cys Pro Phe Pro Leu Ala Asp His Leu Asn Ser Thr
   340 345 350
   Asn His Ala Ile Val Gln Thr Leu Val Asn Ser Val Asn Ser Ser Ile
   355 360 365
   Pro Lys Ala Cys Cys Val Pro Thr Glu Leu Ser Ala Ile Ser Met Leu
   370 375 380
   Tyr Leu Asp Glu Tyr Asp Lys Val Val Leu Lys Asn Tyr Gln Glu Met
   385 390 395 400
   Val Val Glu Gly Cys Gly Cys Arg
   405
<210> 11
   <211> 2207
   <212> DNA
   <213> Homo sapiens
<400> 11
   ctcttgaaga gggctggtat atttgtgcct gctggaggtg gaattaacag taagaaggag 60
   aaagggattg aatggactta caggaaggat ttcaagtaaa ttcagggaaa cacatttact 120
   tgaatagtac aacctagagt attattttac actaagacga cacaaaagat gttaaagtta 180
   tcaccaagct gccggacaga tatatattcc aacaccaagg tgcagatcag catagatctg 240
   tgattcagaa atcaggattt gttttggaaa gagctcaagg gttgagaaga actcaaaagc 300
   aagtgaagat tactttggga actacagttt atcagaagat caacttttgc taattcaaat 360
   accaaaggcc tgattatcat aaattcatat aggaatgcat aggtcatctg atcaaataat 420
   attagccgtc ttctgctaca tcaatgcagc aaaaactctt aacaactgtg gataattgga 480
   aatctgagtt tcagctttct tagaaataac tactcttgac atattccaaa atatttaaaa 540
   taggacagga aaatcggtga ggatgttgtg ctcagaaatg tcactgtcat gaaaaatagg 600
   taaatttgtt ttttcagcta ctgggaaact gtacctccta gaaccttagg tttttttttt     660
   ttttaagagg acaagaagga ctaaaaatat caacttttgc ttttggacaa aaatgcatct     720
   gactgtattt ttacttaagg gtattgtggg tttcctctgg agctgctggg ttctagtggg     780
   ttatgcaaaa ggaggtttgg gagacaatca tgttcactcc agttttattt atagaagact     840
   acggaaccac gaaagacggg aaatacaaag ggaaattctc tctatcttgg gtttgcctca     900
   cagacccaga ccattttcac ctggaaaaca agcgtcctct gcacctctct ttatgctgga     960
   tctctacaat gccatgacca atgaagaaaa tcctgaagag tcggagtact cagtaagggc    1020
   atccttggca gaagagacca gaggggcaag aaagggatac ccagcctctc ccaatgggta    1080
   tcctcgtcgc atacagttat ctcggacgac tcctctgacc acccagagtc ctcctctagc    1140
   cagcctccat gataccaact ttctgaatga tgctgacatg gtcatgagct ttgtcaactt    1200
   agttgaaaga gacaaggatt tttctcacca gcgaaggcat tacaaagaat ttcgatttga    1260
   tcttacccaa attcctcatg gagaggcagt gacagcagct gaattccgga tatacaagga    1320
   ccggagcaac aaccgatttg aaaatgaaac aattaagatt agcatatatc aaatcatcaa    1380
   ggaatacaca aatagggatg cagatctgtt cttgttagac acaagaaagg cccaagcttt    1440
   agatgtgggt tggcttgtct ttgatatcac tgtgaccagc aatcattggg tgattaatcc    1500
   ccagaataat ttgggcttac agctctgtgc agaaacaggg gatggacgca gtatcaacgt    1560
   aaaatctgct ggtcttgtgg gaagacaggg acctcagtca aaacaaccat tcatggtggc    1620
   cttcttcaag gcgagtgagg tacttcttcg atccgtgaga gcagccaaca aacgaaaaaa    1680
   tcaaaaccgc aataaatcca gctctcatca ggactcctcc agaatgtcca gtgttggaga    1740
   ttataacaca agtgagcaaa aacaagcctg taagaagcac gaactctatg tgagcttccg    1800
   ggatctggga tggcaggact ggattatagc accagaagga tacgctgcat tttattgtga    1860
   tggagaatgt tcttttccac ttaacgccca tatgaatgcc accaaccacg ctatagttca    1920
   gactctggtt catctgatgt ttcctgacca cgtaccaaag ccttgttgtg ctccaaccaa    1980
   attaaatgcc atctctgttc tgtactttga tgacagctcc aatgtcattt tgaaaaaata    2040
   tagaaatatg gtagtacgct catgtggctg ccactaatat taaataatat tgataataac    2100
   aaaaagatct gtattaaggt ttatggctgc aataaaaagc atactttcag acaaacgggg    2160
   aatttcctaa aaaaaaaaaa aaaaaaaaaa aaaaaaaaaa aaaaaaa                  2207
<210> 12
   <211> 454
   <212> PRT
   <213> Homo sapiens
<400> 12
   Met His Leu Thr Val Phe Leu Leu Lys Gly Ile Val Gly Phe Leu Trp
   1               5                   10                  15
   Ser Cys Trp Val Leu Val Gly Tyr Ala Lys Gly Gly Leu Gly Asp Asn
               20                  25                  30
   His Val His Ser Ser Phe Ile Tyr Arg Arg Leu Arg Asn His Glu Arg
           35                  40                  45
   Arg Glu Ile Gln Arg Glu Ile Leu Ser Ile Leu Gly Leu Pro His Arg
       50                  55                  60
   Pro Arg Pro Phe Ser Pro Gly Lys Gln Ala Ser Ser Ala Pro Leu Phe
   65                  70                  75                  80
   Met Leu Asp Leu Tyr Asn Ala Met Thr Asn Glu Glu Asn Pro Glu Glu
                   85                  90                  95
   Ser Glu Tyr Ser Val Arg Ala Ser Leu Ala Glu Glu Thr Arg Gly Ala
               100                 105                 110
   Arg Lys Gly Tyr Pro Ala Ser Pro Asn Gly Tyr Pro Arg Arg Ile Gln
           115                 120                 125
   Leu Ser Arg Thr Thr Pro Leu Thr Thr Gln Ser Pro Pro Leu Ala Ser
       130                 135                 140
   Leu His Asp Thr Asn Phe Leu Asn Asp Ala Asp Met Val Met Ser Phe
   145                 150                 155                 160
   Val Asn Leu Val Glu Arg Asp Lys Asp Phe Ser His Gln Arg Arg His
                   165                 170                 175
   Tyr Lys Glu Phe Arg Phe Asp Leu Thr Gln Ile Pro His Gly Glu Ala
               180                 185                 190
   Val Thr Ala Ala Glu Phe Arg Ile Tyr Lys Asp Arg Ser Asn Asn Arg
           195                 200                 205
   Phe Glu Asn Glu Thr Ile Lys Ile Ser Ile Tyr Gln Ile Ile Lys Glu
       210                 215                 220
   Tyr Thr Asn Arg Asp Ala Asp Leu Phe Leu Leu Asp Thr Arg Lys Ala
   225                 230                 235                 240
   Gln Ala Leu Asp Val Gly Trp Leu Val Phe Asp Ile Thr Val Thr Ser
                   245                 250                 255
   Asn His Trp Val Ile Asn Pro Gln Asn Asn Leu Gly Leu Gln Leu Cys
               260                 265                 270
   Ala Glu Thr Gly Asp Gly Arg Ser Ile Asn Val Lys Ser Ala Gly Leu
           275                 280                 285
   Val Gly Arg Gln Gly Pro Gln Ser Lys Gln Pro Phe Met Val Ala Phe
       290                 295                 300
   Phe Lys Ala Ser Glu Val Leu Leu Arg Ser Val Arg Ala Ala Asn Lys
   305                 310                 315                 320
   Arg Lys Asn Gln Asn Arg Asn Lys Ser Ser Ser His Gln Asp Ser Ser
                   325                 330                 335
   Arg Met Ser Ser Val Gly Asp Tyr Asn Thr Ser Glu Gln Lys Gln Ala
               340                 345                 350
   Cys Lys Lys His Glu Leu Tyr Val Ser Phe Arg Asp Leu Gly Trp Gln
           355                 360                 365
   Asp Trp Ile Ile Ala Pro Glu Gly Tyr Ala Ala Phe Tyr Cys Asp Gly
       370                 375                 380
   Glu Cys Ser Phe Pro Leu Asn Ala His Met Asn Ala Thr Asn His Ala
   385                 390                 395                 400
   Ile Val Gln Thr Leu Val His Leu Met Phe Pro Asp His Val Pro Lys
                   405                 410                 415
   Pro Cys Cys Ala Pro Thr Lys Leu Asn Ala Ile Ser Val Leu Tyr Phe
   420 425 430
   Asp Asp Ser Ser Asn Val Ile Leu Lys Lys Tyr Arg Asn Met Val Val
   435 440 445
   Arg Ser Cys Gly Cys His
   450
<210> 13
   <211> 396
   <212> DNA
   <213> Homo sapiens
<400> 13
   aatcaaaacc gcaataaatc cagctctcat caggactcct ccagaatgtc cagtgttgga 60
   gattataaca caagtgagca aaaacaagcc tgtaagaagc acgaactcta tgtgagcttc 120
   cgggatctgg gatggcagga ctggattata gcaccagaag gatacgctgc attttattgt 180
   gatggagaat gttcttttcc acttaacgcc catatgaatg ccaccaacca cgctatagtt 240
   cagactctgg ttcatctgat gtttcctgac cacgtaccaa agccttgttg tgctccaacc 300
   aaattaaatg ccatctctgt tctgtacttt gatgacagct ccaatgtcat tttgaaaaaa 360
   tatagaaata tggtagtacg ctcatgtggc tgccac 396
<210> 14
   <211> 132
   <212> PRT
   <213> Homo sapiens
<400> 14
   Asn Gln Asn Arg Asn Lys Ser Ser Ser His Gln Asp Ser Ser Arg Met
   1 5 10 15
   Ser Ser Val Gly Asp Tyr Asn Thr Ser Glu Gln Lys Gln Ala Cys Lys
   20 25 30
   Lys His Glu Leu Tyr Val Ser Phe Arg Asp Leu Gly Trp Gln Asp Trp
   35 40 45
   Ile Ile Ala Pro Glu Gly Tyr Ala Ala Phe Tyr Cys Asp Gly Glu Cys
   50 55 60
   Ser Phe Pro Leu Asn Ala His Met Asn Ala Thr Asn His Ala Ile Val
   65 70 75 80
   Gln Thr Leu Val His Leu Met Phe Pro Asp His Val Pro Lys Pro Cys
   85 90 95
   Cys Ala Pro Thr Lys Leu Asn Ala Ile Ser Val Leu Tyr Phe Asp Asp
   100 105 110
   Ser Ser Asn Val Ile Leu Lys Lys Tyr Arg Asn Met Val Val Arg Ser
   115 120 125
   Cys Gly Cys His
   130
<210> 15
   <211> 2943
   <212> DNA
   <213> Homo sapiens
<400> 15
   gcaactgggg gcgccccgga cgaccatgag agataaggac tgagggccag gaaggggaag 60
   cgagcccgcc gagaggtggc ggggactgct cacgccaagg gccacagcgg ccgcgctccg 120
   gcctcgctcc gccgctccac gcctcgcggg atccgcgggg gcagcccggc cgggcgggga 180
   tgccggggct ggggcggagg gcgcagtggc tgtgctggtg gtgggggctg ctgtgcagct 240
   gctgcgggcc cccgccgctg cggccgccct tgcccgctgc cgcggccgcc gccgccgggg 300
   ggcagctgct gggggacggc gggagccccg gccgcacgga gcagccgccg ccgtcgccgc 360
   agtcctcctc gggcttcctg taccggcggc tcaagacgca ggagaagcgg gagatgcaga 420
   aggagatctt gtcggtgctg gggctcccgc accggccccg gcccctgcac ggcctccaac 480
   agccgcagcc cccggcgctc cggcagcagg aggagcagca gcagcagcag cagctgcctc 540
   gcggagagcc ccctcccggg cgactgaagt ccgcgcccct cttcatgctg gatctgtaca 600
   acgccctgtc cgccgacaac gacgaggacg gggcgtcgga gggggagagg cagcagtcct 660
   ggccccacga agcagccagc tcgtcccagc gtcggcagcc gcccccgggc gccgcgcacc 720
   cgctcaaccg caagagcctt ctggcccccg gatctggcag cggcggcgcg tccccactga 780
   ccagcgcgca ggacagcgcc ttcctcaacg acgcggacat ggtcatgagc tttgtgaacc 840
   tggtggagta cgacaaggag ttctcccctc gtcagcgaca ccacaaagag ttcaagttca    900
   acttatccca gattcctgag ggtgaggtgg tgacggctgc agaattccgc atctacaagg    960
   actgtgttat ggggagtttt aaaaaccaaa cttttcttat cagcatttat caagtcttac   1020
   aggagcatca gcacagagac tctgacctgt ttttgttgga cacccgtgta gtatgggcct   1080
   cagaagaagg ctggctggaa tttgacatca cggccactag caatctgtgg gttgtgactc   1140
   cacagcataa catggggctt cagctgagcg tggtgacaag ggatggagtc cacgtccacc   1200
   cccgagccgc aggcctggtg ggcagagacg gcccttacga taagcagccc ttcatggtgg  1260
   ctttcttcaa agtgagtgag gtccacgtgc gcaccaccag gtcagcctcc agccggcgcc   1320
   gacaacagag tcgtaatcgc tctacccagt cccaggacgt ggcgcgggtc tccagtgctt   1380
   cagattacaa cagcagtgaa ttgaaaacag cctgcaggaa gcatgagctg tatgtgagtt   1440
   tccaagacct gggatggcag gactggatca ttgcacccaa gggctatgct gccaattact   1500
   gtgatggaga atgctccttc ccactcaacg cacacatgaa tgcaaccaac cacgcgattg   1560
   tgcagacctt ggttcacctt atgaaccccg agtatgtccc caaaccgtgc tgtgcgccaa   1620
   ctaagctaaa tgccatctcg gttctttact ttgatgacaa ctccaatgtc attctgaaaa   1680
   aatacaggaa tatggttgta agagcttgtg gatgccacta actcgaaacc agatgctggg   1740
   gacacacatt ctgccttgga ttcctagatt acatctgcct taaaaaaaca cggaagcaca   1800
   gttggaggtg ggacgatgag actttgaaac tatctcatgc cagtgcctta ttacccagga   1860
   agattttaaa ggacctcatt aataatttgc tcacttggta aatgacgtga gtagttgttg   1920
   gtctgtagca agctgagttt ggatgtctgt agcataaggt ctggtaactg cagaaacata   1980
   accgtgaagc tcttcctacc ctcctccccc aaaaacccac caaaattagt tttagctgta   2040
   gatcaagcta tttggggtgt ttgttagtaa atagggaaaa taatctcaaa ggagttaaat   2100
   gtattcttgg ctaaaggatc agctggttca gtactgtcta tcaaaggtag attttacaga   2160
   gaacagaaat cggggaagtg gggggaacgc ctctgttcag ttcattccca gaagtccaca   2220
   ggacgcacag cccaggccac agccagggct ccacggggcg cccttgtctc agtcattgct  2280
   gttgtatgtt cgtgctggag ttttgttggt gtgaaaatac acttatttca gccaaaacat   2340
   accatttcta cacctcaatc ctccatttgc tgtactcttt gctagtacca aaagtagact   2400
   gattacactg aggtgaggct acaaggggtg tgtaaccgtg taacacgtga aggcagtgct   2460
   cacctcttct ttaccagaac ggttctttga ccagcacatt aacttctgga ctgccggctc   2520
   tagtaccttt tcagtaaagt ggttctctgc ctttttacta tacagcatac cacgccacag   2580
   ggttagaacc aacgaagaaa ataaaatgag ggtgcccagc ttataagaat ggtgttaggg   2640
   ggatgagcat gctgtttatg aacggaaatc atgatttccc tgtagaaagt gaggctcaga   2700
   ttaaatttta gaatattttc taaatgtctt tttcacaatc atgtgactgg gaaggcaatt   2760
   tcatactaaa ctgattaaat aatacattta taatctacaa ctgtttgcac ttacagcttt   2820
   ttttgtaaat ataaactata atttattgtc tattttatat ctgttttgct gtggcgttgg   2880
   ggggggggcc gggcttttgg gggggggggt ttgtttgggg ggtgtcgtgg tgtgggcggg   2940
   cgg                                                                 2943
<210> 16
   <211> 513
   <212> PRT
   <213> Homo sapiens
<400> 16
<210> 17
   <211> 420
   <212> DNA
   <213> Homo sapiens
<400> 17
   aggtcagcct ccagccggcg ccgacaacag agtcgtaatc gctctaccca gtcccaggac 60
   gtggcgcggg tctccagtgc ttcagattac aacagcagtg aattgaaaac agcctgcagg    120
   aagcatgagc tgtatgtgag tttccaagac ctgggatggc aggactggat cattgcaccc    180
   aagggctatg ctgccaatta ctgtgatgga gaatgctcct tcccactcaa cgcacacatg    240
   aatgcaacca accacgcgat tgtgcagacc ttggttcacc ttatgaaccc cgagtatgtc    300
   cccaaaccgt gctgtgcgcc aactaagcta aatgccatct cggttcttta ctttgatgac    360
   aactccaatg tcattctgaa aaaatacagg aatatggttg taagagcttg tggatgccac    420
<210> 18
   <211> 140
   <212> PRT
   <213> Homo sapiens
<400> 18
   Arg Ser Ala Ser Ser Arg Arg Arg Gln Gln Ser Arg Asn Arg Ser Thr
   1               5                   10                  15
   Gln Ser Gln Asp Val Ala Arg Val Ser Ser Ala Ser Asp Tyr Asn Ser
               20                  25                  30
   Ser Glu Leu Lys Thr Ala Cys Arg Lys His Glu Leu Tyr Val Ser Phe
           35                  40                  45
   Gln Asp Leu Gly Trp Gln Asp Trp Ile Ile Ala Pro Lys Gly Tyr Ala
       50                  55                  60
   Ala Asn Tyr Cys Asp Gly Glu Cys Ser Phe Pro Leu Asn Ala His Met
   65                  70                  75                  80
   Asn Ala Thr Asn His Ala Ile Val Gln Thr Leu Val His Leu Met Asn
                   85                  90                  95
   Pro Glu Tyr Val Pro Lys Pro Cys Cys Ala Pro Thr Lys Leu Asn Ala
               100                 105                 110
   Ile Ser Val Leu Tyr Phe Asp Asp Asn Ser Asn Val Ile Leu Lys Lys
           115                 120                 125
   Tyr Arg Asn Met Val Val Arg Ala Cys Gly Cys His
       130                 135                 140
<210> 19
   <211> 1878
   <212> DNA
   <213> Homo sapiens
<400> 19
   gggcgcagcg gggcccgtct gcagcaagtg accgacggcc gggacggccg cctgccccct     60
   ctgccacctg gggcggtgcg ggcccggagc ccggagcccg ggtagcgcgt agagccggcg    120
   cgatgcacgt gcgctcactg cgagctgcgg cgccgcacag cttcgtggcg ctctgggcac    180
   ccctgttcct gctgcgctcc gccctggccg acttcagcct ggacaacgag gtgcactcga    240
   gcttcatcca ccggcgcctc cgcagccagg agcggcggga gatgcagcgc gagatcctct    300
   ccattttggg cttgccccac cgcccgcgcc cgcacctcca gggcaagcac aactcggcac    360
   ccatgttcat gctggacctg tacaacgcca tggcggtgga ggagggcggc gggcccggcg    420
   gccagggctt ctcctacccc tacaaggccg tcttcagtac ccagggcccc cctctggcca    480
   gcctgcaaga tagccatttc ctcaccgacg ccgacatggt catgagcttc gtcaacctcg    540
   tggaacatga caaggaattc ttccacccac gctaccacca tcgagagttc cggtttgatc    600
   tttccaagat cccagaaggg gaagctgtca cggcagccga attccggatc tacaaggact    660
   acatccggga acgcttcgac aatgagacgt tccggatcag cgtttatcag gtgctccagg    720
   agcacttggg cagggaatcg gatctcttcc tgctcgacag ccgtaccctc tgggcctcgg    780
   aggagggctg gctggtgttt gacatcacag ccaccagcaa ccactgggtg gtcaatccgc    840
   ggcacaacct gggcctgcag ctctcggtgg agacgctgga tgggcagagc atcaacccca    900
   agttggcggg cctgattggg cggcacgggc cccagaacaa gcagcccttc atggtggctt    960
   tcttcaaggc cacggaggtc cacttccgca gcatccggtc cacggggagc aaacagcgca   1020
   gccagaaccg ctccaagacg cccaagaacc aggaagccct gcggatggcc aacgtggcag   1080
   agaacagcag cagcgaccag aggcaggcct gtaagaagca cgagctgtat gtcagcttcc   1140
   gagacctggg ctggcaggac tggatcatcg cgcctgaagg ctacgccgcc tactactgtg   1200
   agggggagtg tgccttccct ctgaactcct acatgaacgc caccaaccac gccatcgtgc   1260
   agacgctggt ccacttcatc aacccggaaa cggtgcccaa gccctgctgt gcgcccacgc   1320
   agctcaatgc catctccgtc ctctacttcg atgacagctc caacgtcatc ctgaagaaat   1380
   acagaaacat ggtggtccgg gcctgtggct gccactagct cctccgagaa ttcagaccct   1440
   ttggggccaa gtttttctgg atcctccatt gctcgccttg gccaggaacc agcagaccaa   1500
   ctgccttttg tgagaccttc ccctccctat ccccaacttt aaaggtgtga gagtattagg   1560
   aaacatgagc agcatatggc ttttgatcag tttttcagtg gcagcatcca atgaacaaga   1620
   tcctacaagc tgtgcaggca aaacctagca ggaaaaaaaa acaacgcata aagaaaaatg   1680
   gccgggccag gtcattggct gggaagtctc agccatgcac ggactcgttt ccagaggtaa   1740
   ttatgagcgc ctaccagcca ggccacccag ccgtgggagg aagggggcgt ggcaaggggt   1800
   gggcacattg gtgtctgtgc gaaaggaaaa ttgacccgga agttcctgta ataaatgtca   1860
   caataaaacg aatgaatg                                                 1878
<210> 20
   <211> 431
   <212> PRT
   <213> Homo sapiens
<400> 20
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Arg Ser Gln Asn Arg Ser
       290                 295                 300
   Lys Thr Pro Lys Asn Gln Glu Ala Leu Arg Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
<210> 21
   <211> 417
   <212> DNA
   <213> Homo sapiens
<400> 21
   tccacgggga gcaaacagcg cagccagaac cgctccaaga cgcccaagaa ccaggaagcc     60
   ctgcggatgg ccaacgtggc agagaacagc agcagcgacc agaggcaggc ctgtaagaag    120
   cacgagctgt atgtcagctt ccgagacctg ggctggcagg actggatcat cgcgcctgaa    180
   ggctacgccg cctactactg tgagggggag tgtgccttcc ctctgaactc ctacatgaac    240
   gccaccaacc acgccatcgt gcagacgctg gtccacttca tcaacccgga aacggtgccc    300
   aagccctgct gtgcgcccac gcagctcaat gccatctccg tcctctactt cgatgacagc    360
   tccaacgtca tcctgaagaa atacagaaac atggtggtcc gggcctgtgg ctgccac       417
<210> 22
   <211> 139
   <212> PRT
   <213> Homo sapiens
<400> 22
   Ser Thr Gly Ser Lys Gln Arg Ser Gln Asn Arg Ser Lys Thr Pro Lys
   1               5                   10                  15
   Asn Gln Glu Ala Leu Arg Met Ala Asn Val Ala Glu Asn Ser Ser Ser
   20 25 30
   Asp Gln Arg Gln Ala Cys Lys Lys His Glu Leu Tyr Val Ser Phe Arg
   35 40 45
   Asp Leu Gly Trp Gln Asp Trp Ile Ile Ala Pro Glu Gly Tyr Ala Ala
   50 55 60
   Tyr Tyr Cys Glu Gly Glu Cys Ala Phe Pro Leu Asn Ser Tyr Met Asn
   65 70 75 80
   Ala Thr Asn His Ala Ile Val Gln Thr Leu Val His Phe Ile Asn Pro
   85 90 95
   Glu Thr Val Pro Lys Pro Cys Cys Ala Pro Thr Gln Leu Asn Ala Ile
   100 105 110
   Ser Val Leu Tyr Phe Asp Asp Ser Ser Asn Val Ile Leu Lys Lys Tyr
   115 120 125
   Arg Asn Met Val Val Arg Ala Cys Gly Cys His
   130 135
<210> 23
   <211> 3536
   <212> DNA
   <213> Homo sapiens
<400> 23
   ccgcccgtcc cgccccgccc cgccgcccgc cgcccgccga gcccagcctc cttgccgtcg      60
   gggcgtcccc aggccctggg tcggccgcgg agccgatgcg cgcccgctga gcgccccagc 120
   tgagcgcccc cggcctgcca tgaccgcgct ccccggcccg ctctggctcc tgggcctggc 180
   gctatgcgcg ctgggcgggg gcggccccgg cctgcgaccc ccgcccggct gtccccagcg 240
   acgtctgggc gcgcgcgagc gccgggacgt gcagcgcgag atcctggcgg tgctcgggct 300
   gcctgggcgg ccccggcccc gcgcgccacc cgccgcctcc cggctgcccg cgtccgcgcc 360
   gctcttcatg ctggacctgt accacgccat ggccggcgac gacgacgagg acggcgcgcc 420
   cgcggagcgg cgcctgggcc gcgccgacct ggtcatgagc ttcgttaaca tggtggagcg 480
   agaccgtgcc ctgggccacc aggagcccca ttggaaggag ttccgctttg acctgaccca 540
   gatcccggct ggggaggcgg tcacagctgc ggagttccgg atttacaagg tgcccagcat    600
   ccacctgctc aacaggaccc tccacgtcag catgttccag gtggtccagg agcagtccaa    660
   cagggagtct gacttgttct ttttggatct tcagacgctc cgagctggag acgagggctg    720
   gctggtgctg gatgtcacag cagccagtga ctgctggttg ctgaagcgtc acaaggacct    780
   gggactccgc ctctatgtgg agactgagga cgggcacagc gtggatcctg gcctggccgg    840
   cctgctgggt caacgggccc cacgctccca acagcctttc gtggtcactt tcttcagggc    900
   cagtccgagt cccatccgca cccctcgggc agtgaggcca ctgaggagga ggcagccgaa    960
   gaaaagcaac gagctgccgc aggccaaccg actcccaggg atctttgatg acgtccacgg   1020
   ctcccacggc cggcaggtct gccgtcggca cgagctctac gtcagcttcc aggacctcgg   1080
   ctggctggac tgggtcatcg ctccccaagg ctactcggcc tattactgtg agggggagtg   1140
   ctccttccca ctggactcct gcatgaatgc caccaaccac gccatcctgc agtccctggt   1200
   gcacctgatg atgccagacg cagtccccaa ggcgtgctgt gcacccacca agctgagcgc   1260
   cacctctgtg ctctactatg acagcagcaa caatgtcatc ctgcgcaagc accgcaacat   1320
   ggtggtcaag gcctgcggct gccactgagt ccacccgccc ggcccagctg cagccaccct   1380
   tctcatctgg atcgggcccc tcagaagcag gaaaccctca aacccagcca gaccccaggc   1440
   cggggcattg ccagggagga ccctcacaac cacgtacatg accctttctc cttcatgcca   1500
   ggctcctatg ctccccttgc cctgccaggc atttgtgtga ctgtcctgtt tccagcccag   1560
   gtggtctcaa tcatcaggca gtgttctacc caaatgcaaa cgcctctccc ggaggcatgt   1620
   cctggctggt tctttggggt tggcacagaa gtcctgtctg aggtcctatc catgcccctt   1680
   actggctcag gtcgtgagat agatgtggaa tgacctgaga ggcacctgga gcccactgtt   1740
   ggccaccttg agctcttcac catccatcac agggtgtggt gtgtgtagtc agggtctggt   1800
   tggctcccca ttgcctgccc gaggtgcaag gtggggtata aaactggata acccctgaag   1860
   tattgtatat tcatggatct gaagcactga tccactggtc acaggtagac atgtggagtc   1920
   aactcaagaa aaagctgagt gaacagcatg atttagggct aaagccaatg gcatttatct   1980
   tcccttgtct tcctgctttg catttgcctc tgccatctag gaaagacatg taagagcatg   2040
   gacattttac tttggagaaa cagaaaaatc ttggggcttc caattgaccc atctatctgc   2100
   caccatgttg ccccaccagg agctcagctc tgtggagttt tccctttgct gagcaagcat   2160
   gtggttgcat tgggtggccc aggatgacaa tgcacagcac agatgccatc atttcccttt 2220
   cccctctgaa tggcagacat cagtaatcaa tctggaatgt ttttcttcca aatctgagtg 2280
   gaattttcaa atgatcagca cagccactgc caacagatat gatgtaaagt gaaacctggt 2340
   tgccatcttc tgccatgctg aggagcagtc catccctgcc cgagcatgta tcggcaacat 2400
   gggcagcctg tgaccgggtc tggggcgagg ccaggggcca tcaaaaacag gctgatcacc 2460
   aaagtcagtg tcaccctgga tgcccagcag ccctgtcctg tgtcttgggc ctgtgagtca 2520
   aagaaaaggt ccttttcagg gagtgacaag tagtaattag gctgagttgg gtggagaggt 2580
   ttgtctcagc ctctgctgtt ctcggaaact gctgttctcc ttggagcagc cactgggagt 2640
   tggagtgttt atttgatttc tgacttgcta agcctgtaat ttacctgctg gaatagacag 2700
   agtccagctg cccaaaccgt gtcattaaaa gcagatcctg cgcccgcccc atccacaggc 2760
   acagcccggc agagtggttc cacctcccca tggcccaagg gatgcgcctc tctggagttc 2820
   acgtgctgca cccccaggga ggggcctggg gaaagctggt ccagcagcag gggtggaggc 2880
   tggggccaca ctgcgggaca gcagcccctc cacctggacc agggagggcc tccatgtgca 2940
   agcgcagagg aagagaccct cccatgtacg caaagggcag ccccaggctg tctggaagtt 3000
   ggagaattcc ctatcagcac agggatctca gctctggcct ggaggtgaag agacctgcct 3060
   tgtaggtggc ttccttatct gcgcctccat tttctatctg cactttttga tctccaaaca 3120
   accttcagcc aaagaatctg tctaccaact cctcatagtg agccagaagc agcctcataa 3180
   ccctgaatgt ggggctctgg tggctgtcac gaagcagagt tggcacataa catggaacct 3240
   ggccaggcat ggtggctcac acctataacc ccagcacttt gggaggccaa ggcaggcaga 3300
   tcacctgaag tcaggagttc aagaccatcc tggccaacac agtgaaaccc catctgtact 3360
   aaaaatacaa gattacctgg gcatggtggt gcatgcctat aatcccagct actcaggagg 3420
   ctgaggcaga attgcttgaa cctgggaggt ggaggttgca gtgagcagag atcacaacat 3480
   tgcacttcag cctggtgaca tgagcaaaac tgttgtctca acaaaatgaa attatg 3536
<210> 24
   <211> 402
   <212> PRT
   <213> Homo sapiens
<400> 24
   Met Thr Ala Leu Pro Gly Pro Leu Trp Leu Leu Gly Leu Ala Leu Cys
   1               5                   10                  15
   Ala Leu Gly Gly Gly Gly Pro Gly Leu Arg Pro Pro Pro Gly Cys Pro
               20                  25                  30
   Gln Arg Arg Leu Gly Ala Arg Glu Arg Arg Asp Val Gln Arg Glu Ile
           35                  40                  45
   Leu Ala Val Leu Gly Leu Pro Gly Arg Pro Arg Pro Arg Ala Pro Pro
       50                  55                  60
   Ala Ala Ser Arg Leu Pro Ala Ser Ala Pro Leu Phe Met Leu Asp Leu
   65                  70                  75                  80
   Tyr His Ala Met Ala Gly Asp Asp Asp Glu Asp Gly Ala Pro Ala Glu
                   85                  90                  95
   Arg Arg Leu Gly Arg Ala Asp Leu Val Met Ser Phe Val Asn Met Val
               100                 105                 110
   Glu Arg Asp Arg Ala Leu Gly His Gln Glu Pro His Trp Lys Glu Phe
           115                 120                 125
   Arg Phe Asp Leu Thr Gln Ile Pro Ala Gly Glu Ala Val Thr Ala Ala
       130                 135                 140
   Glu Phe Arg Ile Tyr Lys Val Pro Ser Ile His Leu Leu Asn Arg Thr
   145                 150                 155                 160
   Leu His Val Ser Met Phe Gln Val Val Gln Glu Gln Ser Asn Arg Glu
                   165                 170                 175
   Ser Asp Leu Phe Phe Leu Asp Leu Gln Thr Leu Arg Ala Gly Asp Glu
               180                 185                 190
   Gly Trp Leu Val Leu Asp Val Thr Ala Ala Ser Asp Cys Trp Leu Leu
           195                 200                 205
   Lys Arg His Lys Asp Leu Gly Leu Arg Leu Tyr Val Glu Thr Glu Asp
       210                 215                 220
   Gly His Ser Val Asp Pro Gly Leu Ala Gly Leu Leu Gly Gln Arg Ala
   225                 230                 235                 240
   Pro Arg Ser Gln Gln Pro Phe Val Val Thr Phe Phe Arg Ala Ser Pro
                   245                 250                 255
   Ser Pro Ile Arg Thr Pro Arg Ala Val Arg Pro Leu Arg Arg Arg Gln
               260                 265                 270
   Pro Lys Lys Ser Asn Glu Leu Pro Gln Ala Asn Arg Leu Pro Gly Ile
           275                 280                 285
   Phe Asp Asp Val His Gly Ser His Gly Arg Gln Val Cys Arg Arg His
       290                 295                 300
   Glu Leu Tyr Val Ser Phe Gln Asp Leu Gly Trp Leu Asp Trp Val Ile
   305                 310                 315                 320
   Ala Pro Gln Gly Tyr Ser Ala Tyr Tyr Cys Glu Gly Glu Cys Ser Phe
                   325                 330                 335
   Pro Leu Asp Ser Cys Met Asn Ala Thr Asn His Ala Ile Leu Gln Ser
               340                 345                 350
   Leu Val His Leu Met Met Pro Asp Ala Val Pro Lys Ala Cys Cys Ala
           355                 360                 365
   Pro Thr Lys Leu Ser Ala Thr Ser Val Leu Tyr Tyr Asp Ser Ser Asn
       370                 375                 380
   Asn Val Ile Leu Arg Lys His Arg Asn Met Val Val Lys Ala Cys Gly
   385                 390                 395                 400
   Cys His
<210> 25
   <211> 417
   <212> DNA
   <213> Homo sapiens
<400> 25
   gcagtgaggc cactgaggag gaggcagccg aagaaaagca acgagctgcc gcaggccaac      60
   cgactcccag ggatctttga tgacgtccac ggctcccacg gccggcaggt ctgccgtcgg 120
   cacgagctct acgtcagctt ccaggacctc ggctggctgg actgggtcat cgctccccaa 180
   ggctactcgg cctattactg tgagggggag tgctccttcc cactggactc ctgcatgaat 240
   gccaccaacc acgccatcct gcagtccctg gtgcacctga tgatgccaga cgcagtcccc 300
   aaggcgtgct gtgcacccac caagctgagc gccacctctg tgctctacta tgacagcagc 360
   aacaatgtca tcctgcgcaa gcaccgcaac atggtggtca aggcctgcgg ctgccac 417
<210> 26
   <211> 139
   <212> PRT
   <213> Homo sapiens
<400> 26
   Ala Val Arg Pro Leu Arg Arg Arg Gln Pro Lys Lys Ser Asn Glu Leu
   1               5                   10                  15
   Pro Gln Ala Asn Arg Leu Pro Gly Ile Phe Asp Asp Val His Gly Ser
               20                  25                  30
   His Gly Arg Gln Val Cys Arg Arg His Glu Leu Tyr Val Ser Phe Gln
           35                  40                  45
   Asp Leu Gly Trp Leu Asp Trp Val Ile Ala Pro Gln Gly Tyr Ser Ala
       50                  55                  60
   Tyr Tyr Cys Glu Gly Glu Cys Ser Phe Pro Leu Asp Ser Cys Met Asn
   65                  70                  75                  80
   Ala Thr Asn His Ala Ile Leu Gln Ser Leu Val His Leu Met Met Pro
                   85                  90                  95
   Asp Ala Val Pro Lys Ala Cys Cys Ala Pro Thr Lys Leu Ser Ala Thr
               100                 105                 110
   Ser Val Leu Tyr Tyr Asp Ser Ser Asn Asn Val Ile Leu Arg Lys His
           115                 120                 125
   Arg Asn Met Val Val Lys Ala Cys Gly Cys His
       130                 135
<210> 27
   <211> 1942
   <212> DNA
   <213> Homo sapiens
<400> 27
   cggtccagcc cggcagcggg tgagagtggg tgctggccag gacggttcct tcagagcaaa      60
   cagcagggag atgccggccc gctccttccc agctcctccc cgtgcccgct aacacagcac     120
   ggccgcctgc agtctcctct ctgggtgatt gcgcgggcct aagatgtgtc ctggggcact     180
   gtgggtggcc ctgcccctgc tgtccctgct ggctggctcc ctacagggga agccactgca     240
   gagctgggga cgagggtctg ctgggggaaa cgcccacagc ccactggggg tgcctggagg     300
   tgggctgcct gagcacacct tcaacctgaa gatgtttctg gagaacgtga aggtggattt     360
   cctgcgcagc cttaacctga gtggggtccc ttcgcaggac aaaaccaggg tggagccgcc     420
   gcagtacatg attgacctgt acaacaggta cacgtccgat aagtcgacta cgccagcgtc     480
   caacattgtg cggagcttca gcatggaaga tgccatctcc ataactgcca cagaggactt     540
   ccccttccag aagcacatct tgctcttcaa catctccatt cctaggcatg agcagatcac     600
   cagagctgag ctccgactct atgtctcctg tcaaaatcac gtggacccct ctcatgacct     660
   gaaaggaagc gtggtcattt atgatgttct ggatggaaca gatgcctggg atagtgctac     720
   agagaccaag accttcctgg tgtcccagga cattcaggat gagggctggg agaccttgga     780
   agtgtccagc gccgtgaagc gctgggtccg gtccgactcc accaagagca aaaataagct     840
   ggaagtgact gtggagagcc acaggaaggg ctgcgacacg ctggacatca gtgtcccccc     900
   aggttccaga aacctgccct tctttgttgt cttctccaat gaccacagca gtgggaccaa     960
   ggagaccagg ctggagctga gggagatgat cagccatgaa caagagagcg tgctcaagaa    1020
   gctgtccaag gacggctcca cagaggcagg tgagagcagt cacgaggagg acacggatgg    1080
   ccacgtggct gcggggtcga ctttagccag gcggaaaagg agcgccgggg ctggcagcca    1140
   ctgtcaaaag acctccctgc gggtaaactt cgaggacatc ggctgggaca gctggatcat    1200
   tgcacccaag gagtatgaag cctacgagtg taagggcggc tgcttcttcc ccttggctga    1260
   cgatgtgacg ccgacgaaac acgctatcgt gcagaccctg gtgcatctca agttccccac    1320
   aaaggtgggc aaggcctgct gtgtgcccac caaactgagc cccatctccg tcctctacaa    1380
   ggatgacatg ggggtgccca ccctcaagta ccattacgag ggcatgagcg tggcagagtg    1440
   tgggtgcagg tagtatctgc ctgcggggct ggggaggcag gccaaagggg ctccacatga   1500
   gaggtcctgc atgcccctgg gcacaacaag gactgattca atctgcatgc cagcctggag   1560
   gaggaaaggg agcctgctct ccctccccac accccaccca aagcatacac cgctgagctc   1620
   aactgccagg gaaggctaag gaaatgggga tttgagcaca acaggaaagc ctgggagggt   1680
   tgttgggatg caaggaggtg atgaaaagga gacaggggga aaaataatcc atagtcagca   1740
   gaaaacaaca gcagtgagcc agaggagcac aggcgggcag gtcactgcag agactgatgg   1800
   aagttagaga ggtggaggag gccagctcgc tccaaaaccc ttggggagta gagggaagga   1860
   gcaggccgcg tgtcacaccc atcattgtat gttatttccc acaacccagt tggaggggca   1920
   tggcttccaa tttagagacc cg                                            1942
<210> 28
   <211> 429
   <212> PRT
   <213> Homo sapiens
<400> 28
<210> 29
   <211> 1584
   <212> DNA
   <213> Homo sapiens
<400> 29
   ggggagagga agagtggtag ggggagggag agagagagga agagtttcca aacttgtctc      60
   cagtgacagg agacatttac gttccacaag ataaaactgc cacttagagc ccagggaagc     120
   taaaccttcc tggcttggcc taggagctcg agcggagtca tgggctctct ggtcctgaca     180
   ctgtgcgctc ttttctgcct ggcagcttac ttggtttctg gcagccccat catgaaccta     240
   gagcagtctc ctctggaaga agatatgtcc ctctttggtg atgttttctc agagcaagac     300
   ggtgtcgact ttaacacact gctccagagc atgaaggatg agtttcttaa gacactaaac     360
   ctctctgaca tccccacgca ggattcagcc aaggtggacc caccagagta catgttggaa     420
   ctctacaaca aatttgcaac agatcggacc tccatgccct ctgccaacat cattaggagt     480
   ttcaagaatg aagatctgtt ttcccagccg gtcagtttta atgggctccg aaaatacccc     540
   ctcctcttca atgtgtccat tcctcaccat gaagaggtca tcatggctga acttaggcta     600
   tacacactgg tgcaaaggga tcgtatgata tacgatggag tagaccggaa aattaccatt     660
   tttgaagtgc tggagagcaa aggggataat gagggagaaa gaaacatgct ggtcttggtg     720
   tctggggaga tatatggaac caacagtgag tgggagactt ttgatgtcac agatgccatc 780
   agacgttggc aaaagtcagg ctcatccacc caccagctgg aggtccacat tgagagcaaa 840
   cacgatgaag ctgaggatgc cagcagtgga cggctagaaa tagataccag tgcccagaat 900
   aagcataacc ctttgctcat cgtgttttct gatgaccaaa gcagtgacaa ggagaggaag 960
   gaggaactga atgaaatgat ttcccatgag caacttccag agctggacaa cttgggcctg 1020
   gatagctttt ccagtggacc tggggaagag gctttgttgc agatgagatc aaacatcatc 1080
   tatgactcca ctgcccgaat cagaaggaac gccaaaggaa actactgtaa gaggaccccg 1140
   ctctacatcg acttcaagga gattgggtgg gactcctgga tcatcgctcc gcctggatac 1200
   gaagcctatg aatgccgtgg tgtttgtaac taccccctgg cagagcatct cacacccaca 1260
   aagcatgcaa ttatccaggc cttggtccac ctcaagaatt cccagaaagc ttccaaagcc 1320
   tgctgtgtgc ccacaaagct agagcccatc tccatcctct atttagacaa aggcgtcgtc 1380
   acctacaagt ttaaatacga aggcatggcc gtctccgaat gtggctgtag atagaagaag 1440
   agtcctatgg cttatttaat aactgtaaat gtgtatattt ggtgttccta tttaatgaga 1500
   ttatttaata agggtgtaca gtaatagagg cttgctgcct tcaggaaatg gacaggtcag 1560
   tttgttgtag gaaatgcata tttt 1584
<210> 30
   <211> 424
   <212> PRT
   <213> Homo sapiens
<400> 30
   Met Gly Ser Leu Val Leu Thr Leu Cys Ala Leu Phe Cys Leu Ala Ala
   1 5 10 15
   Tyr Leu Val Ser Gly Ser Pro Ile Met Asn Leu Glu Gln Ser Pro Leu
   20 25 30
   Glu Glu Asp Met Ser Leu Phe Gly Asp Val Phe Ser Glu Gln Asp Gly
   35 40 45
   Val Asp Phe Asn Thr Leu Leu Gln Ser Met Lys Asp Glu Phe Leu Lys
   50 55 60
   Thr Leu Asn Leu Ser Asp Ile Pro Thr Gln Asp Ser Ala Lys Val Asp
   65                  70                  75                  80
   Pro Pro Glu Tyr Met Leu Glu Leu Tyr Asn Lys Phe Ala Thr Asp Arg
                   85                  90                  95
   Thr Ser Met Pro Ser Ala Asn Ile Ile Arg Ser Phe Lys Asn Glu Asp
               100                 105                 110
   Leu Phe Ser Gln Pro Val Ser Phe Asn Gly Leu Arg Lys Tyr Pro Leu
           115                 120                 125
   Leu Phe Asn Val Ser Ile Pro His His Glu Glu Val Ile Met Ala Glu
       130                 135                 140
   Leu Arg Leu Tyr Thr Leu Val Gln Arg Asp Arg Met Ile Tyr Asp Gly
   145                 150                 155                 160
   Val Asp Arg Lys Ile Thr Ile Phe Glu Val Leu Glu Ser Lys Gly Asp
                   165                 170                 175
   Asn Glu Gly Glu Arg Asn Met Leu Val Leu Val Ser Gly Glu Ile Tyr
               180                 185                 190
   Gly Thr Asn Ser Glu Trp Glu Thr Phe Asp Val Thr Asp Ala Ile Arg
           195                 200                 205
   Arg Trp Gln Lys Ser Gly Ser Ser Thr His Gln Leu Glu Val His Ile
       210                 215                 220
   Glu Ser Lys His Asp Glu Ala Glu Asp Ala Ser Ser Gly Arg Leu Glu
   225                 230                 235                 240
   Ile Asp Thr Ser Ala Gln Asn Lys His Asn Pro Leu Leu Ile Val Phe
                   245                 250                 255
   Ser Asp Asp Gln Ser Ser Asp Lys Glu Arg Lys Glu Glu Leu Asn Glu
               260                 265                 270
   Met Ile Ser His Glu Gln Leu Pro Glu Leu Asp Asn Leu Gly Leu Asp
           275                 280                 285
   Ser Phe Ser Ser Gly Pro Gly Glu Glu Ala Leu Leu Gln Met Arg Ser
   290 295 300
   Asn Ile Ile Tyr Asp Ser Thr Ala Arg Ile Arg Arg Asn Ala Lys Gly
   305 310 315 320
   Asn Tyr Cys Lys Arg Thr Pro Leu Tyr Ile Asp Phe Lys Glu Ile Gly
   325 330 335
   Trp Asp Ser Trp Ile Ile Ala Pro Pro Gly Tyr Glu Ala Tyr Glu Cys
   340 345 350
   Arg Gly Val Cys Asn Tyr Pro Leu Ala Glu His Leu Thr Pro Thr Lys
   355 360 365
   His Ala Ile Ile Gln Ala Leu Val His Leu Lys Asn Ser Gln Lys Ala
   370 375 380
   Ser Lys Ala Cys Cys Val Pro Thr Lys Leu Glu Pro Ile Ser Ile Leu
   385 390 395 400
   Tyr Leu Asp Lys Gly Val Val Thr Tyr Lys Phe Lys Tyr Glu Gly Met
   405 410 415
   Ala Val Ser Glu Cys Gly Cys Arg
   420
<210> 31
   <211> 324
   <212> DNA
   <213> Homo sapiens
<400> 31
   aacgccaaag gaaactactg taagaggacc ccgctctaca tcgacttcaa ggagattggg 60
   tgggactcct ggatcatcgc tccgcctgga tacgaagcct atgaatgccg tggtgtttgt 120
   aactaccccc tggcagagca tctcacaccc acaaagcatg caattatcca ggccttggtc 180
   cacctcaaga attcccagaa agcttccaaa gcctgctgtg tgcccacaaa gctagagccc 240
   atctccatcc tctatttaga caaaggcgtc gtcacctaca agtttaaata cgaaggcatg 300
   gccgtctccg aatgtggctg taga 324
<210> 32
   <211> 108
   <212> PRT
   <213> Homo sapiens
<400> 32
   Asn Ala Lys Gly Asn Tyr Cys Lys Arg Thr Pro Leu Tyr Ile Asp Phe
   1               5                   10                  15
   Lys Glu Ile Gly Trp Asp Ser Trp Ile Ile Ala Pro Pro Gly Tyr Glu
               20                  25                  30
   Ala Tyr Glu Cys Arg Gly Val Cys Asn Tyr Pro Leu Ala Glu His Leu
           35                  40                  45
   Thr Pro Thr Lys His Ala Ile Ile Gln Ala Leu Val His Leu Lys Asn
       50                  55                  60
   Ser Gln Lys Ala Ser Lys Ala Cys Cys Val Pro Thr Lys Leu Glu Pro
   65                  70                  75                  80
   Ile Ser Ile Leu Tyr Leu Asp Lys Gly Val Val Thr Tyr Lys Phe Lys
                   85                  90                  95
   Tyr Glu Gly Met Ala Val Ser Glu Cys Gly Cys Arg
               100                 105
<210> 33
   <211> 1443
   <212> DNA
   <213> Homo sapiens
<400> 33
   tctccgcccc ccagtacctc cctcccctcc cctccagcat ggtgctcgcg gccccgctgc 60
   tgctgggctt cctgctcctc gccctggagc tgcggccccg gggggaggcg gccgagggcc 120
   ccgcggcggc ggcggcggcg gcggcggcgg cggcagcggc gggggtcggg ggggagcgct 180
   ccagccggcc agccccgtcc gtggcgcccg agccggacgg ctgccccgtg tgcgtttggc 240
   ggcagcacag ccgcgagctg cgcctagaga gcatcaagtc gcagatcttg agcaaactgc 300
   ggctcaagga ggcgcccaac atcagccgcg aggtggtgaa gcagctgctg cccaaggcgc 360
   cgccgctgca gcagatcctg gacctacacg acttccaggg cgacgcgctg cagcccgagg 420
   acttcctgga ggaggacgag taccacgcca ccaccgagac cgtcattagc atggcccagg     480
   agacggaccc agcagtacag acagatggca gccctctctg ctgccatttt cacttcagcc     540
   ccaaggtgat gttcacaaag gtactgaagg cccagctgtg ggtgtaccta cggcctgtac     600
   cccgcccagc cacagtctac ctgcagatct tgcgactaaa acccctaact ggggaaggga     660
   ccgcaggggg agggggcgga ggccggcgtc acatccgtat ccgctcactg aagattgagc     720
   tgcactcacg ctcaggccat tggcagagca tcgacttcaa gcaagtgcta cacagctggt     780
   tccgccagcc acagagcaac tggggcatcg agatcaacgc ctttgatccc agtggcacag     840
   acctggctgt cacctccctg gggccgggag ccgaggggct gcatccattc atggagcttc     900
   gagtcctaga gaacacaaaa cgttcccggc ggaacctggg tctggactgc gacgagcact     960
   caagcgagtc ccgctgctgc cgatatcccc tcacagtgga ctttgaggct ttcggctggg    1020
   actggatcat cgcacctaag cgctacaagg ccaactactg ctccggccag tgcgagtaca    1080
   tgttcatgca aaaatatccg catacccatt tggtgcagca ggccaatcca agaggctctg    1140
   ctgggccctg ttgtaccccc accaagatgt ccccaatcaa catgctctac ttcaatgaca    1200
   agcagcagat tatctacggc aagatccctg gcatggtggt ggatcgctgt ggctgctctt    1260
   aaggtggggg atagaggatg cctcccccac agaccctacc ccaagacccc tagccctgcc    1320
   cccatccccc caagccctag agctccctcc actcttcccg cgaacatcac accgttcccc    1380
   gaccaagccg tgtgcaatac aacagaggga ggcaggtggg aattgagggt gaggggtttg    1440
   ggg                                                                  1443
<210> 34
   <211> 407
   <212> PRT
   <213> Homo sapiens
<400> 34
   Met Val Leu Ala Ala Pro Leu Leu Leu Gly Phe Leu Leu Leu Ala Leu
   1               5                   10                  15
   Glu Leu Arg Pro Arg Gly Glu Ala Ala Glu Gly Pro Ala Ala Ala Ala
               20                  25                  30
   Ala Ala Ala Ala Ala Ala Ala Ala Ala Gly Val Gly Gly Glu Arg Ser
           35                  40                  45
   Ser Arg Pro Ala Pro Ser Val Ala Pro Glu Pro Asp Gly Cys Pro Val
   50 55 60
   Cys Val Trp Arg Gln His Ser Arg Glu Leu Arg Leu Glu Ser Ile Lys
   65 70 75 80
   Ser Gln Ile Leu Ser Lys Leu Arg Leu Lys Glu Ala Pro Asn Ile Ser
   85 90 95
   Arg Glu Val Val Lys Gln Leu Leu Pro Lys Ala Pro Pro Leu Gln Gln
   100 105 110
   Ile Leu Asp Leu His Asp Phe Gln Gly Asp Ala Leu Gln Pro Glu Asp
   115 120 125
   Phe Leu Glu Glu Asp Glu Tyr His Ala Thr Thr Glu Thr Val Ile Ser
   130 135 140
   Met Ala Gln Glu Thr Asp Pro Ala Val Gln Thr Asp Gly Ser Pro Leu
   145 150 155 160
   Cys Cys His Phe His Phe Ser Pro Lys Val Met Phe Thr Lys Val Leu
   165 170 175
   Lys Ala Gln Leu Trp Val Tyr Leu Arg Pro Val Pro Arg Pro Ala Thr
   180 185 190
   Val Tyr Leu Gln Ile Leu Arg Leu Lys Pro Leu Thr Gly Glu Gly Thr
   195 200 205
   Ala Gly Gly Gly Gly Gly Gly Arg Arg His Ile Arg Ile Arg Ser Leu
   210 215 220
   Lys Ile Glu Leu His Ser Arg Ser Gly His Trp Gln Ser Ile Asp Phe
   225 230 235 240
   Lys Gln Val Leu His Ser Trp Phe Arg Gln Pro Gln Ser Asn Trp Gly
   245 250 255
   Ile Glu Ile Asn Ala Phe Asp Pro Ser Gly Thr Asp Leu Ala Val Thr
   260 265 270
   Ser Leu Gly Pro Gly Ala Glu Gly Leu His Pro Phe Met Glu Leu Arg
   275 280 285
   Val Leu Glu Asn Thr Lys Arg Ser Arg Arg Asn Leu Gly Leu Asp Cys
   290 295 300
   Asp Glu His Ser Ser Glu Ser Arg Cys Cys Arg Tyr Pro Leu Thr Val
   305 310 315 320
   Asp Phe Glu Ala Phe Gly Trp Asp Trp Ile Ile Ala Pro Lys Arg Tyr
   325 330 335
   Lys Ala Asn Tyr Cys Ser Gly Gln Cys Glu Tyr Met Phe Met Gln Lys
   340 345 350
   Tyr Pro His Thr His Leu Val Gln Gln Ala Asn Pro Arg Gly Ser Ala
   355 360 365
   Gly Pro Cys Cys Thr Pro Thr Lys Met Ser Pro Ile Asn Met Leu Tyr
   370 375 380
   Phe Asn Asp Lys Gln Gln Ile Ile Tyr Gly Lys Ile Pro Gly Met Val
   385 390 395 400
   Val Asp Arg Cys Gly Cys Ser
   405
<210> 35
   <211> 1456
   <212> DNA
   <213> Homo sapiens
<400> 35
   cccggagcca cggagcccgc gccgcccgcc cgcccggccc acggagccca tggacctgag 60
   cgccgccgcc gcgctgtgcc tttggctgct gagcgcctgc cgcccccgcg acgggctgga 120
   agcggccgcc gtgctgcgag cggcgggggc tgggccggtc cggagcccag gggcggcggg 180
   cggcggcggc ggcggcggcg gcggcgggcg gactcttgcc caggctgcgg gcgccgcggc 240
   tgtcccggcc gccgcggttc cccgggcccg cgccgcgcgc cgcgccgcgg gctccggctt 300
   caggaacggc tcggtggtgc cgcaccactt catgatgtcg ctttaccgga gcctggccgg 360
   gagggctccg gccggggcag ccgctgtctc cgcctcgggc catggtcgcg cggacacgat 420
   caccggcttc acagaccagg cgacccaaga cgaatcggca gccgaaacag gccagagctt 480
   cctgttcgac gtgtccagcc ttaacgacgc agacgaggtg gtgggtgccg agctgcgcgt 540
   gctgcgccgg ggatctccag agtcgggccc aggcagctgg acttctccgc cgttgctgct 600
   gctgtccacg tgcccgggcg ccgcccgagc gccacgcctg ctgtactcgc gggcagctga 660
   gcccctagtc ggtcagcgct gggaggcgtt cgacgtggcg gacgccatga ggcgccaccg 720
   tcgtgaaccg cgcccccccc gcgcgttctg cctcttgctg cgcgcagtgg caggcccggt 780
   gccgagcccg ttggcactgc ggcgactggg cttcggctgg ccgggcggag ggggctctgc 840
   ggcagaggag cgcgcggtgc tagtcgtctc ctcccgcacg cagaggaaag agagcttatt 900
   ccgggagatc cgcgcccagg cccgcgcgct cggggccgct ctggcctcag agccgctgcc 960
   cgacccagga accggcaccg cgtcgccaag ggcagtcatt ggcggccgca gacggaggag 1020
   gacggcgttg gccgggacgc ggacagcgca gggcagcggc gggggcgcgg gccggggcca 1080
   cgggcgcagg ggccggagcc gctgcagccg caagccgttg cacgtggact tcaaggagct 1140
   cggctgggac gactggatca tcgcgccgct ggactacgag gcgtaccact gcgagggcct 1200
   ttgcgacttc cctttgcgtt cgcacctcga gcccaccaac catgccatca ttcagacgct 1260
   gctcaactcc atggcaccag acgcggcgcc ggcctcctgc tgtgtgccag cgcgcctcag 1320
   ccccatcagc atcctctaca tcgacgccgc caacaacgtt gtctacaagc aatacgagga 1380
   catggtggtg gaggcctgcg gctgcaggta gcgcgcgggc cggggagggg gcagccacgc 1440
   ggccgaggat ccccag 1456
<210> 36
   <211> 453
   <212> PRT
   <213> Homo sapiens
<400> 36
<210> 37
   <211> 4561
   <212> DNA
   <213> Homo sapiens
<400> 37
   atggatactc ccagggtcct gctctcggcc gtcttcctca tcagttttct gtgggatttg 60
   cccggtttcc agcaggcttc catctcatcc tcctcgtcgt ccgccgagct gggttccacc 120
   aagggcatgc gaagccgcaa ggaaggcaag atgcagcggg cgccgcgcga cagtgacgcg 180
   ggccgggagg gccaggaacc acagccgcgg cctcaggacg aaccccgggc tcagcagccc 240
   cgggcgcagg agccgccagg caggggtccg cgcgtggtgc cccacgagta catgctgtca 300
   atctacagga cttactccat cgctgagaag ctgggcatca atgccagctt tttccagtct 360
   tccaagtcgg ctaatacgat caccagcttt gtagacaggg gactagcctc cccaccccca 420
   tccccgccac tgcgctgtat gcccccgatc ctggagatag aggagggatt gcatgtgcac 480
   ccagtctggg gaggcctctg ttcccgagcc aaggcctcca gactagcagg accgagatcc 540
   acaagtaccg ctacaacccc ggaatgcggt ggagggctgg ggatgcgagc ggtccacagc 600
   gggaggagac gctgcatgac cacccggaca gcatcttcaa aggcgctgtc tcctccgcgt 660
   ctccgggttc tcagaaaatg cgaagaaagg cagttcgttg actgggaagg cgccttcgtg 720
   gaggacctgg ttgctcccct gggctcccgg gtgacttacc ggtcgacagt gcagggaagg 780
   ccgcccctag ccaacccccc agagacaaaa ggagtttcct taaagcgcct tggagctcct 840
   gataggactg gccctgtggt cagatcgtgc ccccacaaaa ggacaccaaa gtggggaaag 900
   aaacgaatcc ttaggcctct cagggctgca ctgccagcca gggagtcatt tgggttagca 960
   caagggctgt cactggcctt gacccctgca gaagaggaaa cctgcaagaa gggctggatc 1020
   cttgatgtgg ctggggtgga ggtcatctgt tcacctggca ctgtgggagg gacagcagtg 1080
   gtgacagggc ctcacaggac tgagaagaat cgggatgatg agcacctttc ctccactcac 1140
   ttctggaatc ttgtggagag aagtggacca caaggactga agtccagaga actggatttc 1200
   aaaggaagtc aagagaagga cagctgccag gaaacgaaga taagccctaa tccagcaatt 1260
   gtaactggaa atgtttatat gtctaatgtt cagcaaacac cgttaacaga ggtcagcagg 1320
   cagcaggcaa agtgtcttca gggtaacagt gcatggtgtt gtattcactc cccaggttgg 1380
   atgcctcctc tgagctattt ctgtgatgca ggtttagaca ggttggggca tgtgcgtaag 1440
   agtgaaccag ccagtgctac ggtcagcccc gagtacaaac tgggtgtggc agaggctcca 1500
   gagccagtgc ccccgggcta catctggact gacgatctct cgcacactcc tctccggaga 1560
   cagaagtatt tgtttgatgt gtccatgctc tcagacaaag aagagctggt gggcgcggag 1620
   ctgcggctct ttcgccaggc gccctcagcg ccctgggggc caccagccgg gccgctccac 1680
   gtgcagctct tcccttgcct ttcgccccta ctgctggacg cgcggaccct ggacccgcag 1740
   ggggcgccgc cggccggctg ggaagtcttc gacgtgtggc agggcctgcg ccaccagccc 1800
   tggaagcagc tgtgcttgga gctgcgggcc gcatggggcg agctggacgc cggggaggcc 1860
   gaggcgcgcg cgcggggacc ccagcaaccg ccgcccccgg acctgcggag tctgggcttc 1920
   ggccggaggg tgcggcctcc ccaggagcgg gccctgctgg tggtattcac cagatcccag 1980
   cgcaagaacc tgttcgcaga gatgcgcgag cagctgggct cggccgaggc tgcgggcccg 2040
   ggcgcgggcg ccgaggggtc gtggccgccg ccgtcgggcg ccccggatgc caggccttgg 2100
   ctgccctcgc ccggccgccg gcggcggcgc acggccttcg ccagtcgcca tggcaagcgg 2160
   cacggcaaga agtccaggct acgctgcagc aagaagcccc tgcacgtgaa cttcaaggag 2220
   ctgggctggg acgactggat tatcgcgccc ctggagtacg aggcctatca ctgcgagggt 2280
   gtatgcgact tcccgctgcg ctcgcacctg gagcccacca accacgccat catccagacg 2340
   ctgatgaact ccatggaccc cggctccacc ccgcccagct gctgcgtgcc caccaaattg 2400
   actcccatca gcattctata catcgacgcg ggcaataatg tggtctacaa gcagtacgag 2460
   gacatggtgg tggagtcgtg cggctgcagg tagcggtgcc tttcccgccg ccttggcccg 2520
   gaaccaaggt gggccaaggt ccgccttgca ggggaggcct ggctgcagag aggcggagga 2580
   ggaagctggc gctgggggag gctgagggtg agggaacagc ctggatgtga gagccggtgg 2640
   gagagaaggg agcgcagcct tcccagtaac ttctacctgc cagcccagag ggaaatatgg 2700
   attttcacac cttgcctggc caccctggaa aaacaagcca aggaggattt cttttgttct 2760
   gttttctctc tctctctctc tctctctctc tctctctctc tctctctctc tattactgtg 2820
   gctttggatt tccttatgtg tcttacaggc tttgatagaa ggggagggga ggagagatgc 2880
   atacccgttt ctcaactgct ccatggattg aaaaaataac agtttaaaaa gggaaacaat 2940
   gtgggaggaa gaatcaccgt tgacgcatct tgatttggtt ggtttttaca tgtgtaaaga 3000
   aggtggggtc tctggccatg tcatagccca tgtcttgtgc cctcccacac agaaagtgtt 3060
   agatagggaa attggcaaaa agaatagtta agtcaggaat ggtcctgcct atagaagagc 3120
   tttgagagag gtgggcccac gggtgcccct ctcacccatt tgtgtactct gtgagtttac 3180
   cagctctgcc ctggcctctt tcggtaccag gaactggcaa ccttcatctc actcctgagg 3240
   gcccaggtct ctgccttcat tgttgctttt tctggtgggg gcaaggggag ctggtatgga 3300
   tggaatgaca agaattagtc caaatggaac cccttgaagg ataatgagaa accacaaggc 3360
   ctgcctctga ctggggctga cacggaggtg cattagccca ggctggaggt agcccaccca 3420
   aatgcccttt ctgattctaa ttgatttctt tcaacagaat ttgccaaaat tcagacatgc 3480
   acttctaagg ggaaggtgat tttccagttc aaaaaaatgg gcaggagtgg ggaacaaaac 3540
   aattaacgta agagctacaa aggagggaaa aggaaccaag aagtagaagg agtcccatca 3600
   ggagggaaga tggtgggcct cagggaggat ggggatcaag ggacaggcca ggagccagga 3660
   gtggggaagg gagggatgaa aggggacaca agtccctgtc tctgaagttt ctttaaaatc 3720
   tgagttccct cccctctctt tgacattcct gaaagattac cagccagcaa tagcccaggg 3780
   ctcccccaaa agaattggtt cagattgtaa ttatcagtta ggcaatgttt ttaaaactta 3840
   gtaatgagaa actgtgaaaa gagccaagtg ttacattgag cttggggtgg gagatgggga 3900
   acaggcagtg aggaaggaga caggggtgga attcgtcttc tgggaggaag ctggagagag 3960
   cacagtgaaa ttgaaatacc cattcccaga tagtcaaaaa catgaacttt cccccagcct 4020
   gcaccagtat tgttttcaaa cattgcccat gagtaggccc tttgaagagt tagcttcctc 4080
   ctcatctttg actataaaat tgtttaatca atggaatttg taccagcctt ttaaaaagtt 4140
   ttagtttttc ctaagtgatt ttgctctctt ccaatctaaa cctgttgctt gtttggttca 4200
   gagaactaca aactgtcaaa gaaagggtgg ggatgataag aaatgctaat ataaaaatgc 4260
   taagtgaaaa aaagacttgg ccaggagaaa taatttaaaa tgcacatttg ctttggatgc 4320
   actgttgttc tgttaaggct gtatatattt gtttatttaa ggtgactgaa agtgcaaaga 4380
   ggaaatggac agcatgcaat tcatcctaat gtacaaaacg ttatatgcac tcaaatgtta 4440
   taatttctaa tatttttaaa gtttatattc gagttgtaca aagttaagca ttaatcagat 4500
   atttcatttt ttcataatgt taccattttc ttaaatatta ttacaaaatt ttaagtctgt 4560
   c 4561
<210> 38
   <211> 830
   <212> PRT
   <213> Homo sapiens
<400> 38
   Met Asp Thr Pro Arg Val Leu Leu Ser Ala Val Phe Leu Ile Ser Phe
   1               5                   10                  15
   Leu Trp Asp Leu Pro Gly Phe Gln Gln Ala Ser Ile Ser Ser Ser Ser
               20                  25                  30
   Ser Ser Ala Glu Leu Gly Ser Thr Lys Gly Met Arg Ser Arg Lys Glu
           35                  40                  45
   Gly Lys Met Gln Arg Ala Pro Arg Asp Ser Asp Ala Gly Arg Glu Gly
       50                  55                  60
   Gln Glu Pro Gln Pro Arg Pro Gln Asp Glu Pro Arg Ala Gln Gln Pro
   65                  70                  75                  80
   Arg Ala Gln Glu Pro Pro Gly Arg Gly Pro Arg Val Val Pro His Glu
                   85                  90                  95
   Tyr Met Leu Ser Ile Tyr Arg Thr Tyr Ser Ile Ala Glu Lys Leu Gly
               100                 105                 110
   Ile Asn Ala Ser Phe Phe Gln Ser Ser Lys Ser Ala Asn Thr Ile Thr
           115                 120                 125
   Ser Phe Val Asp Arg Gly Leu Ala Ser Pro Pro Pro Ser Pro Pro Leu
       130                 135                 140
   Arg Cys Met Pro Pro Ile Leu Glu Ile Glu Glu Gly Leu His Val His
   145                 150                 155                 160
   Pro Val Trp Gly Gly Leu Cys Ser Arg Ala Lys Ala Ser Arg Leu Ala
                   165                 170                 175
   Gly Pro Arg Ser Thr Ser Thr Ala Thr Thr Pro Glu Cys Gly Gly Gly
               180                 185                 190
   Leu Gly Met Arg Ala Val His Ser Gly Arg Arg Arg Cys Met Thr Thr
           195                 200                 205
   Arg Thr Ala Ser Ser Lys Ala Leu Ser Pro Pro Arg Leu Arg Val Leu
       210                 215                 220
   Arg Lys Cys Glu Glu Arg Gln Phe Val Asp Trp Glu Gly Ala Phe Val
   225                 230                 235                 240
   Glu Asp Leu Val Ala Pro Leu Gly Ser Arg Val Thr Tyr Arg Ser Thr
                   245                 250                 255
   Val Gln Gly Arg Pro Pro Leu Ala Asn Pro Pro Glu Thr Lys Gly Val
               260                 265                 270
   Ser Leu Lys Arg Leu Gly Ala Pro Asp Arg Thr Gly Pro Val Val Arg
           275                 280                 285
   Ser Cys Pro His Lys Arg Thr Pro Lys Trp Gly Lys Lys Arg Ile Leu
       290                 295                 300
   Arg Pro Leu Arg Ala Ala Leu Pro Ala Arg Glu Ser Phe Gly Leu Ala
   305                 310                 315                 320
   Gln Gly Leu Ser Leu Ala Leu Thr Pro Ala Glu Glu Glu Thr Cys Lys
                   325                 330                 335
   Lys Gly Trp Ile Leu Asp Val Ala Gly Val Glu Val Ile Cys Ser Pro
               340                 345                 350
   Gly Thr Val Gly Gly Thr Ala Val Val Thr Gly Pro His Arg Thr Glu
           355                 360                 365
   Lys Asn Arg Asp Asp Glu His Leu Ser Ser Thr His Phe Trp Asn Leu
       370                 375                 380
   Val Glu Arg Ser Gly Pro Gln Gly Leu Lys Ser Arg Glu Leu Asp Phe
   385                 390                 395                 400
   Lys Gly Ser Gln Glu Lys Asp Ser Cys Gln Glu Thr Lys Ile Ser Pro
                   405                 410                 415
   Asn Pro Ala Ile Val Thr Gly Asn Val Tyr Met Ser Asn Val Gln Gln
               420                 425                 430
   Thr Pro Leu Thr Glu Val Ser Arg Gln Gln Ala Lys Cys Leu Gln Gly
               435                 440                 445
   Asn Ser Ala Trp Cys Cys Ile His Ser Pro Gly Trp Met Pro Pro Leu
       450                 455                 460
   Ser Tyr Phe Cys Asp Ala Gly Leu Asp Arg Leu Gly His Val Arg Lys
   465                 470                 475                 480
   Ser Glu Pro Ala Ser Ala Thr Val Ser Pro Glu Tyr Lys Leu Gly Val
                   485                 490                 495
   Ala Glu Ala Pro Glu Pro Val Pro Pro Gly Tyr Ile Trp Thr Asp Asp
               500                 505                 510
   Leu Ser His Thr Pro Leu Arg Arg Gln Lys Tyr Leu Phe Asp Val Ser
           515                 520                 525
   Met Leu Ser Asp Lys Glu Glu Leu Val Gly Ala Glu Leu Arg Leu Phe
       530                 535                 540
   Arg Gln Ala Pro Ser Ala Pro Trp Gly Pro Pro Ala Gly Pro Leu His
   545                 550                 555                 560
   Val Gln Leu Phe Pro Cys Leu Ser Pro Leu Leu Leu Asp Ala Arg Thr
                   565                 570                 575
   Leu Asp Pro Gln Gly Ala Pro Pro Ala Gly Trp Glu Val Phe Asp Val
               580                 585                 590
   Trp Gln Gly Leu Arg His Gln Pro Trp Lys Gln Leu Cys Leu Glu Leu
           595                 600                 605
   Arg Ala Ala Trp Gly Glu Leu Asp Ala Gly Glu Ala Glu Ala Arg Ala
       610                 615                 620
   Arg Gly Pro Gln Gln Pro Pro Pro Pro Asp Leu Arg Ser Leu Gly Phe
   625                 630                 635                 640
   Gly Arg Arg Val Arg Pro Pro Gln Glu Arg Ala Leu Leu Val Val Phe
                   645                 650                 655
   Thr Arg Ser Gln Arg Lys Asn Leu Phe Ala Glu Met Arg Glu Gln Leu
   660 665 670
   Gly Ser Ala Glu Ala Ala Gly Pro Gly Ala Gly Ala Glu Gly Ser Trp
   675 680 685
   Pro Pro Pro Ser Gly Ala Pro Asp Ala Arg Pro Trp Leu Pro Ser Pro
   690 695 700
   Gly Arg Arg Arg Arg Arg Thr Ala Phe Ala Ser Arg His Gly Lys Arg
   705 710 715 720
   His Gly Lys Lys Ser Arg Leu Arg Cys Ser Lys Lys Pro Leu His Val
   725 730 735
   Asn Phe Lys Glu Leu Gly Trp Asp Asp Trp Ile Ile Ala Pro Leu Glu
   740 745 750
   Tyr Glu Ala Tyr His Cys Glu Gly Val Cys Asp Phe Pro Leu Arg Ser
   755 760 765
   His Leu Glu Pro Thr Asn His Ala Ile Ile Gln Thr Leu Met Asn Ser
   770 775 780
   Met Asp Pro Gly Ser Thr Pro Pro Ser Cys Cys Val Pro Thr Lys Leu
   785 790 795 800
   Thr Pro Ile Ser Ile Leu Tyr Ile Asp Ala Gly Asn Asn Val Val Tyr
   805 810 815
   Lys Gln Tyr Glu Asp Met Val Val Glu Ser Cys Gly Cys Arg
   820 825 830
<210> 39
   <211> 1179
   <212> DNA
   <213> Homo sapiens
<400> 39
   atggtcctcc tcagtattct tagaattctt tttctttgtg aactcgtgct tttcatggaa 60
   cacagggccc aaatggcaga aggagggcag tcctctattg cccttctggc tgaggcccct 120
   actttgcccc tgattgagga gctgctagaa gaatcccctg gcgaacagcc aaggaagccc 180
   cggctcctag ggcattcact gcggtacatg ctggagttgt accggcgttc agctgactcg 240
   catgggcacc ctagagagaa ccgcaccatt ggggccacca tggtgaggct ggtgaagccc 300
   ttgaccagtg tggcaaggcc tcacagaggt acctggcata tacagatcct gggctttcct 360
   ctcagaccaa accgaggact ataccaacta gttagagcca ctgtggttta ccgccatcat 420
   ctccaactaa ctcgcttcaa tctctcctgc catgtggagc cctgggtgca gaaaaaccca 480
   accaaccact tcccttcctc agaaggagat tcctcaaaac cttccctgat gtctaacgct 540
   tggaaagaga tggatatcac acaacttgtt cagcaaaggt tctggaataa caagggacac 600
   aggatcctac gactccgttt tatgtgtcag cagcaaaaag atagtggtgg tcttgagctc 660
   tggcatggca cttcatcctt ggacattgcc ttcttgttac tctatttcaa tgatactcat 720
   aaaagcattc ggaaggctaa atttcttccc aggggcatgg aggagttcat ggaaagggaa 780
   tctcttctcc ggagaacccg acaagcagat ggtatctcag ctgaggttac tgcctcttcc 840
   tcaaaacata gcgggcctga aaataaccag tgttccctcc accctttcca aatcagcttc 900
   cgccagctgg gttgggatca ctggatcatt gctcccccct tctacacccc aaactactgt 960
   aaaggaactt gtctccgagt actacgcgat ggtctcaatt cccccaatca cgccattatt 1020
   cagaacctta tcaatcagtt ggtggaccag agtgtccccc ggccctcctg tgtcccgtat 1080
   aagtatgttc caattagtgt ccttatgatt gaggcaaatg ggagtatttt gtacaaggag 1140
   tatgagggta tgattgctga gtcttgtaca tgcagatga 1179
<210> 40
   <211> 392
   <212> PRT
   <213> Homo sapiens
<400> 40
   Met Val Leu Leu Ser Ile Leu Arg Ile Leu Phe Leu Cys Glu Leu Val
   1               5                   10                  15
   Leu Phe Met Glu His Arg Ala Gln Met Ala Glu Gly Gly Gln Ser Ser
               20                  25                  30
   Ile Ala Leu Leu Ala Glu Ala Pro Thr Leu Pro Leu Ile Glu Glu Leu
           35                  40                  45
   Leu Glu Glu Ser Pro Gly Glu Gln Pro Arg Lys Pro Arg Leu Leu Gly
   50 55 60
   His Ser Leu Arg Tyr Met Leu Glu Leu Tyr Arg Arg Ser Ala Asp Ser
   65 70 75 80
   His Gly His Pro Arg Glu Asn Arg Thr Ile Gly Ala Thr Met Val Arg
   85 90 95
   Leu Val Lys Pro Leu Thr Ser Val Ala Arg Pro His Arg Gly Thr Trp
   100 105 110
   His Ile Gln Ile Leu Gly Phe Pro Leu Arg Pro Asn Arg Gly Leu Tyr
   115 120 125
   Gln Leu Val Arg Ala Thr Val Val Tyr Arg His His Leu Gln Leu Thr
   130 135 140
   Arg Phe Asn Leu Ser Cys His Val Glu Pro Trp Val Gln Lys Asn Pro
   145 150 155 160
   Thr Asn His Phe Pro Ser Ser Glu Gly Asp Ser Ser Lys Pro Ser Leu
   165 170 175
   Met Ser Asn Ala Trp Lys Glu Met Asp Ile Thr Gln Leu Val Gln Gln
   180 185 190
   Arg Phe Trp Asn Asn Lys Gly His Arg Ile Leu Arg Leu Arg Phe Met
   195 200 205
   Cys Gln Gln Gln Lys Asp Ser Gly Gly Leu Glu Leu Trp His Gly Thr
   210 215 220
   Ser Ser Leu Asp Ile Ala Phe Leu Leu Leu Tyr Phe Asn Asp Thr His
   225 230 235 240
   Lys Ser Ile Arg Lys Ala Lys Phe Leu Pro Arg Gly Met Glu Glu Phe
   245 250 255
   Met Glu Arg Glu Ser Leu Leu Arg Arg Thr Arg Gln Ala Asp Gly Ile
   260 265 270
   Ser Ala Glu Val Thr Ala Ser Ser Ser Lys His Ser Gly Pro Glu Asn
   275 280 285
   Asn Gln Cys Ser Leu His Pro Phe Gln Ile Ser Phe Arg Gln Leu Gly
   290 295 300
   Trp Asp His Trp Ile Ile Ala Pro Pro Phe Tyr Thr Pro Asn Tyr Cys
   305 310 315 320
   Lys Gly Thr Cys Leu Arg Val Leu Arg Asp Gly Leu Asn Ser Pro Asn
   325 330 335
   His Ala Ile Ile Gln Asn Leu Ile Asn Gln Leu Val Asp Gln Ser Val
   340 345 350
   Pro Arg Pro Ser Cys Val Pro Tyr Lys Tyr Val Pro Ile Ser Val Leu
   355 360 365
   Met Ile Glu Ala Asn Gly Ser Ile Leu Tyr Lys Glu Tyr Glu Gly Met
   370 375 380
   Ile Ala Glu Ser Cys Thr Cys Arg
   385 390
<210> 41
   <211> 375
   <212> DNA
   <213> Homo sapiens
<400> 41
   caagcagatg gtatctcagc tgaggttact gcctcttcct caaaacatag cgggcctgaa 60
   aataaccagt gttccctcca ccctttccaa atcagcttcc gccagctggg ttgggatcac 120
   tggatcattg ctcccccttt ctacacccca aactactgta aaggaacttg tctccgagta 180
   ctacgcgatg gtctcaattc ccccaatcac gccattattc agaaccttat caatcagttg 240
   gtggaccaga gtgtcccccg gccctcctgt gtcccgtata agtatgttcc aattagtgtc 300
   cttatgattg aggcaaatgg gagtattttg tacaaggagt atgagggtat gattgctgag 360
   tcttgtacat gcaga 375
<210> 42
   <211> 125
   <212> PRT
   <213> Homo sapiens
<400> 42
   Gln Ala Asp Gly Ile Ser Ala Glu Val Thr Ala Ser Ser Ser Lys His
   1 5 10 15
   Ser Gly Pro Glu Asn Asn Gln Cys Ser Leu His Pro Phe Gln Ile Ser
   20 25 30
   Phe Arg Gln Leu Gly Trp Asp His Trp Ile Ile Ala Pro Pro Phe Tyr
   35 40 45
   Thr Pro Asn Tyr Cys Lys Gly Thr Cys Leu Arg Val Leu Arg Asp Gly
   50 55 60
   Leu Asn Ser Pro Asn His Ala Ile Ile Gln Asn Leu Ile Asn Gln Leu
   65 70 75 80
   Val Asp Gln Ser Val Pro Arg Pro Ser Cys Val Pro Tyr Lys Tyr Val
   85 90 95
   Pro Ile Ser Val Leu Met Ile Glu Ala Asn Gly Ser Ile Leu Tyr Lys
   100 105 110
   Glu Tyr Glu Gly Met Ile Ala Glu Ser Cys Thr Cys Arg
   115 120 125
<210> 43
   <211> 1744
   <212> DNA
   <213> Homo sapiens
<400> 43
   atgcacgccc actgcctgcc cttccttctg cacgcctggt gggccctact ccaggcgggt 60
   gctgcgacgg tggccactgc gctcctgcgt acgcgggggc agccctcgtc gccatcccct 120
   ctggcgtaca tgctgagcct ctaccgcgac ccgctgccga gggcagacat catccgcagc 180
   ctacaggcag aagatgtggc agtggatggg cagaactgga cgtttgcttt tgacttctcc 240
   ttcctgagcc aacaagagga tctggcatgg gctgagctcc ggctgcagct gtccagccct 300
   gtggacctcc ccactgaggg ctcacttgcc attgagattt tccaccagcc aaagcccgac 360
   acagagcagg cttcagacag ctgcttagag cggtttcaga tggacctatt cactgtcact    420
   ttgtcccagg tcaccttttc cttgggcagc atggttttgg aggtgaccag gcctctctcc    480
   aagtggctga agcgccctgg ggccctggag aagcagatgt ccagggtagc tggagagtgc    540
   tggccgcggc cccccacacc gcctgccacc aatgtgctcc ttatgctcta ctccaacctc    600
   tcgcaggagc agaggcagct gggtgggtcc accttgctgt gggaagccga gagctcctgg    660
   cgggcccagg agggacagct gtcctgggag tggggcaaga ggcaccgtcg acatcacttg    720
   ccagacagaa gtcaactgtg tcggaaggtc aagttccagg tggacttcaa cctgatcgga    780
   tggggctcct ggatcatcta ccccaagcag tacaacgcct atcgctgtga gggcgagtgt    840
   cctaatcctg ttggggagga gtttcatccg accaaccatg catacatcca gagtctgctg    900
   aaacgttacc agccccaccg agtcccttcc acttgttgtg ccccagtgaa gaccaagccg    960
   ctgagcatgc tgtatgtgga taatggcaga gtgctcctag atcaccataa agacatgatc   1020
   gtggaagaat gtgggtgcct ctgatgacat cctggaggga gactggattt gcctgcactc   1080
   tggaaggctg ggaaactcct ggaagacatg ataaccatct aatccagtaa ggagaaacag   1140
   agaggggcaa agttgctctg cccaccagaa ctgaagagga ggggctgccc actctgtaaa   1200
   tgaagggctc agtggagtct ggccaagcac agaggctgct gtcaggaaga gggaggaaga   1260
   agcctgtgca gggggctggc tggatgttct ctttactgaa aagacagtgg caaggaaaag   1320
   cacaagtgca tgagttcttt actggatttt ttaaaaacct gtgaaccccc cgaaactgta   1380
   tgtgaaagtt gagacatatg tgcatgtatt ttggaggtgg gatgaagtca cctatagctt   1440
   tcatgtattc tccaaagtag tctgtgtgtg acctgtcccc ctccccaaag attaaggatc   1500
   actgtataga ttaaaaagag tccgtcaatc tcattgcctc aggctgggtt gggggagccc   1560
   cacagctttc tggctggcca gtggcaatct actggccttg tccagaggct cactggagtg   1620
   gttctctgct aatgagctgt acaacaataa agccattgtc tagttaaaaa aaaaaaaaaa   1680
   aaaaaaaaaa aaaaaaaaaa aaaaaaaaaa aaaaaaaaaa aaaaaaaaaa aaaaaaaaaa   1740
   aaaa                                                                1744
<210> 44
   <211> 347
   <212> PRT
   <213> Homo sapiens
<400> 44
   Met His Ala His Cys Leu Pro Phe Leu Leu His Ala Trp Trp Ala Leu
   1 5 10 15
   Leu Gln Ala Gly Ala Ala Thr Val Ala Thr Ala Leu Leu Arg Thr Arg
   20 25 30
   Gly Gln Pro Ser Ser Pro Ser Pro Leu Ala Tyr Met Leu Ser Leu Tyr
   35 40 45
   Arg Asp Pro Leu Pro Arg Ala Asp Ile Ile Arg Ser Leu Gln Ala Glu
   50 55 60
   Asp Val Ala Val Asp Gly Gln Asn Trp Thr Phe Ala Phe Asp Phe Ser
   65 70 75 80
   Phe Leu Ser Gln Gln Glu Asp Leu Ala Trp Ala Glu Leu Arg Leu Gln
   85 90 95
   Leu Ser Ser Pro Val Asp Leu Pro Thr Glu Gly Ser Leu Ala Ile Glu
   100 105 110
   Ile Phe His Gln Pro Lys Pro Asp Thr Glu Gln Ala Ser Asp Ser Cys
   115 120 125
   Leu Glu Arg Phe Gln Met Asp Leu Phe Thr Val Thr Leu Ser Gln Val
   130 135 140
   Thr Phe Ser Leu Gly Ser Met Val Leu Glu Val Thr Arg Pro Leu Ser
   145 150 155 160
   Lys Trp Leu Lys Arg Pro Gly Ala Leu Glu Lys Gln Met Ser Arg Val
   165 170 175
   Ala Gly Glu Cys Trp Pro Arg Pro Pro Thr Pro Pro Ala Thr Asn Val
   180 185 190
   Leu Leu Met Leu Tyr Ser Asn Leu Ser Gln Glu Gln Arg Gln Leu Gly
   195 200 205
   Gly Ser Thr Leu Leu Trp Glu Ala Glu Ser Ser Trp Arg Ala Gln Glu
   210 215 220
   Gly Gln Leu Ser Trp Glu Trp Gly Lys Arg His Arg Arg His His Leu
   225 230 235 240
   Pro Asp Arg Ser Gln Leu Cys Arg Lys Val Lys Phe Gln Val Asp Phe
   245 250 255
   Asn Leu Ile Gly Trp Gly Ser Trp Ile Ile Tyr Pro Lys Gln Tyr Asn
   260 265 270
   Ala Tyr Arg Cys Glu Gly Glu Cys Pro Asn Pro Val Gly Glu Glu Phe
   275 280 285
   His Pro Thr Asn His Ala Tyr Ile Gln Ser Leu Leu Lys Arg Tyr Gln
   290 295 300
   Pro His Arg Val Pro Ser Thr Cys Cys Ala Pro Val Lys Thr Lys Pro
   305 310 315 320
   Leu Ser Met Leu Tyr Val Asp Asn Gly Arg Val Leu Leu Asp His His
   325 330 335
   Lys Asp Met Ile Val Glu Glu Cys Gly Cys Leu
   340 345
<210> 45
   <211> 504
   <212> DNA
   <213> Homo sapiens
<400> 45
   tggccgcggc cccccacacc gcctgccacc aatgtgctcc ttatgctcta ctccaacctc 60
   tcgcaggagc agaggcagct gggtgggtcc accttgctgt gggaagccga gagctcctgg 120
   cgggcccagg agggacagct gtcctgggag tggggcaaga ggcaccgtcg acatcacttg 180
   ccagacagaa gtcaactgtg tcggaaggtc aagttccagg tggacttcaa cctgatcgga 240
   tggggctcct ggatcatcta ccccaagcag tacaacgcct atcgctgtga gggcgagtgt 300
   cctaatcctg ttggggagga gtttcatccg accaaccatg catacatcca gagtctgctg 360
   aaacgttacc agccccaccg agtcccttcc acttgttgtg ccccagtgaa gaccaagccg 420
   ctgagcatgc tgtatgtgga taatggcaga gtgctcctag atcaccataa agacatgatc     480
   gtggaagaat gtgggtgcct ctga                                            504
<210> 46
   <211> 167
   <212> PRT
   <213> Homo sapiens
<400> 46
   Trp Pro Arg Pro Pro Thr Pro Pro Ala Thr Asn Val Leu Leu Met Leu
   1               5                   10                  15
   Tyr Ser Asn Leu Ser Gln Glu Gln Arg Gln Leu Gly Gly Ser Thr Leu
               20                  25                  30
   Leu Trp Glu Ala Glu Ser Ser Trp Arg Ala Gln Glu Gly Gln Leu Ser
           35                  40                  45
   Trp Glu Trp Gly Lys Arg His Arg Arg His His Leu Pro Asp Arg Ser
       50                  55                  60
   Gln Leu Cys Arg Lys Val Lys Phe Gln Val Asp Phe Asn Leu Ile Gly
   65                  70                  75                  80
   Trp Gly Ser Trp Ile Ile Tyr Pro Lys Gln Tyr Asn Ala Tyr Arg Cys
                   85                  90                  95
   Glu Gly Glu Cys Pro Asn Pro Val Gly Glu Glu Phe His Pro Thr Asn
               100                 105                 110
   His Ala Tyr Ile Gln Ser Leu Leu Lys Arg Tyr Gln Pro His Arg Val
           115                 120                 125
   Pro Ser Thr Cys Cys Ala Pro Val Lys Thr Lys Pro Leu Ser Met Leu
       130                 135                 140
   Tyr Val Asp Asn Gly Arg Val Leu Leu Asp His His Lys Asp Met Ile
   145                 150                 155                 160
   Val Glu Glu Cys Gly Cys Leu
                   165
<210> 47
   <211> 1101
   <212> DNA
   <213> Homo sapiens
<400> 47
   atgcagcccc tgtggctctg ctgggcactc tgggtgttgc ccctggccag ccccggggcc     60
   gccctgaccg gggagcagct cctgggcagc ctgctgcggc agctgcagct caaagaggtg   120
   cccaccctgg acagggccga catggaggag ctggtcatcc ccacccacgt gagggcccag   180
   tacgtggccc tgctgcagcg cagccacggg gaccgctccc gcggaaagag gttcagccag   240
   agcttccgag aggtggccgg caggttcctg gcgttggagg ccagcacaca cctgctggtg   300
   ttcggcatgg agcagcggct gccgcccaac agcgagctgg tgcaggccgt gctgcggctc   360
   ttccaggagc cggtccccaa ggccgcgctg cacaggcacg ggcggctgtc cccgcgcagc   420
   gcccgggccc gggtgaccgt cgagtggctg cgcgtccgcg acgacggctc caaccgcacc   480
   tccctcatcg actccaggct ggtgtccgtc cacgagagcg gctggaaggc cttcgacgtg   540
   accgaggccg tgaacttctg gcagcagctg agccggcccc ggcagccgct gctgctacag   600
   gtgtcggtgc agagggagca tctgggcccg ctggcgtccg gcgcccacaa gctggtccgc   660
   tttgcctcgc agggggcgcc agccgggctt ggggagcccc agctggagct gcacaccctg   720
   gaccttgggg actatggagc tcagggcgac tgtgaccctg aagcaccaat gaccgagggc   780
   acccgctgct gccgccagga gatgtacatt gacctgcagg ggatgaagtg ggccgagaac   840
   tgggtgctgg agcccccggg cttcctggct tatgagtgtg tgggcacctg ccggcagccc   900
   ccggaggccc tggccttcaa gtggccgttt ctggggcctc gacagtgcat cgcctcggag   960
   actgcctcgc tgcccatgat cgtcagcatc aaggagggag gcaggaccag gccccaggtg  1020
   gtcagcctgc ccaacatgag ggtgcagaag tgcagctgtg cctcggatgg tgcgctcgtg  1080
   ccaaggaggc tccagccata g                                            1101
<210> 48
   <211> 366
   <212> PRT
   <213> Homo sapiens
<400> 48
<210> 49
   <211> 1098
   <212> DNA
   <213> Homo sapiens
<400> 49
   atgtggcccc tgtggctctg ctgggcactc tgggtgctgc cctggctggg ccccggggcg 60
   gccctgaccg aggagcagct cctgggcagc ctgctgcggc agctgcagct cagcgaggtg 120
   cccgtactgg acagggccga catggagaag ctggtcatcc ccgcccacgt gagggcccag 180
   tatgtagtcc tgctgcggcg cagccacggg gaccgctccc gcggaaagag gttcagccag 240
   agcttccgag aggtggccgg caggttcctg gcgtcggagg ccagcacaca cctgctggtg 300
   ttcggcatgg agcagcggct gccgcccaac agcgagctgg tgcaggccgt gctgcggctc 360
   ttccaggagc cggtccccaa ggccgcgctg cacaggcacg ggcggctgtc cccgcgcagc 420
   gcccaggccc gggtgaccgt cgagtggctg cgcgtccgcg acgacggctc caaccgcacc 480
   tccctcatcg actccaggct ggtgtccgtc cacgagagcg gctggaaggc cttcgacgtg 540
   accgaggccg tgaacttctg gcagcagctg agccggcccc ggcagccgct gctgctacag 600
   gtgtcggtgc agagggagca tctgggcccg ctggcgtccg gcgcccacaa gctggtccgc 660
   tttgcctcgc agggggcgcc agccgggctt ggggagcccc agctggagct gcacaccctg 720
   gacctcaggg actatggagc tcagggcgac tgtgaccctg aagcaccaat gaccgagggc 780
   acccgctgct gccgccagga gatgtacatt gacctgcagg ggatgaagtg ggccaagaac 840
   tgggtgctgg agcccccggg cttcctggct tacgagtgtg tgggcacctg ccagcagccc 900
   ccggaggccc tggccttcaa ttggccattt ctggggccgc gacagtgtat cgcctcggag 960
   actgcctcgc tgcccatgat cgtcagcatc aaggagggag gcaggaggcc ccaggtggtc 1020
   agcctgccca acatgagggt gcagaagtgc agctgtgcct cggatggggc gctcgtgcca 1080
   aggaggctcc agccatag 1098
<210> 50
   <211> 365
   <212> PRT
   <213> Homo sapiens
<400> 50
   Met Trp Pro Leu Trp Leu Cys Trp Ala Leu Trp Val Leu Pro Leu Ala
   1 5 10 15
   Gly Pro Gly Ala Ala Leu Thr Glu Glu Gln Leu Leu Gly Ser Leu Leu
   20 25 30
   Arg Gln Leu Gln Leu Ser Glu Val Pro Val Leu Asp Arg Ala Asp Met
   35 40 45
   Glu Lys Leu Val Ile Pro Ala His Val Arg Ala Gln Tyr Val Val Leu
   50 55 60
   Leu Arg Arg Ser His Gly Asp Arg Ser Arg Gly Lys Arg Phe Ser Gln
   65 70 75 80
   Ser Phe Arg Glu Val Ala Gly Arg Phe Leu Ala Ser Glu Ala Ser Thr
   85 90 95
   His Leu Leu Val Phe Gly Met Glu Gln Arg Leu Pro Pro Asn Ser Glu
   100 105 110
   Leu Val Gln Ala Val Leu Arg Leu Phe Gln Glu Pro Val Pro Lys Ala
   115 120 125
   Ala Leu His Arg His Gly Arg Leu Ser Pro Arg Ser Ala Gln Ala Arg
   130 135 140
   Val Thr Val Glu Trp Leu Arg Val Arg Asp Asp Gly Ser Asn Arg Thr
   145 150 155 160
   Ser Leu Ile Asp Ser Arg Leu Val Ser Val His Glu Ser Gly Trp Lys
   165 170 175
   Ala Phe Asp Val Thr Glu Ala Val Asn Phe Trp Gln Gln Leu Ser Arg
   180 185 190
   Pro Arg Gln Pro Leu Leu Leu Gln Val Ser Val Gln Arg Glu His Leu
   195 200 205
   Gly Pro Leu Ala Ser Gly Ala His Lys Leu Val Arg Phe Ala Ser Gln
   210 215 220
   Gly Ala Pro Ala Gly Leu Gly Glu Pro Gln Leu Glu Leu His Thr Leu
   225 230 235 240
   Asp Leu Arg Asp Tyr Gly Ala Gln Gly Asp Cys Asp Pro Glu Ala Pro
   245 250 255
   Met Thr Glu Gly Thr Arg Cys Cys Arg Gln Glu Met Tyr Ile Asp Leu
   260 265 270
   Gln Gly Met Lys Trp Ala Lys Asn Trp Val Leu Glu Pro Pro Gly Phe
   275 280 285
   Leu Ala Tyr Glu Cys Val Gly Thr Cys Gln Gln Pro Pro Glu Ala Leu
   290 295 300
   Ala Phe Asn Trp Pro Phe Leu Gly Pro Arg Gln Cys Ile Ala Ser Glu
   305 310 315 320
   Thr Ala Ser Leu Pro Met Ile Val Ser Ile Lys Glu Gly Gly Arg Arg
   325 330 335
   Pro Gln Val Val Ser Leu Pro Asn Met Arg Val Gln Lys Cys Ser Cys
   340 345 350
   Ala Ser Asp Gly Ala Leu Val Pro Arg Arg Leu Gln Pro
   355 360 365
<210> 51
   <211> 2291
   <212> DNA
   <213> Homo sapiens
<400> 51
   ctttccgccc cagccctgaa agcgttaacc ctggagcttt ctgcacaccc cccgaccgct     60
   cccgcccaag cttcctaaaa aagaaaggtg caaagtttgg tccaggatag aaaaatgact    120
   gatcaaaggc aggcgatact tcctgttgcc gggacgctat atataacgtg atgagcgcac    180
   gggctgcgga gacgcaccgg agcgctcgcc cagccgccgc ctccaagccc ctgaggtttc    240
   cggggaccac aatgaacaag ttgctgtgct gcgcgctcgt gtttctggac atctccatta    300
   agtggaccac ccaggaaacg tttcctccaa agtaccttca ttatgacgaa gaaacctctc    360
   atcagctgtt gtgtgacaaa tgtcctcctg gtacctacct aaaacaacac tgtacagcaa    420
   agtggaagac cgtgtgcgcc ccttgccctg accactacta cacagacagc tggcacacca    480
   gtgacgagtg tctatactgc agccccgtgt gcaaggagct gcagtacgtc aagcaggagt    540
   gcaatcgcac ccacaaccgc gtgtgcgaat gcaaggaagg gcgctacctt gagatagagt    600
   tctgcttgaa acataggagc tgccctcctg gatttggagt ggtgcaagct ggaaccccag    660
   agcgaaatac agtttgcaaa agatgtccag atgggttctt ctcaaatgag acgtcatcta    720
   aagcaccctg tagaaaacac acaaattgca gtgtctttgg tctcctgcta actcagaaag    780
   gaaatgcaac acacgacaac atatgttccg gaaacagtga atcaactcaa aaatgtggaa    840
   tagatgttac cctgtgtgag gaggcattct tcaggtttgc tgttcctaca aagtttacgc    900
   ctaactggct tagtgtcttg gtagacaatt tgcctggcac caaagtaaac gcagagagtg    960
   tagagaggat aaaacggcaa cacagctcac aagaacagac tttccagctg ctgaagttat   1020
   ggaaacatca aaacaaagac caagatatag tcaagaagat catccaagat attgacctct   1080
   gtgaaaacag cgtgcagcgg cacattggac atgctaacct caccttcgag cagcttcgta    1140
   gcttgatgga aagcttaccg ggaaagaaag tgggagcaga agacattgaa aaaacaataa    1200
   aggcatgcaa acccagtgac cagatcctga agctgctcag tttgtggcga ataaaaaatg    1260
   gcgaccaaga caccttgaag ggcctaatgc acgcactaaa gcactcaaag acgtaccact    1320
   ttcccaaaac tgtcactcag agtctaaaga agaccatcag gttccttcac agcttcacaa    1380
   tgtacaaatt gtatcagaag ttatttttag aaatgatagg taaccaggtc caatcagtaa    1440
   aaataagctg cttataactg gaaatggcca ttgagctgtt tcctcacaat tggcgagatc    1500
   ccatggatga gtaaactgtt tctcaggcac ttgaggcttt cagtgatatc tttctcatta    1560
   ccagtgacta attttgccac agggtactaa aagaaactat gatgtggaga aaggactaac    1620
   atctcctcca ataaacccca aatggttaat ccaactgtca gatctggatc gttatctact    1680
   gactatattt tcccttatta ctgcttgcag taattcaact ggaaattaaa aaaaaaaaac    1740
   tagactccat tgtgccttac taaatatggg aatgtctaac ttaaatagct ttgagatttc    1800
   agctatgcta gaggctttta ttagaaagcc atattttttt ctgtaaaagt tactaatata    1860
   tctgtaacac tattacagta ttgctattta tattcattca gatataagat ttgtacatat    1920
   tatcatccta taaagaaacg gtatgactta attttagaaa gaaaattata ttctgtttat    1980
   tatgacaaat gaaagagaaa atatatattt ttaatggaaa gtttgtagca tttttctaat    2040
   aggtactgcc atatttttct gtgtggagta tttttataat tttatctgta taagctgtaa    2100
   tatcatttta tagaaaatgc attatttagt caattgttta atgttggaaa acatatgaaa    2160
   tataaattat ctgaatatta gatgctctga gaaattgaat gtaccttatt taaaagattt    2220
   tatggtttta taactatata aatgacatta ttaaagtttt caaattattt tttaaaaaaa    2280
   aaaaaaaaaa a                                                         2291
<210> 52
   <211> 401
   <212> PRT
   <213> Homo sapiens
<400> 52
   Met Asn Lys Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
               180                 185                 190
   Gln Lys Cys Gly Ile Asp Val Thr Leu Cys Glu Glu Ala Phe Phe Arg
           195                 200                 205
   Phe Ala Val Pro Thr Lys Phe Thr Pro Asn Trp Leu Ser Val Leu Val
       210                 215                 220
   Asp Asn Leu Pro Gly Thr Lys Val Asn Ala Glu Ser Val Glu Arg Ile
   225                 230                 235                 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Cys Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Gln Val Gln Ser Val Lys Ile Ser Cys
   385 390 395 400
   Leu
<210> 53
   <211> 1014
   <212> DNA
   <213> Homo sapiens
<400> 53
   ccttgccctg accactacta cacagacagc tggcacacca gtgacgagtg tctatactgc 60
   agccccgtgt gcaaggagct gcagtacgtc aagcaggagt gcaatcgcac ccacaaccgc 120
   gtgtgcgaat gcaaggaagg gcgctacctt gagatagagt tctgcttgaa acataggagc 180
   tgccctcctg gatttggagt ggtgcaagct ggaaccccag agcgaaatac agtttgcaaa 240
   agatgtccag atgggttctt ctcaaatgag acgtcatcta aagcaccctg tagaaaacac 300
   acaaattgca gtgtctttgg tctcctgcta actcagaaag gaaatgcaac acacgacaac 360
   atatgttccg gaaacagtga atcaactcaa aaatgtggaa tagatgttac cctgtgtgag 420
   gaggcattct tcaggtttgc tgttcctaca aagtttacgc ctaactggct tagtgtcttg 480
   gtagacaatt tgcctggcac caaagtaaac gcagagagtg tagagaggat aaaacggcaa 540
   cacagctcac aagaacagac tttccagctg ctgaagttat ggaaacatca aaacaaagac 600
   caagatatag tcaagaagat catccaagat attgacctct gtgaaaacag cgtgcagcgg 660
   cacattggac atgctaacct caccttcgag cagcttcgta gcttgatgga aagcttaccg 720
   ggaaagaaag tgggagcaga agacattgaa aaaacaataa aggcatgcaa acccagtgac 780
   cagatcctga agctgctcag tttgtggcga ataaaaaatg gcgaccaaga caccttgaag 840
   ggcctaatgc acgcactaaa gcactcaaag acgtaccact ttcccaaaac tgtcactcag 900
   agtctaaaga agaccatcag gttccttcac agcttcacaa tgtacaaatt gtatcagaag 960
   ttatttttag aaatgatagg taaccaggtc caatcagtaa aaataagctg ctta 1014
<210> 54
   <211> 338
   <212> PRT
   <213> Homo sapiens
<400> 54
   Pro Cys Pro Asp His Tyr Tyr Thr Asp Ser Trp His Thr Ser Asp Glu
   1 5 10 15
   Cys Leu Tyr Cys Ser Pro Val Cys Lys Glu Leu Gln Tyr Val Lys Gln
   20 25 30
   Glu Cys Asn Arg Thr His Asn Arg Val Cys Glu Cys Lys Glu Gly Arg
   35 40 45
   Tyr Leu Glu Ile Glu Phe Cys Leu Lys His Arg Ser Cys Pro Pro Gly
   50 55 60
   Phe Gly Val Val Gln Ala Gly Thr Pro Glu Arg Asn Thr Val Cys Lys
   65 70 75 80
   Arg Cys Pro Asp Gly Phe Phe Ser Asn Glu Thr Ser Ser Lys Ala Pro
   85 90 95
   Cys Arg Lys His Thr Asn Cys Ser Val Phe Gly Leu Leu Leu Thr Gln
   100 105 110
   Lys Gly Asn Ala Thr His Asp Asn Ile Cys Ser Gly Asn Ser Glu Ser
   115 120 125
   Thr Gln Lys Cys Gly Ile Asp Val Thr Leu Cys Glu Glu Ala Phe Phe
   130 135 140
   Arg Phe Ala Val Pro Thr Lys Phe Thr Pro Asn Trp Leu Ser Val Leu
   145 150 155 160
   Val Asp Asn Leu Pro Gly Thr Lys Val Asn Ala Glu Ser Val Glu Arg
   165 170 175
   Ile Lys Arg Gln His Ser Ser Gln Glu Gln Thr Phe Gln Leu Leu Lys
   180 185 190
   Leu Trp Lys His Gln Asn Lys Asp Gln Asp Ile Val Lys Lys Ile Ile
   195 200 205
   Gln Asp Ile Asp Leu Cys Glu Asn Ser Val Gln Arg His Ile Gly His
   210 215 220
   Ala Asn Leu Thr Phe Glu Gln Leu Arg Ser Leu Met Glu Ser Leu Pro
   225 230 235 240
   Gly Lys Lys Val Gly Ala Glu Asp Ile Glu Lys Thr Ile Lys Ala Cys
   245 250 255
   Lys Pro Ser Asp Gln Ile Leu Lys Leu Leu Ser Leu Trp Arg Ile Lys
   260 265 270
   Asn Gly Asp Gln Asp Thr Leu Lys Gly Leu Met His Ala Leu Lys His
   275 280 285
   Ser Lys Thr Tyr His Phe Pro Lys Thr Val Thr Gln Ser Leu Lys Lys
       290                 295                 300
   Thr Ile Arg Phe Leu His Ser Phe Thr Met Tyr Lys Leu Tyr Gln Lys
   305                 310                 315                 320
   Leu Phe Leu Glu Met Ile Gly Asn Gln Val Gln Ser Val Lys Ile Ser
                   325                 330                 335
   Cys Leu
<210> 55
   <211> 2312
   <212> DNA
   <213> Homo sapiens
<400> 55
   tccagtgacg gagccgcccg gccgacagcc ccgagacgac agcccggcgc gtcccggtcc      60
   ccacctccga ccaccgccag cgctccaggc cccgcgctcc ccgctcgccg ccaccgcgcc     120
   ctccgctccg cccgcagtgc caaccatgac cgccgccagt atgggccccg tccgcgtcgc     180
   cttcgtggtc ctcctcgccc tctgcagccg gccggccgtc ggccagaact gcagcgggcc     240
   gtgccggtgc ccggacgagc cggcgccgcg ctgcccggcg ggcgtgagcc tcgtgctgga     300
   cggctgcggc tgctgccgcg tctgcgccaa gcagctgggc gagctgtgca ccgagcgcga     360
   cccctgcgac ccgcacaagg gcctcttctg tgacttcggc tccccggcca accgcaagat     420
   cggcgtgtgc accgccaaag atggtgctcc ctgcatcttc ggtggtacgg tgtaccgcag     480
   cggagagtcc ttccagagca gctgcaagta ccagtgcacg tgcctggacg gggcggtggg     540
   ctgcatgccc ctgtgcagca tggacgttcg tctgcccagc cctgactgcc ccttcccgag     600
   gagggtcaag ctgcccggga aatgctgcga ggagtgggtg tgtgacgagc ccaaggacca     660
   aaccgtggtt gggcctgccc tcgcggctta ccgactggaa gacacgtttg gcccagaccc     720
   aactatgatt agagccaact gcctggtcca gaccacagag tggagcgcct gttccaagac     780
   ctgtgggatg ggcatctcca cccgggttac caatgacaac gcctcctgca ggctagagaa     840
   gcagagccgc ctgtgcatgg tcaggccttg cgaagctgac ctggaagaga acattaagaa     900
   gggcaaaaag tgcatccgta ctcccaaaat ctccaagcct atcaagtttg agctttctgg     960
   ctgcaccagc atgaagacat accgagctaa attctgtgga gtatgtaccg acggccgatg    1020
   ctgcaccccc cacagaacca ccaccctgcc ggtggagttc aagtgccctg acggcgaggt 1080
   catgaagaag aacatgatgt tcatcaagac ctgtgcctgc cattacaact gtcccggaga 1140
   caatgacatc tttgaatcgc tgtactacag gaagatgtac ggagacatgg catgaagcca 1200
   gagagtgaga gacattaact cattagactg gaacttgaac tgattcacat ctcatttttc 1260
   cgtaaaaatg atttcagtag cacaagttat ttaaatctgt ttttctaact gggggaaaag 1320
   attcccaccc aattcaaaac attgtgccat gtcaaacaaa tagtctatct tccccagaca 1380
   ctggtttgaa gaatgttaag acttgacagt ggaactacat tagtacacag caccagaatg 1440
   tatattaagg tgtggcttta ggagcagtgg gagggtacca gcagaaaggt tagtatcatc 1500
   agatagctct tatacgagta atatgcctgc tatttgaagt gtaattgaga aggaaaattt 1560
   tagcgtgctc actgacctgc ctgtagcccc agtgacagct aggatgtgca ttctccagcc 1620
   atcaagagac tgagtcaagt tgttccttaa gtcagaacag cagactcagc tctgacattc 1680
   tgattcgaat gacactgttc aggaatcgga atcctgtcga ttagactgga cagcttgtgg 1740
   caagtgaatt tcctgtaaca agccagattt tttaaaattt atattgtaaa tattgtgtgt 1800
   gtgtgtgtgt gtgtatatat atatatatat gtacagttat ctaagttaat ttaaagttgt 1860
   ttgtgccttt ttatttttgt ttttaatgct ttgatatttc aatgttagcc tcaatttctg 1920
   aacaccatag gtagaatgta aagcttgtct gatcgttcaa agcatgaaat ggatacttat 1980
   atggaaattc tctcagatag aatgacagtc cgtcaaaaca gattgtttgc aaaggggagg 2040
   catcagtgtc cttggcaggc tgatttctag gtaggaaatg tggtagctca cgctcacttt 2100
   taatgaacaa atggccttta ttaaaaactg agtgactcta tatagctgat cagttttttc 2160
   acctggaagc atttgtttct actttgatat gactgttttt cggacagttt atttgttgag 2220
   agtgtgacca aaagttacat gtttgcacct ttctagttga aaataaagta tattttttct 2280
   aaaaaaaaaa aaaaacgaca gcaacggaat tc 2312
<210> 56
   <211> 349
   <212> PRT
   <213> Homo sapiens
<400> 56
   Met Thr Ala Ala Ser Met Gly Pro Val Arg Val Ala Phe Val Val Leu
   1 5 10 15
   Leu Ala Leu Cys Ser Arg Pro Ala Val Gly Gln Asn Cys Ser Gly Pro
               20                  25                  30
   Cys Arg Cys Pro Asp Glu Pro Ala Pro Arg Cys Pro Ala Gly Val Ser
           35                  40                  45
   Leu Val Leu Asp Gly Cys Gly Cys Cys Arg Val Cys Ala Lys Gln Leu
       50                  55                  60
   Gly Glu Leu Cys Thr Glu Arg Asp Pro Cys Asp Pro His Lys Gly Leu
   65                  70                  75                  80
   Phe Cys Asp Phe Gly Ser Pro Ala Asn Arg Lys Ile Gly Val Cys Thr
                   85                  90                  95
   Ala Lys Asp Gly Ala Pro Cys Ile Phe Gly Gly Thr Val Tyr Arg Ser
               100                 105                 110
   Gly Glu Ser Phe Gln Ser Ser Cys Lys Tyr Gln Cys Thr Cys Leu Asp
           115                 120                 125
   Gly Ala Val Gly Cys Met Pro Leu Cys Ser Met Asp Val Arg Leu Pro
       130                 135                 140
   Ser Pro Asp Cys Pro Phe Pro Arg Arg Val Lys Leu Pro Gly Lys Cys
   145                 150                 155                 160
   Cys Glu Glu Trp Val Cys Asp Glu Pro Lys Asp Gln Thr Val Val Gly
                   165                 170                 175
   Pro Ala Leu Ala Ala Tyr Arg Leu Glu Asp Thr Phe Gly Pro Asp Pro
               180                 185                 190
   Thr Met Ile Arg Ala Asn Cys Leu Val Gln Thr Thr Glu Trp Ser Ala
           195                 200                 205
   Cys Ser Lys Thr Cys Gly Met Gly Ile Ser Thr Arg Val Thr Asn Asp
       210                 215                 220
   Asn Ala Ser Cys Arg Leu Glu Lys Gln Ser Arg Leu Cys Met Val Arg
   225 230 235 240
   Pro Cys Glu Ala Asp Leu Glu Glu Asn Ile Lys Lys Gly Lys Lys Cys
   245 250 255
   Ile Arg Thr Pro Lys Ile Ser Lys Pro Ile Lys Phe Glu Leu Ser Gly
   260 265 270
   Cys Thr Ser Met Lys Thr Tyr Arg Ala Lys Phe Cys Gly Val Cys Thr
   275 280 285
   Asp Gly Arg Cys Cys Thr Pro His Arg Thr Thr Thr Leu Pro Val Glu
   290 295 300
   Phe Lys Cys Pro Asp Gly Glu Val Met Lys Lys Asn Met Met Phe Ile
   305 310 315 320
   Lys Thr Cys Ala Cys His Tyr Asn Cys Pro Gly Asp Asn Asp Ile Phe
   325 330 335
   Glu Ser Leu Tyr Tyr Arg Lys Met Tyr Gly Asp Met Ala
   340 345
<210> 57
   <211> 1069
   <212> DNA
   <213> Homo sapiens
<400> 57
   cagaactgca gcgggccgtg ccggtgcccg gacgagccgg cgccgcgctg cccggcgggc 60
   gtgagcctcg tgctggacgg ctgcggctgc tgccgcgtct gcgccaagca gctgggcgag 120
   ctgtgcaccg agcgcgaccc ctgcgacccg cacaagggcc tcttctgtga cttcggctcc 180
   ccggccaacc gcaagatcgg cgtgtgcacc gccaaagatg gtgctccctg catcttcggt 240
   ggtacggtgt accgcagcgg agagtccttc cagagcagct gcaagtacca gtgcacgtgc 300
   ctggacgggg cggtgggctg catgcccctg tgcagcatgg acgttcgtct gcccagccct 360
   gactgcccct tcccgaggag ggtcaagctg cccgggaaat gctgcgagga gtgggtgtgt 420
   gacgagccca aggaccaaac cgtggttggg cctgccctcg cggcttaccg actggaagac 480
   acgtttggcc cagacccaac tatgattaga gccaactgcc tggtccagac cacagagtgg 540
   agcgcctgtt ccaagacctg tgggatgggc atctccaccc gggttaccaa tgacaacgcc      600
   tcctgcaggc tagagaagca gagccgcctg tgcatggtca ggccttgcga agctgacctg      660
   gaagagaaca ttaagaaggg caaaaagtgc atccgtactc ccaaaatctc caagcctatc      720
   aagtttgagc tttctggctg caccagcatg aagacatacc gagctaaatt ctgtggagta      780
   tgtaccgacg gccgatgctg caccccccac agaaccacca ccctgccggt ggagttcaag      840
   tgccctgacg gcgaggtcat gaagaagaac atgatgttca tcaagacctg tgcctgccat      900
   tacaactgtc ccggagacaa tgacatcttt gaatcgctgt actacaggaa gatgtacgga      960
   gacatggcat gaagccagag agtgagagac attaactcat tagactggaa cttgaactga     1020
   ttcacatctc atttttccgt aaaaatgatt tcagtagcac aagttattt                 1069
<210> 58
   <211> 323
   <212> PRT
   <213> Homo sapiens
<400> 58
   Gln Asn Cys Ser Gly Pro Cys Arg Cys Pro Asp Glu Pro Ala Pro Arg
   1               5                   10                  15
   Cys Pro Ala Gly Val Ser Leu Val Leu Asp Gly Cys Gly Cys Cys Arg
               20                  25                  30
   Val Cys Ala Lys Gln Leu Gly Glu Leu Cys Thr Glu Arg Asp Pro Cys
           35                  40                  45
   Asp Pro His Lys Gly Leu Phe Cys Asp Phe Gly Ser Pro Ala Asn Arg
       50                  55                  60
   Lys Ile Gly Val Cys Thr Ala Lys Asp Gly Ala Pro Cys Ile Phe Gly
   65                  70                  75                  80
   Gly Thr Val Tyr Arg Ser Gly Glu Ser Phe Gln Ser Ser Cys Lys Tyr
                   85                  90                  95
   Gln Cys Thr Cys Leu Asp Gly Ala Val Gly Cys Met Pro Leu Cys Ser
               100                 105                 110
   Met Asp Val Arg Leu Pro Ser Pro Asp Cys Pro Phe Pro Arg Arg Val
   115 120 125
   Lys Leu Pro Gly Lys Cys Cys Glu Glu Trp Val Cys Asp Glu Pro Lys
   130 135 140
   Asp Gln Thr Val Val Gly Pro Ala Leu Ala Ala Tyr Arg Leu Glu Asp
   145 150 155 160
   Thr Phe Gly Pro Asp Pro Thr Met Ile Arg Ala Asn Cys Leu Val Gln
   165 170 175
   Thr Thr Glu Trp Ser Ala Cys Ser Lys Thr Cys Gly Met Gly Ile Ser
   180 185 190
   Thr Arg Val Thr Asn Asp Asn Ala Ser Cys Arg Leu Glu Lys Gln Ser
   195 200 205
   Arg Leu Cys Met Val Arg Pro Cys Glu Ala Asp Leu Glu Glu Asn Ile
   210 215 220
   Lys Lys Gly Lys Lys Cys Ile Arg Thr Pro Lys Ile Ser Lys Pro Ile
   225 230 235 240
   Lys Phe Glu Leu Ser Gly Cys Thr Ser Met Lys Thr Tyr Arg Ala Lys
   245 250 255
   Phe Cys Gly Val Cys Thr Asp Gly Arg Cys Cys Thr Pro His Arg Thr
   260 265 270
   Thr Thr Leu Pro Val Glu Phe Lys Cys Pro Asp Gly Glu Val Met Lys
   275 280 285
   Lys Asn Met Met Phe Ile Lys Thr Cys Ala Cys His Tyr Asn Cys Pro
   290 295 300
   Gly Asp Asn Asp Ile Phe Glu Ser Leu Tyr Tyr Arg Lys Met Tyr Gly
   305 310 315 320
   Asp Met Ala
<210> 59
   <211> 1985
   <212> RNA
   <213> Homo sapiens
<400> 59
   gagcgaaaga cgcccgcccg ccgcccagcc ctcgcctccc tgcccaccgg gcccaccgcg 60
   ccgccacccc gaccccgctg cgcacggcct gtccgctgca caccagcttg ttggcgtctt 120
   cgtcgccgcg ctcgccccgg gctactcctg cgcgccacaa tgagctcccg catcgccagg 180
   gcgctcgcct tagtcgtcac ccttctccac ttgaccaggc tggcgctctc cacctgcccc 240
   gctgcctgcc actgccccct ggaggcgccc aagtgcgcgc cgggagtcgg gctggtccgg 300
   gacggctgcg gctgctgtaa ggtctgcgcc aagcagctca acgaggactg cagcaaaacg 360
   cagccctgcg accacaccaa ggggctggaa tgcaacttcg gcgccagctc caccgctctg 420
   aaggggatct gcagagctca gtcagagggc agaccctgtg aatataactc cagaatctac 480
   caaaacgggg aaagtttcca gcccaactgt aaacatcagt gcacatgtat tgatggcgcc 540
   gtgggctgca ttcctctgtg tccccaagaa ctatctctcc ccaacttggg ctgtcccaac 600
   cctcggctgg tcaaagttac cgggcagtgc tgcgaggagt gggtctgtga cgaggatagt 660
   atcaaggacc ccatggagga ccaggacggc ctccttggca aggagctggg attcgatgcc 720
   tccgaggtgg agttgacgag aaacaatgaa ttgattgcag ttggaaaagg cagctcactg 780
   aagcggctcc ctgtttttgg aatggagcct cgcatcctat acaacccttt acaaggccag 840
   aaatgtattg ttcaaacaac ttcatggtcc cagtgctcaa agacctgtgg aactggtatc 900
   tccacacgag ttaccaatga caaccctgag tgccgccttg tgaaagaaac ccggatttgt 960
   gaggtgcggc cttgtggaca gccagtgtac agcagcctga aaaagggcaa gaaatgcagc 1020
   aagaccaaga aatcccccga accagtcagg tttacttacg ctggatgttt gagtgtgaag 1080
   aaataccggc ccaagtactg cggttcctgc gtggacggcc gatgctgcac gccccagctg 1140
   accaggactg tgaagatgcg gttccgctgc gaagatgggg agacattttc caagaacgtc 1200
   atgatgatcc agtcctgcaa atgcaactac aactgcccgc atgccaatga agcagcgttt 1260
   cccttctaca ggctgttcaa tgacattcac aaatttaggg actaaatgct acctgggttt 1320
   ccagggcaca cctagacaaa caagggagaa gagtgtcaga atcagaatca tggagaaaat 1380
   gggcgggggt ggtgtgggtg atgggactca ttgtagaaag gaagccttgc tcattcttga 1440
   ggagcattaa ggtatttcga aactgccaag ggtgctggtg cggatggaca ctaatgcagc 1500
   cacgattgga gaatactttg cttcatagta ttggagcaca tgttactgct tcattttgga 1560
   gcttgtggag ttgatgactt tctgttttct gtttgtaaat tatttgctaa gcatattttc 1620
   tctaggcttt tttccttttg gggttctaca gtcgtaaaag agataataag attagttgga 1680
   cagtttaaag cttttattcg tcctttgaca aaagtaaatg ggagggcatt ccatcccttc 1740
   ctgaaggggg acactccatg agtgtctgtg agaggcagct atctgcactc taaactgcaa 1800
   acagaaatca ggtgttttaa gactgaatgt tttatttatc aaaatgtagc ttttggggag 1860
   ggaggggaaa tgtaatactg gaataatttg taaatgattt taattttata ttcagtgaaa 1920
   agattttatt tatggaatta accatttaat aaagaaatat ttacctaata aaaaaaaaaa 1980
   aaaaa 1985
<210> 60
   <211> 381
   <212> PRT
   <213> Homo sapiens
<400> 60
<210> 61
   <211> 2830
   <212> DNA
   <213> Homo sapiens
<400> 61
   cccacgcgtc cgctgggccc agctcccccg agaggtggtc ggatcctctg ggctgctcgg 60
   tcgatgcctg tgccactgac gtccaggcat gaggtggttc ctgccctgga cgctggcagc 120
   agtgacagca gcagccgcca gcaccgtcct ggccacggcc ctctctccag cccctacgac 180
   catggacttt actccagctc cactggagga cacctcctca cgcccccaat tctgcaagtg 240
   gccatgtgag tgcccgccat ccccaccccg ctgcccgctg ggggtcagcc tcatcacaga 300
   tggctgtgag tgctgtaaga tgtgcgctca gcagcttggg gacaactgca cggaggctgc 360
   catctgtgac ccccaccggg gcctctactg tgactacagc ggggaccgcc cgaggtacgc 420
   aataggagtg tgtgcacagg tggtcggtgt gggctgcgtc ctggatgggg tgcgctacaa 480
   caacggccag tccttccagc ctaactgcaa gtacaactgc acgtgcatcg acggcgcggt 540
   gggctgcaca ccactgtgcc tccgagtgcg ccccccgcgt ctctggtgcc cccacccgcg 600
   gcgcgtgagc atacctggcc actgctgtga gcagtgggta tgtgaggacg acgccaagag 660
   gccacgcaag accgcacccc gtgacacagg agccttcgat gctgtgggtg aggtggaggc 720
   atggcacagg aactgcatag cctacacaag cccctggagc ccttgctcca ccagctgcgg 780
   cctgggggtc tccactcgga tctccaatgt taacgcccag tgctggcctg agcaagagag 840
   ccgcctctgc aacttgcggc catgcgatgt ggacatccat acactcatta aggcagggaa 900
   gaagtgtctg gctgtgtacc agccagaggc atccatgaac ttcacacttg cgggctgcat 960
   cagcacacgc tcctatcaac ccaagtactg tggagtttgc atggacaata ggtgctgcat 1020
   cccctacaag tctaagacta tcgacgtgtc cttccagtgt cctgatgggc ttggcttctc 1080
   ccgccaggtc ctatggatta atgcctgctt ctgtaacctg agctgtagga atcccaatga 1140
   catctttgct gacttggaat cctaccctga cttctcagaa attgccaact aggcaggcac 1200
   aaatcttggg tcttggggac taacccaatg cctgtgaagc agtcagccct tatggccaat 1260
   aacttttcac caatgagcct tagttaccct gatctggacc cttggcctcc atttctgtct 1320
   ctaaccattc aaatgacgcc tgatggtgct gctcaggccc atgctatgag ttttctcctt 1380
   gatatcattc agcatctact ctaaagaaaa atgcctgtct ctagctgttc tggactacac 1440
   ccaagcctga tccagccttt ccaagtcact agaagtcctg ctggatcttg cctaaatccc 1500
   aagaaatgga atcaggtaga cttttaatat cactaatttc ttctttagat gccaaaccac 1560
   aagactcttt gggtccattc agatgaatag atggaatttg gaacaataga ataatctatt 1620
   atttggagcc tgccaagagg tactgtaatg ggtaattctg acgtcagcgc accaaaacta 1680
   tcctgattcc aaatatgtat gcacctcaag gtcatcaaac atttgccaag tgagttgaat 1740
   agttgcttaa ttttgatttt taatggaaag ttgtatccat taacctgggc attgttgagg 1800
   ttaagtttct cttcacccct acactgtgaa gggtacagat taggtttgtc ccagtcagaa 1860
   ataaaatttg ataaacattc ctgttgatgg gaaaagcccc cagttaatac tccagagaca 1920
   gggaaaggtc agcccatttc agaaggacca attgactctc acactgaatc agctgctgac 1980
   tggcagggct ttgggcagtt ggccaggctc ttccttgaat cttctccctt gtcctgcttg 2040
   ggttcatagg aattggtaag gcctctggac tggcctgtct ggcccctgag agtggtgccc 2100
   tggaacactc ctctactctt acagagcctt gagagaccca gctgcagacc atgccagacc 2160
   cactgaaatg accaagacag gttcaggtag gggtgtgggt caaaccaaga agtgggtgcc 2220
   cttggtagca gcctggggtg acctctagag ctggaggctg tgggactcca ggggcccccg 2280
   tgttcaggac acatctattg cagagactca tttcacagcc tttcgttctg ctgaccaaat 2340
   ggccagtttt ctggtaggaa gatggaggtt taccagttgt ttagaaacag aaatagactt 2400
   aataaaggtt taaagctgaa gaggttgaag ctaaaaggaa aaggttgttg ttaatgaata 2460
   tcaggctatt atttattgta ttaggaaaat ataatattta ctgttagaat tcttttattt 2520
   agggcctttt ctgtgccaga cattgctctc agtgctttgc atgtattagc tcactgaatc 2580
   ttcacgacaa tgttgagaag ttcccattat tatttctgtt cttacaaatg tgaaacggaa 2640
   gctcatagag gtgagaaaac tcaaccagag tcacccagtt ggtgactggg aaagttagga 2700
   ttcagatcga aattggactg tctttataac ccatattttc cccctgtttt tagagcttcc 2760
   aaatgtgtca gaataggaaa acattgcaat aaatggcttg attttttaaa aaaaaaaaaa 2820
   aaaaaaaaaa 2830
<210> 62
   <211> 367
   <212> PRT
   <213> Homo sapiens
<400> 62
   Met Arg Trp Phe Leu Pro Trp Thr Leu Ala Ala Val Thr Ala Ala Ala
   1 5 10 15
   Ala Ser Thr Val Leu Ala Thr Ala Leu Ser Pro Ala Pro Thr Thr Met
   20 25 30
   Asp Phe Thr Pro Ala Pro Leu Glu Asp Thr Ser Ser Arg Pro Gln Phe
   35 40 45
   Cys Lys Trp Pro Cys Glu Cys Pro Pro Ser Pro Pro Arg Cys Pro Leu
   50 55 60
   Gly Val Ser Leu Ile Thr Asp Gly Cys Glu Cys Cys Lys Met Cys Ala
   65 70 75 80
   Gln Gln Leu Gly Asp Asn Cys Thr Glu Ala Ala Ile Cys Asp Pro His
   85 90 95
   Arg Gly Leu Tyr Cys Asp Tyr Ser Gly Asp Arg Pro Arg Tyr Ala Ile
   100 105 110
   Gly Val Cys Ala Gln Val Val Gly Val Gly Cys Val Leu Asp Gly Val
   115 120 125
   Arg Tyr Asn Asn Gly Gln Ser Phe Gln Pro Asn Cys Lys Tyr Asn Cys
   130 135 140
   Thr Cys Ile Asp Gly Ala Val Gly Cys Thr Pro Leu Cys Leu Arg Val
   145 150 155 160
   Arg Pro Pro Arg Leu Trp Cys Pro His Pro Arg Arg Val Ser Ile Pro
   165 170 175
   Gly His Cys Cys Glu Gln Trp Val Cys Glu Asp Asp Ala Lys Arg Pro
               180                 185                 190
   Arg Lys Thr Ala Pro Arg Asp Thr Gly Ala Phe Asp Ala Val Gly Glu
           195                 200                 205
   Val Glu Ala Trp His Arg Asn Cys Ile Ala Tyr Thr Ser Pro Trp Ser
       210                 215                 220
   Pro Cys Ser Thr Ser Cys Gly Leu Gly Val Ser Thr Arg Ile Ser Asn
   225                 230                 235                 240
   Val Asn Ala Gln Cys Trp Pro Glu Gln Glu Ser Arg Leu Cys Asn Leu
                   245                 250                 255
   Arg Pro Cys Asp Val Asp Ile His Thr Leu Ile Lys Ala Gly Lys Lys
               260                 265                 270
   Cys Leu Ala Val Tyr Gln Pro Glu Ala Ser Met Asn Phe Thr Leu Ala
           275                 280                 285
   Gly Cys Ile Ser Thr Arg Ser Tyr Gln Pro Lys Tyr Cys Gly Val Cys
       290                 295                 300
   Met Asp Asn Arg Cys Cys Ile Pro Tyr Lys Ser Lys Thr Ile Asp Val
   305                 310                 315                 320
   Ser Phe Gln Cys Pro Asp Gly Leu Gly Phe Ser Arg Gln Val Leu Trp
                   325                 330                 335
   Ile Asn Ala Cys Phe Cys Asn Leu Ser Cys Arg Asn Pro Asn Asp Ile
               340                 345                 350
   Phe Ala Asp Leu Glu Ser Tyr Pro Asp Phe Ser Glu Ile Ala Asn
           355                 360                 365
<210> 63
   <211> 1723
   <212> DNA
   <213> Homo sapiens
   <400> 63
   tcgcggaggc ttggggcagc cgggtagctc ggaggtcgtg gcgctggggg ctagcaccag      60
   cgctctgtcg ggaggcgcag cggttaggtg gaccggtcag cggactcacc ggccagggcg     120
   ctcggtgctg gaatttgata ttcattgatc cgggttttat ccctcttctt ttttcttaaa     180
   catttttttt taaaactgta ttgtttctcg ttttaattta tttttgcttg ccattcccca     240
   cttgaatcgg gccgacggct tggggagatt gctctacttc cccaaatcac tgtggatttt     300
   ggaaaccagc agaaagagga aagaggtagc aagagctcca gagagaagtc gaggaagaga     360
   gagacggggt cagagagagc gcgcgggcgt gcgagcagcg aaagcgacag gggcaaagtg     420
   agtgacctgc ttttgggggt gaccgccgga gcgcggcgtg agccctcccc cttgggatcc     480
   cgcagctgac cagtcgcgct gacggacaga cagacagaca ccgcccccag ccccagctac     540
   cacctcctcc ccggccggcg gcggacagtg gacgcggcgg cgagccgcgg gcaggggccg     600
   gagcccgcgc ccggaggcgg ggtggagggg gtcggggctc gcggcgtcgc actgaaactt     660
   ttcgtccaac ttctgggctg ttctcgcttc ggaggagccg tggtccgcgc gggggaagcc     720
   gagccgagcg gagccgcgag aagtgctagc tcgggccggg aggagccgca gccggaggag     780
   ggggaggagg aagaagagaa ggaagaggag agggggccgc agtggcgact cggcgctcgg     840
   aagccgggct catggacggg tgaggcggcg gtgtgcgcag acagtgctcc agccgcgcgc     900
   gctccccagg ccctggcccg ggcctcgggc cggggaggaa gagtagctcg ccgaggcgcc     960
   gaggagagcg ggccgcccca cagcccgagc cggagaggga gcgcgagccg cgccggcccc    1020
   ggtcgggcct ccgaaaccat gaactttctg ctgtcttggg tgcattggag ccttgccttg    1080
   ctgctctacc tccaccatgc caagtggtcc caggctgcac ccatggcaga aggaggaggg    1140
   cagaatcatc acgaagtggt gaagttcatg gatgtctatc agcgcagcta ctgccatcca    1200
   atcgagaccc tggtggacat cttccaggag taccctgatg agatcgagta catcttcaag    1260
   ccatcctgtg tgcccctgat gcgatgcggg ggctgctgca atgacgaggg cctggagtgt    1320
   gtgcccactg aggagtccaa catcaccatg cagattatgc ggatcaaacc tcaccaaggc    1380
   cagcacatag gagagatgag cttcctacag cacaacaaat gtgaatgcag accaaagaaa    1440
   gatagagcaa gacaagaaaa aaaatcagtt cgaggaaagg gaaaggggca aaaacgaaag    1500
   cgcaagaaat cccggtataa gtcctggagc gttccctgtg ggccttgctc agagcggaga    1560
   aagcatttgt ttgtacaaga tccgcagacg tgtaaatgtt cctgcaaaaa cacagactcg    1620
   cgttgcaagg cgaggcagct tgagttaaac gaacgtactt gcagatgtga caagccgagg    1680
   cggtgagccg ggcaggagga aggagcctcc ctcagggttt cgg                       1723
<210> 64
   <211> 215
   <212> PRT
   <213> Homo sapiens
<400> 64
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Tyr
   145                 150                 155                 160
   Lys Ser Trp Ser Val Pro Cys Gly Pro Cys Ser Glu Arg Arg Lys His
                   165                 170                 175
   Leu Phe Val Gln Asp Pro Gln Thr Cys Lys Cys Ser Cys Lys Asn Thr
   180 185 190
   Asp Ser Arg Cys Lys Ala Arg Gln Leu Glu Leu Asn Glu Arg Thr Cys
   195 200 205
   Arg Cys Asp Lys Pro Arg Arg
   210 215
<210> 65
   <211> 1172
   <212> DNA
   <213> Homo sapiens
<400> 65
   gcgatgcggg cgcccccggc gggcggcccc ggcgggcacc atgagccctc tgctccgccg 60
   cctgctgctc gccgcactcc tgcagctggc ccccgcccag gcccctgtct cccagcctga 120
   tgcccctggc caccagagga aagtggtgtc atggatagat gtgtatactc gcgctacctg 180
   ccagccccgg gaggtggtgg tgcccttgac tgtggagctc atgggcaccg tggccaaaca 240
   gctggtgccc agctgcgtga ctgtgcagcg ctgtggtggc tgctgccctg acgatggcct 300
   ggagtgtgtg cccactgggc agcaccaagt ccggatgcag atcctcatga tccggtaccc 360
   gagcagtcag ctgggggaga tgtccctgga agaacacagc cagtgtgaat gcagacctaa 420
   aaaaaaggac agtgctgtga agccagacag ggctgccact ccccaccacc gtccccagcc 480
   ccgttctgtt ccgggctggg actctgcccc cggagcaccc tccccagctg acatcaccca 540
   tcccactcca gccccaggcc cctctgccca cgctgcaccc agcaccacca gcgccctgac 600
   ccccggacct gccgctgccg ctgccgacgc cgcagcttcc tccgttgcca agggcggggc 660
   ttagagctca acccagacac ctgcaggtgc cggaagctgc gaaggtgaca catggctttt 720
   cagactcagc agggtgactt gcctcagagg ctatatccca gtgggggaac aaagaggagc 780
   ctggtaaaaa acagccaagc ccccaagacc tcagcccagg cagaagctgc tctaggacct 840
   gggcctctca gagggctctt ctgccatccc ttgtctccct gaggccatca tcaaacagga 900
   cagagttgga agaggagact gggaggcagc aagaggggtc acataccagc tcaggggaga 960
   atggagtact gtctcagttt ctaaccactc tgtgcaagta agcatcttac aactggctct 1020
   tcctcccctc actaagaaga cccaaacctc tgcataatgg gatttgggct ttggtacaag 1080
   aactgtgacc cccaaccctg ataaaagaga tggaaggaaa aaaaaaaaaa aaaaaaaaaa 1140
   aaaaaaaaaa aaaaaaaaaa aaaaaaaaaa aa 1172
<210> 66
   <211> 207
   <212> PRT
   <213> Homo sapiens
<400> 66
   Met Ser Pro Leu Leu Arg Arg Leu Leu Leu Ala Ala Leu Leu Gln Leu
   1 5 10 15
   Ala Pro Ala Gln Ala Pro Val Ser Gln Pro Asp Ala Pro Gly His Gln
   20 25 30
   Arg Lys Val Val Ser Trp Ile Asp Val Tyr Thr Arg Ala Thr Cys Gln
   35 40 45
   Pro Arg Glu Val Val Val Pro Leu Thr Val Glu Leu Met Gly Thr Val
   50 55 60
   Ala Lys Gln Leu Val Pro Ser Cys Val Thr Val Gln Arg Cys Gly Gly
   65 70 75 80
   Cys Cys Pro Asp Asp Gly Leu Glu Cys Val Pro Thr Gly Gln His Gln
   85 90 95
   Val Arg Met Gln Ile Leu Met Ile Arg Tyr Pro Ser Ser Gln Leu Gly
   100 105 110
   Glu Met Ser Leu Glu Glu His Ser Gln Cys Glu Cys Arg Pro Lys Lys
   115 120 125
   Lys Asp Ser Ala Val Lys Pro Asp Arg Ala Ala Thr Pro His His Arg
   130 135 140
   Pro Gln Pro Arg Ser Val Pro Gly Trp Asp Ser Ala Pro Gly Ala Pro
   145 150 155 160
   Ser Pro Ala Asp Ile Thr His Pro Thr Pro Ala Pro Gly Pro Ser Ala
   165 170 175
   His Ala Ala Pro Ser Thr Thr Ser Ala Leu Thr Pro Gly Pro Ala Ala
   180 185 190
   Ala Ala Ala Asp Ala Ala Ala Ser Ser Val Ala Lys Gly Gly Ala
   195 200 205
<210> 67
   <211> 2076
   <212> DNA
   <213> Homo sapiens
<400> 67
   cggggaaggg gagggaggag ggggacgagg gctctggcgg gtttggaggg gctgaacatc 60
   gcggggtgtt ctggtgtccc ccgccccgcc tctccaaaaa gctacaccga cgcggaccgc 120
   ggcggcgtcc tccctcgccc tcgcttcacc tcgcgggctc cgaatgcggg gagctcggat 180
   gtccggtttc ctgtgaggct tttacctgac acccgccgcc tttccccggc actggctggg 240
   agggcgccct gcaaagttgg gaacgcggag ccccggaccc gctcccgccg cctccggctc 300
   gcccaggggg ggtcgccggg aggagcccgg gggagaggga ccaggagggg cccgcggcct 360
   cgcaggggcg cccgcgcccc cacccctgcc cccgccagcg gaccggtccc ccacccccgg 420
   tccttccacc atgcacttgc tgggcttctt ctctgtggcg tgttctctgc tcgccgctgc 480
   gctgctcccg ggtcctcgcg aggcgcccgc cgccgccgcc gccttcgagt ccggactcga 540
   cctctcggac gcggagcccg acgcgggcga ggccacggct tatgcaagca aagatctgga 600
   ggagcagtta cggtctgtgt ccagtgtaga tgaactcatg actgtactct acccagaata 660
   ttggaaaatg tacaagtgtc agctaaggaa aggaggctgg caacataaca gagaacaggc 720
   caacctcaac tcaaggacag aagagactat aaaatttgct gcagcacatt ataatacaga 780
   gatcttgaaa agtattgata atgagtggag aaagactcaa tgcatgccac gggaggtgtg 840
   tatagatgtg gggaaggagt ttggagtcgc gacaaacacc ttctttaaac ctccatgtgt 900
   gtccgtctac agatgtgggg gttgctgcaa tagtgagggg ctgcagtgca tgaacaccag 960
   cacgagctac ctcagcaaga cgttatttga aattacagtg cctctctctc aaggccccaa 1020
   accagtaaca atcagttttg ccaatcacac ttcctgccga tgcatgtcta aactggatgt 1080
   ttacagacaa gttcattcca ttattagacg ttccctgcca gcaacactac cacagtgtca 1140
   ggcagcgaac aagacctgcc ccaccaatta catgtggaat aatcacatct gcagatgcct 1200
   ggctcaggaa gattttatgt tttcctcgga tgctggagat gactcaacag atggattcca   1260
   tgacatctgt ggaccaaaca aggagctgga tgaagagacc tgtcagtgtg tctgcagagc   1320
   ggggcttcgg cctgccagct gtggacccca caaagaacta gacagaaact catgccagtg   1380
   tgtctgtaaa aacaaactct tccccagcca atgtggggcc aaccgagaat ttgatgaaaa   1440
   cacatgccag tgtgtatgta aaagaacctg ccccagaaat caacccctaa atcctggaaa   1500
   atgtgcctgt gaatgtacag aaagtccaca gaaatgcttg ttaaaaggaa agaagttcca   1560
   ccaccaaaca tgcagctgtt acagacggcc atgtacgaac cgccagaagg cttgtgagcc   1620
   aggattttca tatagtgaag aagtgtgtcg ttgtgtccct tcatattgga aaagaccaca   1680
   aatgagctaa gattgtactg ttttccagtt catcgatttt ctattatgga aaactgtgtt   1740
   gccacagtag aactgtctgt gaacagagag acccttgtgg gtccatgcta acaaagacaa   1800
   aagtctgtct ttcctgaacc atgtggataa ctttacagaa atggactgga gctcatctgc   1860
   aaaaggcctc ttgtaaagac tggttttctg ccaatgacca aacagccaag attttcctct   1920
   tgtgatttct ttaaaagaat gactatataa tttatttcca ctaaaaatat tgtttctgca   1980
   ttcattttta tagcaacaac aattggtaaa actcactgtg atcaatattt ttatatcatg   2040
   caaaatatgt ttaaaataaa atgaaaattg tattat                             2076
<210> 68
   <211> 419
   <212> PRT
   <213> Homo sapiens
<400> 68
   Met His Leu Leu Gly Phe Phe Ser Val Ala Cys Ser Leu Leu Ala Ala
   1               5                   10                  15
   Ala Leu Leu Pro Gly Pro Arg Glu Ala Pro Ala Ala Ala Ala Ala Phe
               20                  25                  30
   Glu Ser Gly Leu Asp Leu Ser Asp Ala Glu Pro Asp Ala Gly Glu Ala
           35                  40                  45
   Thr Ala Tyr Ala Ser Lys Asp Leu Glu Glu Gln Leu Arg Ser Val Ser
       50                  55                  60
   Ser Val Asp Glu Leu Met Thr Val Leu Tyr Pro Glu Tyr Trp Lys Met
   65 70 75 80
   Tyr Lys Cys Gln Leu Arg Lys Gly Gly Trp Gln His Asn Arg Glu Gln
   85 90 95
   Ala Asn Leu Asn Ser Arg Thr Glu Glu Thr Ile Lys Phe Ala Ala Ala
   100 105 110
   His Tyr Asn Thr Glu Ile Leu Lys Ser Ile Asp Asn Glu Trp Arg Lys
   115 120 125
   Thr Gln Cys Met Pro Arg Glu Val Cys Ile Asp Val Gly Lys Glu Phe
   130 135 140
   Gly Val Ala Thr Asn Thr Phe Phe Lys Pro Pro Cys Val Ser Val Tyr
   145 150 155 160
   Arg Cys Gly Gly Cys Cys Asn Ser Glu Gly Leu Gln Cys Met Asn Thr
   165 170 175
   Ser Thr Ser Tyr Leu Ser Lys Thr Leu Phe Glu Ile Thr Val Pro Leu
   180 185 190
   Ser Gln Gly Pro Lys Pro Val Thr Ile Ser Phe Ala Asn His Thr Ser
   195 200 205
   Cys Arg Cys Met Ser Lys Leu Asp Val Tyr Arg Gln Val His Ser Ile
   210 215 220
   Ile Arg Arg Ser Leu Pro Ala Thr Leu Pro Gln Cys Gln Ala Ala Asn
   225 230 235 240
   Lys Thr Cys Pro Thr Asn Tyr Met Trp Asn Asn His Ile Cys Arg Cys
   245 250 255
   Leu Ala Gln Glu Asp Phe Met Phe Ser Ser Asp Ala Gly Asp Asp Ser
   260 265 270
   Thr Asp Gly Phe His Asp Ile Cys Gly Pro Asn Lys Glu Leu Asp Glu
   275 280 285
   Glu Thr Cys Gln Cys Val Cys Arg Ala Gly Leu Arg Pro Ala Ser Cys
   290 295 300
   Gly Pro His Lys Glu Leu Asp Arg Asn Ser Cys Gln Cys Val Cys Lys
   305 310 315 320
   Asn Lys Leu Phe Pro Ser Gln Cys Gly Ala Asn Arg Glu Phe Asp Glu
   325 330 335
   Asn Thr Cys Gln Cys Val Cys Lys Arg Thr Cys Pro Arg Asn Gln Pro
   340 345 350
   Leu Asn Pro Gly Lys Cys Ala Cys Glu Cys Thr Glu Ser Pro Gln Lys
   355 360 365
   Cys Leu Leu Lys Gly Lys Lys Phe His His Gln Thr Cys Ser Cys Tyr
   370 375 380
   Arg Arg Pro Cys Thr Asn Arg Gln Lys Ala Cys Glu Pro Gly Phe Ser
   385 390 395 400
   Tyr Ser Glu Glu Val Cys Arg Cys Val Pro Ser Tyr Trp Lys Arg Pro
   405 410 415
   Gln Met Ser
<210> 69
   <211> 2029
   <212> DNA
   <213> Homo sapiens
<400> 69
   gttgggttcc agctttctgt agctgtaagc attggtggcc acaccacctc cttacaaagc 60
   aactagaacc tgcggcatac attggagaga tttttttaat tttctggaca tgaagtaaat 120
   ttagagtgct ttctaatttc aggtagaaga catgtccacc ttctgattat ttttggagaa 180
   cattttgatt tttttcatct ctctctcccc acccctaaga ttgtgcaaaa aaagcgtacc 240
   ttgcctaatt gaaataattt cattggattt tgatcagaac tgattatttg gttttctgtg 300
   tgaagttttg aggtttcaaa ctttccttct ggagaatgcc ttttgaaaca attttctcta 360
   gctgcctgat gtcaactgct tagtaatcag tggatattga aatattcaaa atgtacagag 420
   agtgggtagt ggtgaatgtt ttcatgatgt tgtacgtcca gctggtgcag ggctccagta 480
   atgaacatgg accagtgaag cgatcatctc agtccacatt ggaacgatct gaacagcaga 540
   tcagggctgc ttctagtttg gaggaactac ttcgaattac tcactctgag gactggaagc 600
   tgtggagatg caggctgagg ctcaaaagtt ttaccagtat ggactctcgc tcagcatccc 660
   atcggtccac taggtttgcg gcaactttct atgacattga aacactaaaa gttatagatg 720
   aagaatggca aagaactcag tgcagcccta gagaaacgtg cgtggaggtg gccagtgagc 780
   tggggaagag taccaacaca ttcttcaagc ccccttgtgt gaacgtgttc cgatgtggtg 840
   gctgttgcaa tgaagagagc cttatctgta tgaacaccag cacctcgtac atttccaaac 900
   agctctttga gatatcagtg cctttgacat cagtacctga attagtgcct gttaaagttg 960
   ccaatcatac aggttgtaag tgcttgccaa cagccccccg ccatccatac tcaattatca 1020
   gaagatccat ccagatccct gaagaagatc gctgttccca ttccaagaaa ctctgtccta 1080
   ttgacatgct atgggatagc aacaaatgta aatgtgtttt gcaggaggaa aatccacttg 1140
   ctggaacaga agaccactct catctccagg aaccagctct ctgtgggcca cacatgatgt 1200
   ttgacgaaga tcgttgcgag tgtgtctgta aaacaccatg tcccaaagat ctaatccagc 1260
   accccaaaaa ctgcagttgc tttgagtgca aagaaagtct ggagacctgc tgccagaagc 1320
   acaagctatt tcacccagac acctgcagct gtgaggacag atgccccttt cataccagac 1380
   catgtgcaag tggcaaaaca gcatgtgcaa agcattgccg ctttccaaag gagaaaaggg 1440
   ctgcccaggg gccccacagc cgaaagaatc cttgattcag cgttccaagt tccccatccc 1500
   tgtcattttt aacagcatgc tgctttgcca agttgctgtc actgtttttt tcccaggtgt 1560
   taaaaaaaaa atccatttta cacagcacca cagtgaatcc agaccaacct tccattcaca 1620
   ccagctaagg agtccctggt tcattgatgg atgtcttcta gctgcagatg cctctgcgca 1680
   ccaaggaatg gagaggaggg gacccatgta atccttttgt ttagttttgt ttttgttttt 1740
   tggtgaatga gaaaggtgtg ctggtcatgg aatggcaggt gtcatatgac tgattactca 1800
   gagcagatga ggaaaactgt agtctctgag tcctttgcta atcgcaactc ttgtgaatta 1860
   ttctgattct tttttatgca gaatttgatt cgtatgatca gtactgactt tctgattact 1920
   gtccagctta tagtcttcca gtttaatgaa ctaccatctg atgtttcata tttaagtgta 1980
   tttaaagaaa ataaacacca ttattcaagc caaaaaaaaa aaaaaaaaa 2029
<210> 70
   <211> 354
   <212> PRT
   <213> Homo sapiens
<400> 70
   Met Tyr Arg Glu Trp Val Val Val Asn Val Phe Met Met Leu Tyr Val
   1               5                   10                  15
   Gln Leu Val Gln Gly Ser Ser Asn Glu His Gly Pro Val Lys Arg Ser
               20                  25                  30
   Ser Gln Ser Thr Leu Glu Arg Ser Glu Gln Gln Ile Arg Ala Ala Ser
           35                  40                  45
   Ser Leu Glu Glu Leu Leu Arg Ile Thr His Ser Glu Asp Trp Lys Leu
       50                  55                  60
   Trp Arg Cys Arg Leu Arg Leu Lys Ser Phe Thr Ser Met Asp Ser Arg
   65                  70                  75                  80
   Ser Ala Ser His Arg Ser Thr Arg Phe Ala Ala Thr Phe Tyr Asp Ile
                   85                  90                  95
   Glu Thr Leu Lys Val Ile Asp Glu Glu Trp Gln Arg Thr Gln Cys Ser
               100                 105                 110
   Pro Arg Glu Thr Cys Val Glu Val Ala Ser Glu Leu Gly Lys Ser Thr
           115                 120                 125
   Asn Thr Phe Phe Lys Pro Pro Cys Val Asn Val Phe Arg Cys Gly Gly
       130                 135                 140
   Cys Cys Asn Glu Glu Ser Leu Ile Cys Met Asn Thr Ser Thr Ser Tyr
   145                 150                 155                 160
   Ile Ser Lys Gln Leu Phe Glu Ile Ser Val Pro Leu Thr Ser Val Pro
                   165                 170                 175
   Glu Leu Val Pro Val Lys Val Ala Asn His Thr Gly Cys Lys Cys Leu
               180                 185                 190
   Pro Thr Ala Pro Arg His Pro Tyr Ser Ile Ile Arg Arg Ser Ile Gln
           195                 200                 205
   Ile Pro Glu Glu Asp Arg Cys Ser His Ser Lys Lys Leu Cys Pro Ile
       210                 215                 220
   Asp Met Leu Trp Asp Ser Asn Lys Cys Lys Cys Val Leu Gln Glu Glu
   225                 230                 235                 240
   Asn Pro Leu Ala Gly Thr Glu Asp His Ser His Leu Gln Glu Pro Ala
                   245                 250                 255
   Leu Cys Gly Pro His Met Met Phe Asp Glu Asp Arg Cys Glu Cys Val
               260                 265                 270
   Cys Lys Thr Pro Cys Pro Lys Asp Leu Ile Gln His Pro Lys Asn Cys
           275                 280                 285
   Ser Cys Phe Glu Cys Lys Glu Ser Leu Glu Thr Cys Cys Gln Lys His
       290                 295                 300
   Lys Leu Phe His Pro Asp Thr Cys Ser Cys Glu Asp Arg Cys Pro Phe
   305                 310                 315                 320
   His Thr Arg Pro Cys Ala Ser Gly Lys Thr Ala Cys Ala Lys His Cys
                   325                 330                 335
   Arg Phe Pro Lys Glu Lys Arg Ala Ala Gln Gly Pro His Ser Arg Lys
               340                 345                 350
   Asn Pro
<210> 71
   <211> 2825
   <212> DNA
   <213> Homo sapiens
<220>
   <221> misc_feature
   <222> (2689)..(2689)
   <223> n is a, c, g, or t
<400> 71
   gacgcgtggg cggacgcgtg ggctggttca ggtccaggtt ttgctttgat ccttttcaaa 60
   aactggagac acagaagagg gctctaggaa aaagttttgg atgggattat gtggaaacta 120
   ccctgcgatt ctctgctgcc agagcaggct cggcgcttcc accccagtgc agccttcccc 180
   tggcggtggt gaaagagact cgggagtcgc tgcttccaaa gtgcccgccg tgagtgagct 240
   ctcaccccag tcagccaaat gagcctcttc gggcttctcc tgctgacatc tgccctggcc 300
   ggccagagac aggggactca ggcggaatcc aacctgagta gtaaattcca gttttccagc 360
   aacaaggaac agaacggagt acaagatcct cagcatgaga gaattattac tgtgtctact 420
   aatggaagta ttcacagccc aaggtttcct catacttatc caagaaatac ggtcttggta 480
   tggagattag tagcagtaga ggaaaatgta tggatacaac ttacgtttga tgaaagattt 540
   gggcttgaag acccagaaga tgacatatgc aagtatgatt ttgtagaagt tgaggaaccc 600
   agtgatggaa ctatattagg gcgctggtgt ggttctggta ctgtaccagg aaaacagatt 660
   tctaaaggaa atcaaattag gataagattt gtatctgatg aatattttcc ttctgaacca 720
   gggttctgca tccactacaa cattgtcatg ccacaattca cagaagctgt gagtccttca 780
   gtgctacccc cttcagcttt gccactggac ctgcttaata atgctataac tgcctttagt 840
   accttggaag accttattcg atatcttgaa ccagagagat ggcagttgga cttagaagat 900
   ctatataggc caacttggca acttcttggc aaggcttttg tttttggaag aaaatccaga 960
   gtggtggatc tgaaccttct aacagaggag gtaagattat acagctgcac acctcgtaac 1020
   ttctcagtgt ccataaggga agaactaaag agaaccgata ccattttctg gccaggttgt 1080
   ctcctggtta aacgctgtgg tgggaactgt gcctgttgtc tccacaattg caatgaatgt 1140
   caatgtgtcc caagcaaagt tactaaaaaa taccacgagg tccttcagtt gagaccaaag 1200
   accggtgtca ggggattgca caaatcactc accgacgtgg ccctggagca ccatgaggag 1260
   tgtgactgtg tgtgcagagg gagcacagga ggatagccgc atcaccacca gcagctcttg 1320
   cccagagctg tgcagtgcag tggctgattc tattagagaa cgtatgcgtt atctccatcc 1380
   ttaatctcag ttgtttgctt caaggacctt tcatcttcag gatttacagt gcattctgaa 1440
   agaggagaca tcaaacagaa ttaggagttg tgcaacagct cttttgagag gaggcctaaa 1500
   ggacaggaga aaaggtcttc aatcgtggaa agaaaattaa atgttgtatt aaatagatca 1560
   ccagctagtt tcagagttac catgtacgta ttccactagc tgggttctgt atttcagttc 1620
   tttcgatacg gcttagggta atgtcagtac aggaaaaaaa ctgtgcaagt gagcacctga 1680
   ttccgttgcc ttgcttaact ctaaagctcc atgtcctggg cctaaaatcg tataaaatct 1740
   ggattttttt tttttttttt gctcatattc acatatgtaa accagaacat tctatgtact 1800
   acaaacctgg tttttaaaaa ggaactatgt tgctatgaat taaacttgtg tcatgctgat 1860
   aggacagact ggatttttca tatttcttat taaaatttct gccatttaga agaagagaac 1920
   tacattcatg gtttggaaga gataaacctg aaaagaagag tggccttatc ttcactttat 1980
   cgataagtca gtttatttgt ttcattgtgt acatttttat attctccttt tgacattata 2040
   actgttggct tttctaatct tgttaaatat atctattttt accaaaggta tttaatattc 2100
   ttttttatga caacttagat caactatttt tagcttggta aatttttcta aacacaattg 2160
   ttatagccag aggaacaaag atgatataaa atattgttgc tctgacaaaa atacatgtat 2220
   ttcattctcg tatggtgcta gagttagatt aatctgcatt ttaaaaaact gaattggaat 2280
   agaattggta agttgcaaag actttttgaa aataattaaa ttatcatatc ttccattcct 2340
   gttattggag atgaaaataa aaagcaactt atgaaagtag acattcagat ccagccatta 2400
   ctaacctatt cctttttggg ggaaatctga gcctagctca gaaaaacata aagcaccttg 2460
   aaaaagactt ggcagcttcc tgataaagcg tgctgtgctg tgcagtagga acacatccta 2520
   tttattgtga tgttgtggtt ttattatctt aaactctgtt ccatacactt gtataaatac 2580
   atggatattt ttatgtacag aagtatgtct cttaaccagt tcacttattg tactctggca 2640
   atttaaaaga aaatcagtaa aatattttgc ttgtaaaatg cttaatatng tgcctaggtt 2700
   atgtggtgac tatttgaatc aaaaatgtat tgaatcatca aataaaagaa tgtggctatt 2760
   ttggggagaa aattaaaaaa aaaaaaaaaa aaaaaggttt agggataaca gggtaatgcg 2820
   gccgc 2825
<210> 72
   <211> 345
   <212> PRT
   <213> Homo sapiens
<400> 72
   Met Ser Leu Phe Gly Leu Leu Leu Leu Thr Ser Ala Leu Ala Gly Gln
   1 5 10 15
   Arg Gln Gly Thr Gln Ala Glu Ser Asn Leu Ser Ser Lys Phe Gln Phe
               20                  25                  30
   Ser Ser Asn Lys Glu Gln Asn Gly Val Gln Asp Pro Gln His Glu Arg
           35                  40                  45
   Ile Ile Thr Val Ser Thr Asn Gly Ser Ile His Ser Pro Arg Phe Pro
       50                  55                  60
   His Thr Tyr Pro Arg Asn Thr Val Leu Val Trp Arg Leu Val Ala Val
   65                  70                  75                  80
   Glu Glu Asn Val Trp Ile Gln Leu Thr Phe Asp Glu Arg Phe Gly Leu
                   85                  90                  95
   Glu Asp Pro Glu Asp Asp Ile Cys Lys Tyr Asp Phe Val Glu Val Glu
               100                 105                 110
   Glu Pro Ser Asp Gly Thr Ile Leu Gly Arg Trp Cys Gly Ser Gly Thr
           115                 120                 125
   Val Pro Gly Lys Gln Ile Ser Lys Gly Asn Gln Ile Arg Ile Arg Phe
       130                 135                 140
   Val Ser Asp Glu Tyr Phe Pro Ser Glu Pro Gly Phe Cys Ile His Tyr
   145                 150                 155                 160
   Asn Ile Val Met Pro Gln Phe Thr Glu Ala Val Ser Pro Ser Val Leu
                   165                 170                 175
   Pro Pro Ser Ala Leu Pro Leu Asp Leu Leu Asn Asn Ala Ile Thr Ala
               180                 185                 190
   Phe Ser Thr Leu Glu Asp Leu Ile Arg Tyr Leu Glu Pro Glu Arg Trp
           195                 200                 205
   Gln Leu Asp Leu Glu Asp Leu Tyr Arg Pro Thr Trp Gln Leu Leu Gly
       210                 215                 220
   Lys Ala Phe Val Phe Gly Arg Lys Ser Arg Val Val Asp Leu Asn Leu
   225                 230                 235                 240
   Leu Thr Glu Glu Val Arg Leu Tyr Ser Cys Thr Pro Arg Asn Phe Ser
   245 250 255
   Val Ser Ile Arg Glu Glu Leu Lys Arg Thr Asp Thr Ile Phe Trp Pro
   260 265 270
   Gly Cys Leu Leu Val Lys Arg Cys Gly Gly Asn Cys Ala Cys Cys Leu
   275 280 285
   His Asn Cys Asn Glu Cys Gln Cys Val Pro Ser Lys Val Thr Lys Lys
   290 295 300
   Tyr His Glu Val Leu Gln Leu Arg Pro Lys Thr Gly Val Arg Gly Leu
   305 310 315 320
   His Lys Ser Leu Thr Asp Val Ala Leu Glu His His Glu Glu Cys Asp
   325 330 335
   Cys Val Cys Arg Gly Ser Thr Gly Gly
   340 345
<210> 73
   <211> 2745
   <212> DNA
   <213> Homo sapiens
<400> 73
   acctccctcc gcggagcagc cagacagcga gggccccggc cgggggcagg ggggacgccc 60
   cgtccggggc accccccccg gctctgagcc gcccgcgggg ccggcctcgg cccggagcgg 120
   aggaaggagt cgccgaggag cagcctgagg ccccagagtc tgagacgagc cgccgccgcc 180
   cccgccactg cggggaggag ggggaggagg agcgggagga gggacgagct ggtcgggaga 240
   agaggaaaaa aacttttgag acttttccgt tgccgctggg agccggaggc gcggggacct 300
   cttggcgcga cgctgccccg cgaggaggca ggacttgggg accccagacc gcctcccttt 360
   gccgccgggg acgcttgctc cctccctgcc ccctacacgg cgtccctcag gcgcccccat 420
   tccggaccag ccctcgggag tcgccgaccc ggcctcccgc aaagactttt ccccagacct 480
   cgggcgcacc ccctgcacgc cgccttcatc cccggcctgt ctcctgagcc cccgcgcatc 540
   ctagaccctt tctcctccag gagacggatc tctctccgac ctgccacaga tcccctattc 600
   aagaccaccc accttctggt accagatcgc gcccatctag gttatttccg tgggatactg 660
   agacaccccc ggtccaagcc tcccctccac cactgcgccc ttctccctga ggagcctcag 720
   ctttccctcg aggccctcct accttttgcc gggagacccc cagcccctgc aggggcgggg 780
   cctccccacc acaccagccc tgttcgcgct ctcggcagtg ccggggggcg ccgcctcccc 840
   catgccgccc tccgggctgc ggctgctgcc gctgctgcta ccgctgctgt ggctactggt 900
   gctgacgcct ggcccgccgg ccgcgggact atccacctgc aagactatcg acatggagct 960
   ggtgaagcgg aagcgcatcg aggccatccg cggccagatc ctgtccaagc tgcggctcgc 1020
   cagccccccg agccaggggg aggtgccgcc cggcccgctg cccgaggccg tgctcgccct 1080
   gtacaacagc acccgcgacc gggtggccgg ggagagtgca gaaccggagc ccgagcctga 1140
   ggccgactac tacgccaagg aggtcacccg cgtgctaatg gtggaaaccc acaacgaaat 1200
   ctatgacaag ttcaagcaga gtacacacag catatatatg ttcttcaaca catcagagct 1260
   ccgagaagcg gtacctgaac ccgtgttgct ctcccgggca gagctgcgtc tgctgaggag 1320
   gctcaagtta aaagtggagc agcacgtgga gctgtaccag aaatacagca acaattcctg 1380
   gcgatacctc agcaaccggc tgctggcacc cagcgactcg ccagagtggt tatcttttga 1440
   tgtcaccgga gttgtgcggc agtggttgag ccgtggaggg gaaattgagg gctttcgcct 1500
   tagcgcccac tgctcctgtg acagcaggga taacacactg caagtggaca tcaacgggtt 1560
   cactaccggc cgccgaggtg acctggccac cattcatggc atgaaccggc ctttcctgct 1620
   tctcatggcc accccgctgg agagggccca gcatctgcaa agctcccggc accgccgagc 1680
   cctggacacc aactattgct tcagctccac ggagaagaac tgctgcgtgc ggcagctgta 1740
   cattgacttc cgcaaggacc tcggctggaa gtggatccac gagcccaagg gctaccatgc 1800
   caacttctgc ctcgggccct gcccctacat ttggagcctg gacacgcagt acagcaaggt 1860
   cctggccctg tacaaccagc ataacccggg cgcctcggcg gcgccgtgct gcgtgccgca 1920
   ggcgctggag ccgctgccca tcgtgtacta cgtgggccgc aagcccaagg tggagcagct 1980
   gtccaacatg atcgtgcgct cctgcaagtg cagctgaggt cccgccccgc cccgccccgc 2040
   cccggcaggc ccggccccac cccgccccgc ccccgctgcc ttgcccatgg gggctgtatt 2100
   taaggacacc gtgccccaag cccacctggg gccccattaa agatggagag aggactgcgg 2160
   atctctgtgt cattgggcgc ctgcctgggg tctccatccc tgacgttccc ccactcccac 2220
   tccctctctc tccctctctg cctcctcctg cctgtctgca ctattccttt gcccggcatc 2280
   aaggcacagg ggaccagtgg ggaacactac tgtagttaga tctatttatt gagcaccttg   2340
   ggcactgttg aagtgcctta cattaatgaa ctcattcagt caccatagca acactctgag   2400
   atggcaggga ctctgataac acccatttta aaggttgagg aaacaagccc agagaggtta   2460
   agggaggagt tcctgcccac caggaacctg ctttagtggg ggatagtgaa gaagacaata   2520
   aaagatagta gttcaggcca ggcggggtgc tcacgcctgt aatcctagca cttttgggag   2580
   gcagagatgg gaggatactt gaatccaggc atttgagacc agcctgggta acatagtgag   2640
   accctatctc tacaaaacac ttttaaaaaa tgtacacctg tggtcccagc tactctggag   2700
   gctaaggtgg gaggatcact tgatcctggg aggtcaaggc tgcag                   2745
<210> 74
   <211> 391
   <212> PRT
   <213> Homo sapiens
<400> 74
   Met Pro Pro Ser Gly Leu Arg Leu Leu Pro Leu Leu Leu Pro Leu Leu
   1               5                   10                  15
   Trp Leu Leu Val Leu Thr Pro Gly Pro Pro Ala Ala Gly Leu Ser Thr
               20                  25                  30
   Cys Lys Thr Ile Asp Met Glu Leu Val Lys Arg Lys Arg Ile Glu Ala
           35                  40                  45
   Ile Arg Gly Gln Ile Leu Ser Lys Leu Arg Leu Ala Ser Pro Pro Ser
       50                  55                  60
   Gln Gly Glu Val Pro Pro Gly Pro Leu Pro Glu Ala Val Leu Ala Leu
   65                  70                  75                  80
   Tyr Asn Ser Thr Arg Asp Arg Val Ala Gly Glu Ser Ala Glu Pro Glu
                   85                  90                  95
   Pro Glu Pro Glu Ala Asp Tyr Tyr Ala Lys Glu Val Thr Arg Val Leu
               100                 105                 110
   Met Val Glu Thr His Asn Glu Ile Tyr Asp Lys Phe Lys Gln Ser Thr
           115                 120                 125
   His Ser Ile Tyr Met Phe Phe Asn Thr Ser Glu Leu Arg Glu Ala Val
       130                 135                 140
   Pro Glu Pro Val Leu Leu Ser Arg Ala Glu Leu Arg Leu Leu Arg Arg
   145                 150                 155                 160
   Leu Lys Leu Lys Val Glu Gln His Val Glu Leu Tyr Gln Lys Tyr Ser
                   165                 170                 175
   Asn Asn Ser Trp Arg Tyr Leu Ser Asn Arg Leu Leu Ala Pro Ser Asp
               180                 185                 190
   Ser Pro Glu Trp Leu Ser Phe Asp Val Thr Gly Val Val Arg Gln Trp
           195                 200                 205
   Leu Ser Arg Gly Gly Glu Ile Glu Gly Phe Arg Leu Ser Ala His Cys
       210                 215                 220
   Ser Cys Asp Ser Arg Asp Asn Thr Leu Gln Val Asp Ile Asn Gly Phe
   225                 230                 235                 240
   Thr Thr Gly Arg Arg Gly Asp Leu Ala Thr Ile His Gly Met Asn Arg
                   245                 250                 255
   Pro Phe Leu Leu Leu Met Ala Thr Pro Leu Glu Arg Ala Gln His Leu
               260                 265                 270
   Gln Ser Ser Arg His Arg Arg Ala Leu Asp Thr Asn Tyr Cys Phe Ser
           275                 280                 285
   Ser Thr Glu Lys Asn Cys Cys Val Arg Gln Leu Tyr Ile Asp Phe Arg
       290                 295                 300
   Lys Asp Leu Gly Trp Lys Trp Ile His Glu Pro Lys Gly Tyr His Ala
   305                 310                 315                 320
   Asn Phe Cys Leu Gly Pro Cys Pro Tyr Ile Trp Ser Leu Asp Thr Gln
                   325                 330                 335
   Tyr Ser Lys Val Leu Ala Leu Tyr Asn Gln His Asn Pro Gly Ala Ser
   340 345 350
   Ala Ala Pro Cys Cys Val Pro Gln Ala Leu Glu Pro Leu Pro Ile Val
   355 360 365
   Tyr Tyr Val Gly Arg Lys Pro Lys Val Glu Gln Leu Ser Asn Met Ile
   370 375 380
   Val Arg Ser Cys Lys Cys Ser
   385 390
<210> 75
   <211> 336
   <212> DNA
   <213> Homo sapiens
<400> 75
   gccctggaca ccaactattg cttcagctcc acggagaaga actgctgcgt gcggcagctg      60
   tacattgact tccgcaagga cctcggctgg aagtggatcc acgagcccaa gggctaccat     120
   gccaacttct gcctcgggcc ctgcccctac atttggagcc tggacacgca gtacagcaag     180
   gtcctggccc tgtacaacca gcataacccg ggcgcctcgg cggcgccgtg ctgcgtgccg     240
   caggcgctgg agccgctgcc catcgtgtac tacgtgggcc gcaagcccaa ggtggagcag     300
   ctgtccaaca tgatcgtgcg ctcctgcaag tgcagc                               336
<210> 76
   <211> 112
   <212> PRT
   <213> Homo sapiens
<400> 76
   Ala Leu Asp Thr Asn Tyr Cys Phe Ser Ser Thr Glu Lys Asn Cys Cys
   1 5 10 15
   Val Arg Gln Leu Tyr Ile Asp Phe Arg Lys Asp Leu Gly Trp Lys Trp
   20 25 30
   Ile His Glu Pro Lys Gly Tyr His Ala Asn Phe Cys Leu Gly Pro Cys
   35 40 45
   Pro Tyr Ile Trp Ser Leu Asp Thr Gln Tyr Ser Lys Val Leu Ala Leu
   50 55 60
   Tyr Asn Gln His Asn Pro Gly Ala Ser Ala Ala Pro Cys Cys Val Pro
   65 70 75 80
   Gln Ala Leu Glu Pro Leu Pro Ile Val Tyr Tyr Val Gly Arg Lys Pro
   85 90 95
   Lys Val Glu Gln Leu Ser Asn Met Ile Val Arg Ser Cys Lys Cys Ser
   100 105 110
<210> 77
   <211> 1695
   <212> DNA
   <213> Homo sapiens
<400> 77
   caagcaggat acgtttttct gttgggcatt gactagattg tttgcaaaag tttcgcatca 60
   aaaacaaaca acaacaacaa aaaaccaaac aactctcctt gatctatact ttgagaattg 120
   ttgatttctt tttttttatt ctgactttta aaaacaactt ttttttccac ttttttaaaa 180
   aatgcactac tgtgtgctga gcgcttttct gatcctgcat ctggtcacgg tcgcgctcag 240
   cctgtctacc tgcagcacac tcgatatgga ccagttcatg cgcaagagga tcgaggcgat 300
   ccgcgggcag atcctgagca agctgaagct caccagtccc ccagaagact atcctgagcc 360
   cgaggaagtc cccccggagg tgatttccat ctacaacagc accagggact tgctccagga 420
   gaaggcgagc cggagggcgg ccgcctgcga gcgcgagagg agcgacgaag agtactacgc 480
   caaggaggtt tacaaaatag acatgccgcc cttcttcccc tccgaaaatg ccatcccgcc 540
   cactttctac agaccctact tcagaattgt tcgatttgac gtctcagcaa tggagaagaa 600
   tgcttccaat ttggtgaaag cagagttcag agtctttcgt ttgcagaacc caaaagccag 660
   agtgcctgaa caacggattg agctatatca gattctcaag tccaaagatt taacatctcc 720
   aacccagcgc tacatcgaca gcaaagttgt gaaaacaaga gcagaaggcg aatggctctc 780
   cttcgatgta actgatgctg ttcatgaatg gcttcaccat aaagacagga acctgggatt 840
   taaaataagc ttacactgtc cctgctgcac ttttgtacca tctaataatt acatcatccc 900
   aaataaaagt gaagaactag aagcaagatt tgcaggtatt gatggcacct ccacatatac 960
   cagtggtgat cagaaaacta taaagtccac taggaaaaaa aacagtggga agaccccaca 1020
   tctcctgcta atgttattgc cctcctacag acttgagtca caacagacca accggcggaa 1080
   gaagcgtgct ttggatgcgg cctattgctt tagaaatgtg caggataatt gctgcctacg   1140
   tccactttac attgatttca agagggatct agggtggaaa tggatacacg aacccaaagg   1200
   gtacaatgcc aacttctgtg ctggagcatg cccgtattta tggagttcag acactcagca   1260
   cagcagggtc ctgagcttat ataataccat aaatccagaa gcatctgctt ctccttgctg   1320
   cgtgtcccaa gatttagaac ctctaaccat tctctactac attggcaaaa cacccaagat   1380
   tgaacagctt tctaatatga ttgtaaagtc ttgcaaatgc agctaaaatt cttggaaaag   1440
   tggcaagacc aaaatgacaa tgatgatgat aatgatgatg acgacgacaa cgatgatgct   1500
   tgtaacaaga aaacataaga gagccttggt tcatcagtgt taaaaaattt ttgaaaaggc   1560
   ggtactagtt cagacacttt ggaagtttgt gttctgtttg ttaaaactgg catctgacac   1620
   aaaaaaagtt gaaggcctta ttctacattt cacctacttt gtaagtgaga gagacaagaa   1680
   gcaaattttt ttaaa                                                    1695
<210> 78
   <211> 414
   <212> PRT
   <213> Homo sapiens
<400> 78
<210> 79
   <211> 336
   <212> DNA
   <213> Homo sapiens
<400> 79
   gctttggatg cggcctattg ctttagaaat gtgcaggata attgctgcct acgtccactt 60
   tacattgatt tcaagaggga tctagggtgg aaatggatac acgaacccaa agggtacaat 120
   gccaacttct gtgctggagc atgcccgtat ttatggagtt cagacactca gcacagcagg 180
   gtcctgagct tatataatac cataaatcca gaagcatctg cttctccttg ctgcgtgtcc 240
   caagatttag aacctctaac cattctctac tacattggca aaacacccaa gattgaacag 300
   ctttctaata tgattgtaaa gtcttgcaaa tgcagc 336
<210> 80
   <211> 112
   <212> PRT
   <213> Homo sapiens
<400> 80
   Ala Leu Asp Ala Ala Tyr Cys Phe Arg Asn Val Gln Asp Asn Cys Cys
   1 5 10 15
   Leu Arg Pro Leu Tyr Ile Asp Phe Lys Arg Asp Leu Gly Trp Lys Trp
   20 25 30
   Ile His Glu Pro Lys Gly Tyr Asn Ala Asn Phe Cys Ala Gly Ala Cys
   35 40 45
   Pro Tyr Leu Trp Ser Ser Asp Thr Gln His Ser Arg Val Leu Ser Leu
   50 55 60
   Tyr Asn Thr Ile Asn Pro Glu Ala Ser Ala Ser Pro Cys Cys Val Ser
   65 70 75 80
   Gln Asp Leu Glu Pro Leu Thr Ile Leu Tyr Tyr Ile Gly Lys Thr Pro
   85 90 95
   Lys Ile Glu Gln Leu Ser Asn Met Ile Val Lys Ser Cys Lys Cys Ser
   100 105 110
<210> 81
   <211> 2574
   <212> DNA
   <213> Homo sapiens
<400> 81
   cctgtttaga cacatggaca acaatcccag cgctacaagg cacacagtcc gcttcttcgt 60
   cctcagggtt gccagcgctt cctggaagtc ctgaagctct cgcagtgcag tgagttcatg 120
   caccttcttg ccaagcctca gtctttggga tctggggagg ccgcctggtt ttcctccctc 180
   cttctgcacg tctgctgggg tctcttcctc tccaggcctt gccgtccccc tggcctctct 240
   tcccagctca cacatgaaga tgcacttgca aagggctctg gtggtcctgg ccctgctgaa 300
   ctttgccacg gtcagcctct ctctgtccac ttgcaccacc ttggacttcg gccacatcaa 360
   gaagaagagg gtggaagcca ttaggggaca gatcttgagc aagctcaggc tcaccagccc 420
   ccctgagcca acggtgatga cccacgtccc ctatcaggtc ctggcccttt acaacagcac 480
   ccgggagctg ctggaggaga tgcatgggga gagggaggaa ggctgcaccc aggaaaacac 540
   cgagtcggaa tactatgcca aagaaatcca taaattcgac atgatccagg ggctggcgga 600
   gcacaacgaa ctggctgtct gccctaaagg aattacctcc aaggttttcc gcttcaatgt 660
   gtcctcagtg gagaaaaata gaaccaacct attccgagca gaattccggg tcttgcgggt 720
   gcccaacccc agctctaagc ggaatgagca gaggatcgag ctcttccaga tccttcggcc 780
   agatgagcac attgccaaac agcgctatat cggtggcaag aatctgccca cacggggcac 840
   tgccgagtgg ctgtcctttg atgtcactga cactgtgcgt gagtggctgt tgagaagaga 900
   gtccaactta ggtctagaaa tcagcattca ctgtccatgt cacacctttc agcccaatgg 960
   agatatcctg gaaaacattc acgaggtgat ggaaatcaaa ttcaaaggcg tggacaatga 1020
   ggatgaccat ggccgtggag atctggggcg cctcaagaag cagaaggatc accacaaccc 1080
   tcatctaatc ctcatgatga ttcccccaca ccggctcgac aacccgggcc aggggggtca 1140
   gaggaagaag cgggctttgg acaccaatta ctgcttccgc aacttggagg agaactgctg 1200
   tgtgcgcccc ctctacattg acttccgaca ggatctgggc tggaagtggg tccatgaacc 1260
   taagggctac tatgccaact tctgctcagg cccttgccca tacctccgca gtgcagacac 1320
   aacccacagc acggtgctgg gactgtacaa cactctgaac cctgaagcat ctgcctcgcc 1380
   ttgctgcgtg ccccaggacc tggagcccct gaccatcctg tactatgttg ggaggacccc 1440
   caaagtggag cagctctcca acatggtggt gaagtcttgt aaatgtagct gagaccccac 1500
   gtgcgacaga gagaggggag agagaaccac cactgcctga ctgcccgctc ctcgggaaac 1560
   acacaagcaa caaacctcac tgagaggcct ggagcccaca accttcggct ccgggcaaat 1620
   ggctgagatg gaggtttcct tttggaacat ttctttcttg ctggctctga gaatcacggt 1680
   ggtaaagaaa gtgtgggttt ggttagagga aggctgaact cttcagaaca cacagacttt 1740
   ctgtgacgca gacagagggg atggggatag aggaaaggga tggtaagttg agatgttgtg 1800
   tggcaatggg atttgggcta ccctaaaggg agaaggaagg gcagagaatg gctgggtcag 1860
   ggccagactg gaagacactt cagatctgag gttggatttg ctcattgctg taccacatct 1920
   gctctaggga atctggatta tgttatacaa ggcaagcatt ttttttttta aagacaggtt 1980
   acgaagacaa agtcccagaa ttgtatctca tactgtctgg gattaagggc aaatctatta 2040
   cttttgcaaa ctgtcctcta catcaattaa catcgtgggt cactacaggg agaaaatcca 2100
   ggtcatgcag ttcctggccc atcaactgta ttgggccttt tggatatgct gaacgcagaa 2160
   gaaagggtgg aaatcaaccc tctcctgtct gccctctggg tccctcctct cacctctccc 2220
   tcgatcatat ttccccttgg acacttggtt agacgccttc caggtcagga tgcacatttc 2280
   tggattgtgg ttccatgcag ccttggggca ttatgggtct tcccccactt cccctccaag 2340
   accctgtgtt catttggtgt tcctggaagc aggtgctaca acatgtgagg cattcgggga 2400
   agctgcacat gtgccacaca gtgacttggc cccagacgca tagactgagg tataaagaca 2460
   agtatgaata ttactctcaa aatctttgta taaataaata tttttggggc atcctggatg 2520
   atttcatctt ctggaatatt gtttctagaa cagtaaaagc cttattctaa ggtg 2574
<210> 82
   <211> 412
   <212> PRT
   <213> Homo sapiens
<400> 82
<210> 83
   <211> 3213
   <212> DNA
   <213> Homo sapiens
<400> 83
   agagactcaa gatgattccc tttttaccca tgttttctct actattgctg cttattgtta 60
   accctataaa cgccaacaat cattatgaca agatcttggc tcatagtcgt atcaggggtc 120
   gggaccaagg cccaaatgtc tgtgcccttc aacagatttt gggcaccaaa aagaaatact 180
   tcagcacttg taagaactgg tataaaaagt ccatctgtgg acagaaaacg actgttttat 240
   atgaatgttg ccctggttat atgagaatgg aaggaatgaa aggctgccca gcagttttgc 300
   ccattgacca tgtttatggc actctgggca tcgtgggagc caccacaacg cagcgctatt 360
   ctgacgcctc aaaactgagg gaggagatcg agggaaaggg atccttcact tactttgcac 420
   cgagtaatga ggcttgggac aacttggatt ctgatatccg tagaggtttg gagagcaacg 480
   tgaatgttga attactgaat gctttacata gtcacatgat taataagaga atgttgacca 540
   aggacttaaa aaatggcatg attattcctt caatgtataa caatttgggg cttttcatta 600
   accattatcc taatggggtt gtcactgtta attgtgctcg aatcatccat gggaaccaga 660
   ttgcaacaaa tggtgttgtc catgtcattg accgtgtgct tacacaaatt ggtacctcaa 720
   ttcaagactt cattgaagca gaagatgacc tttcatcttt tagagcagct gccatcacat 780
   cggacatatt ggaggccctt ggaagagacg gtcacttcac actctttgct cccaccaatg 840
   aggcttttga gaaacttcca cgaggtgtcc tagaaaggtt catgggagac aaagtggctt 900
   ccgaagctct tatgaagtac cacatcttaa atactctcca gtgttctgag tctattatgg 960
   gaggagcagt ctttgagacg ctggaaggaa atacaattga gataggatgt gacggtgaca 1020
   gtataacagt aaatggaatc aaaatggtga acaaaaagga tattgtgaca aataatggtg 1080
   tgatccattt gattgatcag gtcctaattc ctgattctgc caaacaagtt attgagctgg 1140
   ctggaaaaca gcaaaccacc ttcacggatc ttgtggccca attaggcttg gcatctgctc 1200
   tgaggccaga tggagaatac actttgctgg cacctgtgaa taatgcattt tctgatgata 1260
   ctctcagcat ggttcagcgc ctccttaaat taattctgca gaatcacata ttgaaagtaa 1320
   aagttggcct taatgagctt tacaacgggc aaatactgga aaccatcgga ggcaaacagc 1380
   tcagagtctt cgtatatcgt acagctgtct gcattgaaaa ttcatgcatg gagaaaggga 1440
   gtaagcaagg gagaaacggt gcgattcaca tattccgcga gatcatcaag ccagcagaga 1500
   aatccctcca tgaaaagtta aaacaagata agcgctttag caccttcctc agcctacttg 1560
   aagctgcaga cttgaaagag ctcctgacac aacctggaga ctggacatta tttgtgccaa 1620
   ccaatgatgc ttttaaggga atgactagtg aagaaaaaga aattctgata cgggacaaaa 1680
   atgctcttca aaacatcatt ctttatcacc tgacaccagg agttttcatt ggaaaaggat 1740
   ttgaacctgg tgttactaac attttaaaga ccacacaagg aagcaaaatc tttctgaaag 1800
   aagtaaatga tacacttctg gtgaatgaat tgaaatcaaa agaatctgac atcatgacaa 1860
   caaatggtgt aattcatgtt gtagataaac tcctctatcc agcagacaca cctgttggaa 1920
   atgatcaact gctggaaata cttaataaat taatcaaata catccaaatt aagtttgttc 1980
   gtggtagcac cttcaaagaa atccccgtga ctgtctatac aactaaaatt ataaccaaag 2040
   ttgtggaacc aaaaattaaa gtgattgaag gcagtcttca gcctattatc aaaactgaag 2100
   gacccacact aacaaaagtc aaaattgaag gtgaacctga attcagactg attaaagaag 2160
   gtgaaacaat aactgaagtg atccatggag agccaattat taaaaaatac accaaaatca 2220
   ttgatggagt gcctgtggaa ataactgaaa aagagacacg agaagaacga atcattacag 2280
   gtcctgaaat aaaatacact aggatttcta ctggaggtgg agaaacagaa gaaactctga 2340
   agaaattgtt acaagaagag gtcaccaagg tcaccaaatt cattgaaggt ggtgatggtc 2400
   atttatttga agatgaagaa attaaaagac tgcttcaggg agacacaccc gtgaggaagt 2460
   tgcaagccaa caaaaaagtt caaggttcta gaagacgatt aagggaaggt cgttctcagt 2520
   gaaaatccaa aaaccagaaa aaaatgttta tacaacccta agtcaataac ctgaccttag 2580
   aaaattgtga gagccaagtt gacttcagga actgaaacat cagcacaaag aagcaatcat 2640
   caaataattc tgaacacaaa tttaatattt ttttttctga atgagaaaca tgagggaaat 2700
   tgtggagtta gcctcctgtg gtaaaggaat tgaagaaaat ataacacctt acaccctttt 2760
   tcatcttgac attaaaagtt ctggctaact ttggaatcca ttagagaaaa atccttgtca 2820
   ccagattcat tacaattcaa atcgaagagt tgtgaactgt tatcccattg aaaagaccga 2880
   gccttgtatg tatgttatgg atacataaaa tgcacgcaag ccattatctc tccatgggaa 2940
   gctaagttat aaaaataggt gcttggtgta caaaactttt tatatcaaaa ggctttgcac 3000
   atttctatat gagtgggttt actggtaaat tatgttattt tttacaacta attttgtact 3060
   ctcagaatgt ttgtcatatg cttcttgcaa tgcatatttt ttaatctcaa acgtttcaat 3120
   aaaaccattt ttcagatata aagagaatta cttcaaattg agtaattcag aaaaactcaa 3180
   gatttaagtt aaaaagtggt ttggacttgg gaa 3213
<210> 84
   <211> 836
   <212> PRT
   <213> Homo sapiens
<400> 84
   Met Ile Pro Phe Leu Pro Met Phe Ser Leu Leu Leu Leu Leu Ile Val
   1 5 10 15
   Asn Pro Ile Asn Ala Asn Asn His Tyr Asp Lys Ile Leu Ala His Ser
   20 25 30
   Arg Ile Arg Gly Arg Asp Gln Gly Pro Asn Val Cys Ala Leu Gln Gln
   35 40 45
   Ile Leu Gly Thr Lys Lys Lys Tyr Phe Ser Thr Cys Lys Asn Trp Tyr
   50 55 60
   Lys Lys Ser Ile Cys Gly Gln Lys Thr Thr Val Leu Tyr Glu Cys Cys
   65 70 75 80
   Pro Gly Tyr Met Arg Met Glu Gly Met Lys Gly Cys Pro Ala Val Leu
   85 90 95
   Pro Ile Asp His Val Tyr Gly Thr Leu Gly Ile Val Gly Ala Thr Thr
   100 105 110
   Thr Gln Arg Tyr Ser Asp Ala Ser Lys Leu Arg Glu Glu Ile Glu Gly
   115 120 125
   Lys Gly Ser Phe Thr Tyr Phe Ala Pro Ser Asn Glu Ala Trp Asp Asn
   130 135 140
   Leu Asp Ser Asp Ile Arg Arg Gly Leu Glu Ser Asn Val Asn Val Glu
   145 150 155 160
   Leu Leu Asn Ala Leu His Ser His Met Ile Asn Lys Arg Met Leu Thr
   165 170 175
   Lys Asp Leu Lys Asn Gly Met Ile Ile Pro Ser Met Tyr Asn Asn Leu
   180 185 190
   Gly Leu Phe Ile Asn His Tyr Pro Asn Gly Val Val Thr Val Asn Cys
   195 200 205
   Ala Arg Ile Ile His Gly Asn Gln Ile Ala Thr Asn Gly Val Val His
   210 215 220
   Val Ile Asp Arg Val Leu Thr Gln Ile Gly Thr Ser Ile Gln Asp Phe
   225 230 235 240
   Ile Glu Ala Glu Asp Asp Leu Ser Ser Phe Arg Ala Ala Ala Ile Thr
   245 250 255
   Ser Asp Ile Leu Glu Ala Leu Gly Arg Asp Gly His Phe Thr Leu Phe
   260 265 270
   Ala Pro Thr Asn Glu Ala Phe Glu Lys Leu Pro Arg Gly Val Leu Glu
   275 280 285
   Arg Phe Met Gly Asp Lys Val Ala Ser Glu Ala Leu Met Lys Tyr His
   290 295 300
   Ile Leu Asn Thr Leu Gln Cys Ser Glu Ser Ile Met Gly Gly Ala Val
   305 310 315 320
   Phe Glu Thr Leu Glu Gly Asn Thr Ile Glu Ile Gly Cys Asp Gly Asp
   325 330 335
   Ser Ile Thr Val Asn Gly Ile Lys Met Val Asn Lys Lys Asp Ile Val
   340 345 350
   Thr Asn Asn Gly Val Ile His Leu Ile Asp Gln Val Leu Ile Pro Asp
   355 360 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
                   405                 410                 415
   Thr Leu Ser Met Val Gln Arg Leu Leu Lys Leu Ile Leu Gln Asn His
               420                 425                 430
   Ile Leu Lys Val Lys Val Gly Leu Asn Glu Leu Tyr Asn Gly Gln Ile
           435                 440                 445
   Leu Glu Thr Ile Gly Gly Lys Gln Leu Arg Val Phe Val Tyr Arg Thr
       450                 455                 460
   Ala Val Cys Ile Glu Asn Ser Cys Met Glu Lys Gly Ser Lys Gln Gly
   465                 470                 475                 480
   Arg Asn Gly Ala Ile His Ile Phe Arg Glu Ile Ile Lys Pro Ala Glu
                   485                 490                 495
   Lys Ser Leu His Glu Lys Leu Lys Gln Asp Lys Arg Phe Ser Thr Phe
               500                 505                 510
   Leu Ser Leu Leu Glu Ala Ala Asp Leu Lys Glu Leu Leu Thr Gln Pro
           515                 520                 525
   Gly Asp Trp Thr Leu Phe Val Pro Thr Asn Asp Ala Phe Lys Gly Met
       530                 535                 540
   Thr Ser Glu Glu Lys Glu Ile Leu Ile Arg Asp Lys Asn Ala Leu Gln
   545                 550                 555                 560
   Asn Ile Ile Leu Tyr His Leu Thr Pro Gly Val Phe Ile Gly Lys Gly
                   565                 570                 575
   Phe Glu Pro Gly Val Thr Asn Ile Leu Lys Thr Thr Gln Gly Ser Lys
   580 585 590
   Ile Phe Leu Lys Glu Val Asn Asp Thr Leu Leu Val Asn Glu Leu Lys
   595 600 605
   Ser Lys Glu Ser Asp Ile Met Thr Thr Asn Gly Val Ile His Val Val
   610 615 620
   Asp Lys Leu Leu Tyr Pro Ala Asp Thr Pro Val Gly Asn Asp Gln Leu
   625 630 635 640
   Leu Glu Ile Leu Asn Lys Leu Ile Lys Tyr Ile Gln Ile Lys Phe Val
   645 650 655
   Arg Gly Ser Thr Phe Lys Glu Ile Pro Val Thr Val Tyr Thr Thr Lys
   660 665 670
   Ile Ile Thr Lys Val Val Glu Pro Lys Ile Lys Val Ile Glu Gly Ser
   675 680 685
   Leu Gln Pro Ile Ile Lys Thr Glu Gly Pro Thr Leu Thr Lys Val Lys
   690 695 700
   Ile Glu Gly Glu Pro Glu Phe Arg Leu Ile Lys Glu Gly Glu Thr Ile
   705 710 715 720
   Thr Glu Val Ile His Gly Glu Pro Ile Ile Lys Lys Tyr Thr Lys Ile
   725 730 735
   Ile Asp Gly Val Pro Val Glu Ile Thr Glu Lys Glu Thr Arg Glu Glu
   740 745 750
   Arg Ile Ile Thr Gly Pro Glu Ile Lys Tyr Thr Arg Ile Ser Thr Gly
   755 760 765
   Gly Gly Glu Thr Glu Glu Thr Leu Lys Lys Leu Leu Gln Glu Glu Val
   770 775 780
   Thr Lys Val Thr Lys Phe Ile Glu Gly Gly Asp Gly His Leu Phe Glu
   785 790 795 800
   Asp Glu Glu Ile Lys Arg Leu Leu Gln Gly Asp Thr Pro Val Arg Lys
   805 810 815
   Leu Gln Ala Asn Lys Lys Val Gln Gly Ser Arg Arg Arg Leu Arg Glu
   820 825 830
   Gly Arg Ser Gln
   835

## Description

### Field of the Invention

The invention relates to a multi-layer polymeric wrap designed to cover an orthopaedic device and provide osteoinductive formulations *in vivo* to tissues in the vicinity of the implantation. The multi-layer polymeric wrap is applied to an orthopaedic device prior to implantation of the device or concurrently with implantation of the device in a patient. In one embodiment of the invention, the wrap is provided as a pre-formed composition that covers the exterior dimensions of an orthopaedic device. The polymeric wrap comprises, or alternatively consists of, multiple polymer layers wherein each polymer layer comprises different osteoinductive agents and/or different concentrations of osteoinductive agents. Alternatively, the multiple polymer layers of the polymeric wrap comprise different degradation rates, thereby releasing osteoinductive agents at differing rates dictated by the degradation rates of the particular polymer.

### Background of the Invention

Thousands of implant surgeries are performed every year in the United States on patients requiring biomedical implants. For example, more than 168,000 total hip replacements are performed each year in the United States alone. Shindle, M., et al., BioMechanics, 11(2):22-32 (2004).

Unfortunately, a number of implant surgeries each year require revision surgery to correct defects that have developed with the implant devices. For example, as discussed by Croci *et al.* regarding segmental resections of bone tumors, the increased rates of survival of patients having bone tumor resections has led to the discovery of the greater need for revision surgery of implant devices, where previously such observations were less frequent due to the unsuccessful oncologic management of the tumors. Croci et al., Rev. Hosp. Clin. Fac. Med. S. Paulo, 55(5):169-176 (2000).

Devices designed to deliver osteoinductive agents in the vicinity of musculoskeletal implant devices are particularly useful in both primary and revision surgeries, in order to prevent the development of osteolysis in the vicinity of implant devices. Devices of this nature also are useful in preventing the need for future revision surgeries. Devices designed to deliver osteoinductive agents in the vicinity of musculoskeletal implants are particularly useful for both primary and revision surgeries such as shoulder surgeries at the stem of the humeral component; in elbow surgeries, at the stem of the humeral and ulna components; in wrist surgeries, at the stem of the ulna component; in hip surgeries, at the femoral stem, associated with acetabular cup implants, and associated with bone screws; and in knee surgeries, at the femoral stem, at the back side of femoral component articulation, at the tibia stem, the underside of the tibia tray, and at the backside of the patella; and in total shoulder, total hip and total knee replacement surgeries.

US Patent No. 6,648,916 discloses an interbody osteogenic fusion device containing an osteogenic material disposed within an annular pocket between opposite end pieces. US Patent No. 5,947,893 discloses a biodegradable composition incorporatable onto or into a microporous surface of a metal or joint replacement prosthesis. US Patent No. 6,261,586 discloses a bone graft substitute containing a therapeutically effective amount, to stimulate bone growth, of a bone growth factor in a pharmaceutically acceptable carrier. US Patent Publication No. 2004/0158329 discloses a hip-joint prosthesis which may be provided with a coating containing an osteoinductive substance. US Patent Publication No. 2003/0045942 discloses a regenerative bone implant including a biocompatible and biodegradable matrix having pores and optionally further including a bone formation promoter that is disposed in the pores. International Patent Publication No. 95/28973 discloses a porous prosthesis impregnated with a biodegradable polymer. The biodegradable carrier may contain a pharmacologically active substance. None of US Patent Nos. 6,648,916, 5,947,893 and 6,261,583, nor US Patent Publication Nos. 2004/0158329 and 2003/045942, nor International Patent Publication No. 95/28973, disclose or suggest a multi-layer polymeric wrap designed to cover an orthopaedic device, in which at least one osteoinductive formulation is either contained within at least one of the biodegradable polymer sheets or is contained between at least two of the biodegradable polymer sheets.

### Summary of the Invention

Accordingly, there remains a need in the art for devices that are implanted with orthopaedic devices and provide sustained or immediate release of osteoinductive formulations in immediate fashion or over extended periods of time. Applicants describe herein preferred coverings for biomedical implant devices that fulfill the need in the art for coverings that provide osteoinductive formulations in the vicinity of the site of implantation of orthopaedic implant devices.

An embodiment of the invention provides a multi-layer polymeric wrap designed to cover an orthopaedic device, the multi-layer polymeric wrap comprising at least two biodegradable polymer sheets wherein the at least two biodegradable polymer sheets are associated with one another, and wherein the multi-layer polymeric wrap further comprises at least one osteoinductive formulation; wherein at least one osteoinductive formulation is either contained within at least one of the biodegradable polymer sheets or is contained between at least two of the biodegradable polymer sheets.

The invention also discloses a method of preventing or treating the development of osteolytic lesions in an implant patient comprising implanting in said patient a polymeric wrap including an osteoinductive formulation. An additional embodiment of the invention includes a kit including the multi-layer polymeric wrap discussed above.

These and other features of embodiments of the invention will be readily apparent to those skilled in the art upon reading the detailed description that follows.

### Brief Description of the Drawings

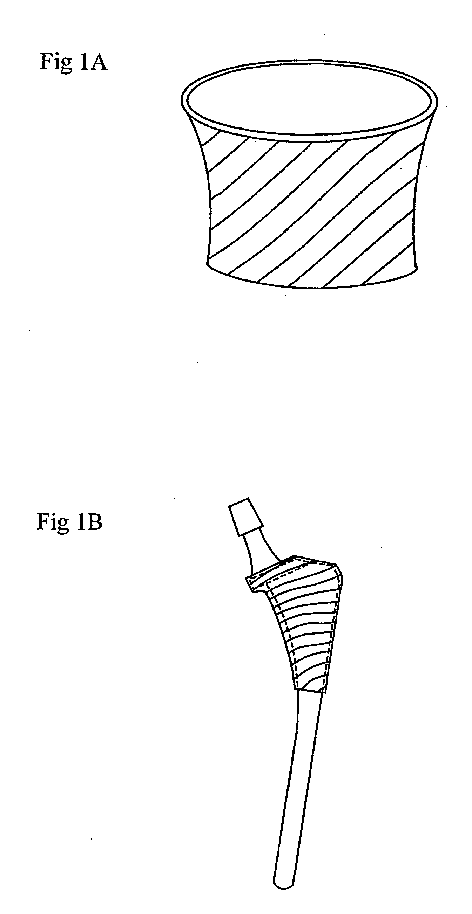

Figures 1A-B illustrate a polymeric wrap that is applied to a femoral stem implant. As can be seen in Figure 1A, the wrap is designed as a sleeve to cover the outer surface of the orthopaedic device prior to implantation. As can be seen in Figure 1B, the wrap fits over exterior portions of the orthopaedic device prior to implantation.

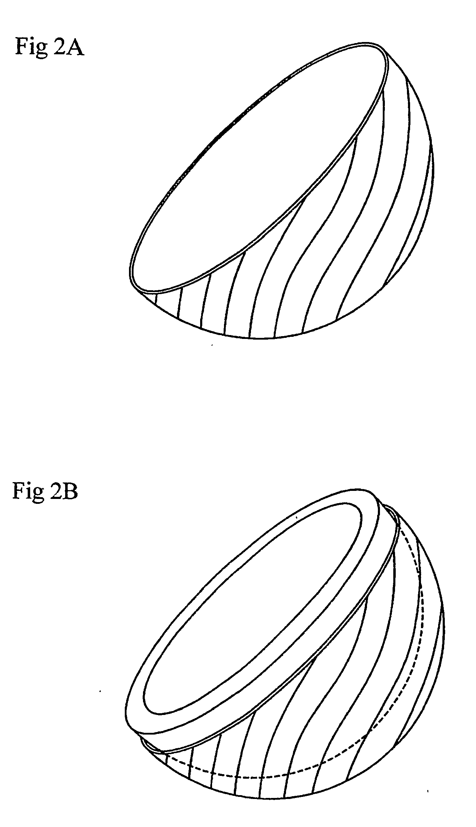

Figures 2A-B illustrate a polymeric wrap designed to cover the exposed surface of an acetabular cup. As can be seen in Figure 1A, the wrap is designed in the shape of the acetabular cup. As can be seen in Figure 1B, the polymer wrap substantially covers the exterior portion of the acetabular cup prior to implantation. The wrap may optionally be pierced by items that assist in securing the acetabular cup to endogenous bone, such as for example, bone or other screws designed to secure the implant.

### Detailed Description of Preferred Embodiments

For the purposes of promoting an understanding of the present invention, reference will now be made to preferred embodiments and specific language will be used to describe the same. The terminology used herein is for the purpose of describing particular embodiments only, and is not intended to limit the scope of the present invention. As used throughout this disclosure, the singular forms "a," "an," and "the" include plural reference unless the context clearly dictates otherwise. Thus, for example, a reference to "an implant" includes a plurality of such implants, as well as a single implant, and a reference to "an osteoinductive agent" is a reference to one or more agents and equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are cited for the purpose of describing and disclosing the various implants, osteoinductive agents, and other components that are reported in the publications and that might be used in connection with the invention. Nothing herein is to be construed as an admission that the invention is not entitled to antedate such disclosures by virtue of prior invention.

As used herein, "bioavailable" shall mean that the osteoinductive agents(s) are provided in vivo in the patient, wherein the osteoinductive agent(s) retain biological activity. By retaining biological activity is meant that the osteoinductive agent(s) retain at least 25% activity, more preferably at least 50% activity, still more preferably at least 75% activity, and most preferably at least 95% or more activity of the osteoinductive agent relative to the activity of the osteoinductive agent prior to implantation.

As used herein, "mature polypeptide" shall mean a post-translationally processed form of a polypeptide. For example, mature polypeptides may lack one or more of a signal peptide, prepropeptide and propeptide domains following expression in a host expression system. One of skill in the art of biology is aware of the meanings of signal peptide, prepropeptide and propeptide domains.

As used herein, "immediate release" shall mean formulations of the invention that provide the osteoinductive formulations in a reasonably immediate period of time.

As used herein, "sustained release" shall mean formulations of the invention that are designed to provide osteoinductive formulations at relatively consistent concentrations in bioavailable form over extended periods of time.
As used herein, "biodegradable" shall mean a polymer that degrades during *in vivo* application. In one embodiment of the invention, the degradation of the polymer produces the polymer monomeric subunits.

As used herein, "polymeric wrap" shall mean a sheet that comprises, or alternatively consists of, one or more polymer layers, each polymer layer optionally comprising one or more osteoinductive agents wherein the osteoinductive agents are present in the multiple polymer layers in similar or differing concentrations. Where the polymeric wrap of the invention comprises, or alternatively consists of, multiple polymer layers, the multiple polymer layers are produced separately and combined to yield the multi-layer polymeric wrap of the invention.

As used herein, "orthopaedic device" shall mean any biomedical implant surgically introduced into a patient and designed to function in conjunction with the skeletal system or bodily joints.

Embodiments of the invention include a multi-layer polymeric wrap including at least two biodegradable polymer sheets. The at least two biodegradable polymer sheets are associated with one another, and the multi-layer polymeric wrap further includes at least one osteoinductive formulation.

The osteoinductive formulations of the invention preferably comprise one or more osteoinductive agent(s) that induce growth of endogenous bone, or tissues related to the endogenous bone such as connective tissues and vascular tissues. Additionally, osteoinductive agents are provided that function to inhibit bone resorption. The osteoinductive formulations comprise osteoinductive agents, such as for example, one or more Bone Morphogenetic Protein (BMP), one or more Connective Tissue Growth Factor (CTGF), one or more Vascular Endothelial Growth Factor (VEGF), Osteoprotegerin (OPG), Periostin and one or more Transforming Growth Factor-*beta* (TGF-β) polynucleotides and polypeptides. The osteoinductive agent is provided in bioavailable form from the polymeric wrap as a component of an immediate release or a sustained release formulation.

In one embodiment of the invention, the multi-layer polymeric wrap comprises biodegradable polymers that release osteoinductive formulation(s) based on the degradation rates of the biodegradable polymers containing the osteoinductive formulation(s). The invention further discloses a method for preventing and treating osteolytic lesion formation in the vicinity of the orthopaedic implant in a patient. The invention also discloses a method of enhancing the growth of tissues surrounding sites of implantation.

The invention includes multi-layer polymeric wraps comprising osteoinductive formulations useful to promote the growth of endogenous bone, connective tissue and vascular tissue, as well as for the prevention of bone resorption. The osteoinductive formulations useful with the invention include one or more osteoinductive agents. The osteoinductive formulations may be incorporated into the polymer composition that forms the multi-layer polymeric wrap. Alternatively, the osteoinductive formulations can be applied to the outer surface of the polymeric wrap prior to implantation.

In one embodiment of the invention, osteoinductive formulations used with the multi-layer polymeric wrap are admixed with sustained release polymers and molded into a preformed solid multi-layer polymeric wrap. The solid multi-layer polymeric wrap can be generated using molds into which liquid polymers are added prior to curing or fixing of the polymer solution to form preformed polymer sheets. Preferably, the duration of sustained release of the osteoinductive agents from the multi-layer polymeric wrap may be manipulated by increasing the resistance to biodegradation of the polymer by, for example, introducing or increasing the percentage of biostable polymers contained within the biodegradable polymer compositions.

In another preferred embodiment of the invention, the polymeric wrap consists of layers of polymer, optionally preformed, that are individually applied over the orthopaedic device prior to implantation. The layers that are applied over the orthopaedic device prior to implantation can be used to form a multi-layer polymeric wrap that is implanted with the orthopaedic device. Optionally, each polymer layer of the multi-layer wrap is formulated to have different degradation characteristics. The use of multiple layer polymeric wraps having different degradation rates enables one of skill in the art to construct polymeric wraps with customized degradation characteristics.

For example, if a patient is in need of a substantial dosage of osteoinductive agent as an initial phase and a sustained concentration of osteoinductive agent over extended periods of time, the multi-layer wrap may be formulated with a high concentration of osteoinductive agent in an outer polymer layer having a high rate of biodegradation, thereby enabling liberation of a high concentration of osteoinductive agent initially, and one or more inner polymer layers having osteoinductive agents and lower rates of biodegradation. Alternatively, if it is determined that the patient will benefit from an extended duration of release of osteoinductive agent, the multi-layer polymeric wrap may be formulated with one or more outer layers of biodegradable polymer optionally comprising osteoinductive agent, and one or more inner layers of biodegradable polymer comprising osteoinductive agent having a reduced rate of biodegradation *in vivo.* In this manner, the multi-layer polymeric wrap provides a relatively sustained source of osteoinductive agent. The multi-layer polymeric wrap may contain one or more osteoinductive agents in the osteoinductive formulations introduced into the polymeric wrap.

In another embodiment of the invention, the multi-layer polymeric wrap comprises, or alternatively consists of, at least one osteoinductive agent contained in differing concentrations throughout each of the individual polymer layers of the polymeric wrap. For example, one or more inner layers of a multi-layer biodegradable polymeric wrap may contain enhanced concentrations of an osteoinductive agent, while the one or more outer layers of the biodegradable polymeric wrap may contain diminished concentrations of an osteoinductive agent. In this embodiment, a steadily increasing concentration of osteoinductive agent is introduced into the patient *in vivo.* Alternatively, where the osteoinductive agent has diminished activity *in vivo* over time, inner polymer layers of the polymeric wrap containing increased concentrations of the osteoinductive agent may help to compensate for diminished activity of the one or more osteoinductive agents in the inner layers, thereby providing a relatively stable concentration of osteoinductive agent (in terms of activity) throughout the useful life of the biodegradable polymeric wrap. In other words, enhanced concentrations of osteoinductive agent in inner layers of a multi-layer polymeric wrap compensate for diminished levels of bioavailable, active osteoinductive agent over time.

Another aspect of the invention relates to osteoinductive formulations incorporated within the multi-layer polymeric wrap. Osteoinductive formulations preferably include one or more osteoinductive agents, and provide the one or more agents in bioavailable form as either immediate release or sustained release formulations. Osteoinductive formulations further optionally comprise one or more of the following components: antibiotics, carriers, bone marrow aspirate, bone marrow concentrate, demineralized bone matrix, immunosuppressives, agents that enhance isotonicity and chemical stability, and any combination of one or more, including all, of the recited components.

The osteoinductive formulations of embodiments of the invention are available as immediate release formulations or sustained release formulations. One of skill in the art of implant surgery is able to determine whether a patient would benefit from immediate release formulations or sustained release formulations based on factors such as age and level of physical activity. Therefore, the osteoinductive formulations of the invention are available as immediate release formulations, sustained release formulations, or both.

Representative immediate release formulations are liquid formulations comprising at least one osteoinductive agent(s) applied to the surface of the polymeric wrap. The liquid formulations provide osteoinductive agent(s) in bioavailable form at rates dictated by the fluid properties of the liquid formulation, such as diffusion rates at the site of implantation, the influence of endogenous fluids, etc. Examples of suitable liquid formulations comprise water, saline, or other acceptable fluid mediums that will not induce host immune responses.

Immediate release formulations of the invention provide the osteoinductive formulations in a reasonably immediate period of time, although factors such as proximity to bodily fluids, composition of the formulations, etc, will influence the period of time within which the osteoinductive agent(s) is liberated from the formulations. However, immediate release formulations are not designed to retain the one or more osteoinductive agents for extended periods of time, and typically will lack a biodegradable polymer as a component of the immediate release formulation.

In another embodiment of the invention, osteoinductive formulations are available in sustained release formulations that provide the osteoinductive formulation(s) in bioavailable form over extended periods of time. The duration of release from the sustained release formulations is dictated by the nature of the formulation and other factors discussed *supra,* such as for example proximity to bodily fluids, as well as density of application of the formulations, degradation rates of biodegradeable polymers comprising the osteoinductive formulations, and other factors. However, sustained release formulations are designed to provide osteoinductive agents in the formulations at relatively consistent concentrations in bioavailable form over extended periods of time. Biodegradable sustained release polymers useful with the polymeric wraps are well known in the art and include, but are not limited to, polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malic acid), poly(amino acids), polyvinylpyrrolidone, polyethylene glycol, polyhydroxycellulose, chitin, chitosan, poly(L-lactic acid), poly(lactide-co-glycolide), poly(hydroxybutyrate-co-valerate), and copolymers, terpolymers, or combinations or mixtures of the above materials. The release profile of the biodegradable polymer can further be modified by inclusion of biostable polymers that influence the biodegradation rate of the polymer composition. Biostable polymers that could be incorporated into the biodegradable polymers, thereby influencing the rates of biodegradation, include but are not limited to silicones, polyesters, vinyl homopolymers and copolymers, acrylate homopolymers and copolymers, polyethers, and cellulosics.

The biodegradable polymers can be solid form polymers or alternatively can be liquid polymers that solidify in a reasonable time after application. Suitable liquid polymers formulations include, but are not limited to those polymer compositions disclosed in, for example, U.S. Patent Nos. 5,744,153, 4,938,763, 5,278,201 and 5,278,202, These patents disclose liquid polymer compositions that are useful as controlled drug-release compositions or as implants. The liquid prepolymer has at least one polymerizable ethylenically unsaturated group (e.g., an acrylic-ester-terminated prepolymer). If a curing agent is employed, the curing agent is typically added to the composition just prior to use. The osteoinductive agents are added to the liquid prepolymer prior to curing or fixing of the prepolymer. The prepolymer remains a liquid for a short period of time after the introduction of the curing agent. The mixture then solidifies to form a solid composition. The liquid polymer compositions may be administered to a patient in liquid form, and will then solidify or cure at the site of introduction to form a solid polymer coating composition. Alternatively, the liquid polymer may be injected into a pre-formed mold to generate a polymeric wrap, preformed to fit over the exterior dimensions of an orthopaedic device prior to implantation in a patient. Biodegradable forms of the polymers are contemplated, and mixtures of biodegradable and biostable polymers that influence the rate of biodegradation of the polymer are further contemplated.

Osteoinductive formulations of the invention further contemplate the use of aqueous and non-aqueous peptide formulations that maintain stability of the osteoinductive agents over extended periods of time. Non-limiting examples of aqueous and non-aqueous formulations useful for the long-term stability of osteoinductive agent(s) include those formulations provided in U.S. Patent Nos. 5,916,582; 5,932,547, and 5,981,489,.

An amount of the liquid sustained-release polymer composition may be dispensed onto the polymeric wrap, such as, for example, by spraying, painting or squirting, and the liquid formulation solidifies following administration to provide a sustained release formulation.

In another embodiment of the invention, the liquid compositions that are useful for the delivery of osteoinductive formulations *in vivo* include conjugates of the osteoinductive agent with a water-insoluble biocompatible polymer, with the dissolution of the resultant polymer-active agent conjugate in a biocompatible solvent to form a liquid polymer system. In addition, the liquid polymer system may also include a water-insoluble biocompatible polymer which is not conjugated to the osteoinductive agent. In one embodiment of the invention, these liquid compositions may be introduced into the body of a subject in liquid form. The liquid composition then solidifies or coagulates *in situ* to form a controlled release implant where the osteoinductive agent is conjugated to the solid matrix polymer.

Osteoinductive agents of embodiments of the invention can be administered in the osteoinductive formulations as isolated polypeptides or polynucleotides. Polynucleotide compositions of the isolated osteoinductive agents include, but are not limited to, isolated Bone Morphogenetic Protein (BMP), Vascular Endothelial Growth Factor (VEGF), Connective Tissue Growth Factor (CTGF), Osteoprotegerin, Periostin and Transforming Growth Factor beta (TGF-β) polynucleotides. Polynucleotide compositions of the osteoinductive agents include, but are not limited to, gene therapy vectors harboring polynucleotides encoding the osteoinductive polypeptides of interest. Gene therapy methods require a polynucleotide which codes for the osteoinductive polypeptide operatively linked or associated to a promoter and any other genetic elements necessary for the expression of the osteoinductive polypeptide by the target tissue. Such gene therapy and delivery techniques are known in the art, *See,* for example, International Publication No. WO90/11092, . Suitable gene therapy vectors include, but are not limited to, gene therapy vectors that do not integrate into the host genome. Alternatively, suitable gene therapy vectors include, but are not limited to, gene therapy vectors that integrate into the host genome.

In one embodiment, the polynucleotide of the invention is delivered in plasmid formulations. Plasmid DNA or RNA formulations refer to sequences encoding osteoinductive polypeptides that are free from any delivery vehicle that acts to assist, promote or facilitate entry into the cell, including viral sequences, viral particles, liposome formulations, lipofectin or precipitating agents and the like. Optionally, gene therapy compositions of the invention can be delivered in liposome formulations and lipofectin formulations, which can be prepared by methods well known to those skilled in the art. General methods are described, for example, in U.S. Pat. Nos. 5,593,972, 5,589,466, and 5,580,859,.

Gene therapy vectors further comprise suitable adenoviral vectors including, but not limited to for example, those described in Kozarsky and Wilson, Curr. Opin. Genet. Devel., 3:499-503 (1993); Rosenfeld et al., Cell, 68:143-155 (1992); Engelhardt et al., Human Genet. Ther., 4:759-769 (1993); Yang et al., Nature Genet., 7:362-369 (1994); Wilson et al., Nature, 365:691-692 (1993); and U.S. Pat. No. 5,652,224, .

Polypeptide compositions of the isolated osteoinductive agents include, but are not limited to, isolated Bone Morphogenetic Protein (BMP), Vascular Endothelial Growth Factor (VEGF), Connective Tissue Growth Factor (CTGF), Osteoprotegerin, Periostin and Transforming Growth Factor beta (TGF-β) polypeptides. Polypeptide compositions of the osteoinductive agents include, but are not limited to, isolated full length proteins, fragments and variants thereof. In a preferred embodiment of the invention, polypeptide fragments of the osteoinductive agents comprise, or alternatively consist of, propeptide forms of the isolated full length polypeptides. In a particularly preferred embodiment of the invention, polypeptide fragments of the osteoinductive agents comprise, or alternatively consist of, mature forms of the isolated full length polypeptides. Also preferred are the polynucleotides encoding the propeptide and mature polypeptides of the osteoinductive agents.

Variants of the osteoinductive agents of the invention include, but are not limited to, protein variants that are designed to increase the duration of activity of the osteoinductive agent *in vivo.* Preferred embodiments of variant osteoinductive agents include, but are not limited to, full length proteins or fragments thereof that are conjugated to polyethylene glycol (PEG) moieties to increase their half-life *in vivo* (also known as pegylation). Methods of pegylating polypeptides are well known in the art (See, e.g., U.S. Patent No. 6,552,170 and European Patent No. 0,401,384 as examples of methods of generating pegylated polypeptides).

In another embodiment of the invention, the osteoinductive agent(s) are provided in the osteoinductive formulation(s) as fusion proteins. In one embodiment, the osteoinductive agent(s) are available as fusion proteins with the F_{c} portion of human IgG. In another embodiment of the invention, the osteoinductive agent(s) of the invention are available as hetero- or homodimers or multimers. Examples of preferred fusion proteins include, but are not limited to, ligand fusions between mature osteoinductive polypeptides and the F_{c} portion of human Immunoglobulin G (IgG). Methods of making fusion proteins and constructs encoding the same are well known in the art.

Osteoinductive agents of the invention that are included with the osteoinductive formulations are sterile. In a non-limiting method, sterility is readily accomplished for example by filtration through sterile filtration membranes (e.g., 0.2 micron membranes or filters).

In one embodiment of the invention, the polymeric wrap is provided without osteoinductive formulations incorporated within the wrap. In this embodiment of the invention, the osteoinductive formulations are introduced to the surface of the polymeric wrap prior to implantation of the wrap in a patient. Alternatively, the osteoinductive formulations are placed between the biodegradable polymer sheets of a multi-layer polymeric wrap. In such a situation, osteoinductive agents generally are placed into a container having a sterile access port, for example, an intravenous solution bag or vial having a stopper pierceable by a hypodermic injection needle. In one embodiment, osteoinductive agents and prepared osteoinductive formulations are stored in separate containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous osteoinductive agent solution, and the resulting mixture is lyophilized. The osteoinductive agent is prepared by reconstituting the lyophilized agent prior to administration in an appropriate solution, admixed with the prepared osteoinductive formulations and administered to the surface of the polymeric wrap or between polymer layers of a multi-layer polymeric wrap prior to or concurrent with implantation into a patient. Application may be achieved by immersion of the polymeric wrap in osteoinductive formulations, by spraying osteoinductive formulations on the surface of the polymeric wrap, or by any other means of application.

As one of skill in the art will recognize, the concentrations of osteoinductive agent can be variable based on the desired length or degree of osteoinduction. Similarly, one of skill in the art will understand that the duration of sustained release can be modified by the manipulation of the compositions comprising the sustained release formulation, such as for example, modifying the percent of biostable polymers found within a sustained release polymer.

A method to provide liquid compositions which are useful to form polymeric sheets for the delivery of osteoinductive agents *in vivo* is to conjugate the active agent with a water-insoluble biocompatible polymer and dissolve the resultant polymer-active agent conjugate in a biocompatible solvent to form a liquid polymer system similar to that described in U.S. Pat. Nos. 4,938,763, 5,278,201 and 5,278,202. The water-insoluble biocompatible polymers may be those described in the above patents or related copolymers. In addition, the liquid polymer system may also include a water-insoluble biocompatible polymer which is not conjugated to the active agent. In one embodiment of the invention, these liquid compositions may be introduced into the body of a subject in liquid form. The liquid composition then solidifies or coagulates *in situ* to form a controlled release implant where the active agent is conjugated to the solid matrix polymer.

Osteoinductive formulations of the invention optionally further comprise demineralized bone matrix compositions (hereinafter "DBM" compositions), bone marrow aspirate, bone marrow concentrate, or combinations or permutations of any of the same. Methods for producing DBM are well known in the art, and DBM may be obtained following the teachings of O'Leary et al (U.S. Patent No. 5,073,373) or by obtaining commercially available DBM formulations such as, for example, AlloGro® available from suppliers such as AlloSource® (Centennial, CO). Methods of obtaining bone marrow aspirates as well as devices facilitating extraction of bone marrow aspirate are well known in the art and are described, for example, by Turkel et al in U.S. Patent No. 5,257,632.

Osteoinductive formulations of the invention optionally further comprise antibiotics that are administered with the osteoinductive agent. As discussed by Vehmeyer *et al.,* the possibility exists that bacterial contamination can occur for example due to the introduction of contaminated allograft tissue from living donors. Vehmeyer, SB, et al., Acta Orthop Scand, 73(2):165-169 (2002). Antibiotics of the invention are also co-administered with the osteoinductive formulations to prevent infection by obligate or opportunistic pathogens that are introduced to the patient during implant surgery.

Antibiotics useful with the osteoinductive formulations of the invention include, but are not limited to, amoxicillin, beta-lactamases, aminoglycosides, beta-lactam (glycopeptide), clindamycin, chloramphenicol, cephalosporins, ciprofloxacin, erythromycin, fluoroquinolones, macrolides, metronidazole, penicillins, quinolones, rapamycin, rifampin, streptomycin, sulfonamide, tetracyclines, trimethoprim, trimethoprim-sulfamthoxazole, and vancomycin. In addition, one skilled in the art of implant surgery or administrators of locations in which implant surgery occurs may prefer the introduction of one or more of the above-recited antibiotics to account for nosocomial infections or other factors specific to the location where the surgery is conducted. Accordingly, the invention further contemplates that one or more of the antibiotics described herein, and any combination of one or more of the same antibiotics, may be included in the osteoinductive formulations of the invention.

The osteoinductive formulations of the invention optionally further comprise immunosuppressive agents, particularly in circumstances where allograft compositions are administered to the patient. Suitable immunosuppressive agents that may be administered in combination with the osteoinductive formulations of the invention include, but are not limited to, steroids, cyclosporine, cyclosporine analogs, cyclophosphamide, methylprednisone, prednisone, azathioprine, FK-506, 15-deoxyspergualin, and other immunosuppressive agents that act by suppressing the function of responding T cells. Other immunosuppressive agents that may be administered in combination with the osteoinductive formulations of the invention include, but are not limited to, prednisolone, methotrexate, thalidomide, methoxsalen, rapamycin, leflunomide, mizoribine (bredinin^{™}), brequinar, deoxyspergualin, and azaspirane (SKF 105685), Orthoclone OKT^{™} 3 (muromonab-CD3). Sandimmune^{™}, Neoral^{™}, Sangdya^{™} (cyclosporine), Prograf^{™} (FK506, tacrolimus), Cellcept^{™} (mycophenolate motefil, of which the active metabolite is mycophenolic acid), Imuran^{™} (azathioprine), glucocorticosteroids, adrenocortical steroids such as Deltasone^{™} (prednisone) and Hydeltrasol^{™} (prednisolone), Folex^{™} and Mexate^{™} (methotrxate), Oxsoralen-Ultra^{™} (methoxsalen) and Rapamuen^{™} (sirolimus).

Osteoinductive formulations of the invention may optionally further comprise a carrier vehicle such as water, saline, Ringer's solution, calcium phosphate based carriers, or dextrose solution. Non-aqueous vehicles such as fixed oils and ethyl oleate are also useful herein, as well as liposomes.

In one embodiment of the invention, collagen is used as a carrier or as the basis of a polymeric wrap for the delivery of osteoinductive formulations. In another embodiment of the invention, collagen in combination with glycosaminoglycan is utilized as a carrier for the osteoinductive formulations, as described in U.S. Patent No. 5,922,356, . The content of glycosaminoglycan in the formulation is preferably less than 40% by weight of the formulation, more preferably 1-10%. Collagen is preferably 20-95% by weight of the formulation, more preferably 40-60 (wt/wt)%.

Any collagen may be used as a carrier for osteoinductive formulations. Examples of suitable collagen to be used as a carrier include, but are not limited to, human collagen type I, human collagen type II, human collagen type III, human collagen type IV, human collagen type V, human collagen type VI, human collagen type VII, human collagen type VIII, human collagen type IX, human collagen type X, human collagen type XI, human collagen type XII, human collagen type XIII, human collagen type XIV, human collagen type XV, human collagen type XVI, human collagen type XVII, human collagen type XVIII, human collagen type XIX, human collagen type XXI, human collagen type XXII, human collagen type XXIII, human collagen type XXIV, human collagen type XXV, human collagen type XXVI, human collagen type XXVII, and human collagen type XXVIII, and combinations thereof. Collagen carriers useful with the invention further comprise, or alternatively consist of, hetero- and homo-trimers of any of the above-recited collagen types. In a preferred embodiment of the invention, collagen carriers comprise, or alternatively consist of, hetero- or homo-trimers of human collagen type I, human collagen type II, and human collagen type III, or combinations thereof.

The collagen recited *supra* is substantially incorporated as the polymeric wrap. For example, collagen generated as a polymeric wrap incorporates osteoinductive formulations incorporating one or more osteoinductive agents. The collagen wrap comprising at least one osteoinductive agent is introduced into the patient as a wrap substantially covering the orthopaedic device at the time of implantation. After implantation, the collagen wrap liberates osteoinductive formulations comprising osteoinductive agents. Examples of useful collagen wraps include, but are not limited to, collagen sheets and reconstituted collagen sheets derived from bovine sources. In another embodiment of the invention, the collagen wraps are covered by one or more biodegradable polymer layers, thereby forming a variant multi-layer polymeric sheet.

The collagen utilized as a carrier in a polymeric wrap may be human or non-human, as well as recombinant or non-recombinant. In a preferred embodiment of the invention, the collagen utilized as a carrier is recombinant collagen. Methods of making recombinant collagen are known in the art, for example, by using recombinant methods such as those methods described in U.S. Patent No. 5,895,833 (trangenic production), J. Myllyharju, et al., Biotechnology of Extracellular Matrix, 353-357 (2000) (production of recombinant human types I-III in *Pichia pastoris*), Wong Po Foo, C., et al., Adv. Drug Del. Rev., 54:1131-1143 (2002), or by Toman, P.D., et al., J. Biol. Chem., 275(30):23303-23309 (2001),. Alternatively, recombinant human collagen types are obtained from commercially available sources, such as for example, as provided by FibroGen (San Francisco, California).

The osteoinductive formulations of the invention further optionally include substances that enhance isotonicity and chemical stability. Such materials are non-toxic to patients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugaralcohols such as mannitol or sorbitol; counterions such as sodium; and/or nonionicsurfactants such as polysorbates, poloxamers, or PEG.

Osteoinductive formulations of the invention further comprise isolated osteoinductive agents. Isolated osteoinductive agents of the invention promote the growth of endogenous bone, or aid in preventing resorption of bone tissue surrounding the implant by osteoclasts. Isolated osteoinductive agents of the invention are available as polypeptides or polynucleotides. Isolated osteoinductive agents of the invention comprise full length proteins and fragments thereof, as well as polypeptide variants or mutants of the isolated osteoinductive agents provided herein.

In another embodiment of the invention, osteoinductive agent polypeptides are available as heterodimers or homodimers, as well as multimers or combinations thereof.

Recombinantly expressed proteins may be in native forms, truncated analogs, muteins, fusion proteins, and other constructed forms capable of inducing bone, cartilage, or other types of tissue formation as demonstrated by in vitro and ex vivo bioassays and in vivo implantation in mammals, including humans.

The invention further contemplates the use of polynucleotides and polypeptides having at least 95% homology, more preferably 97%, and even more preferably 99% homology to the isolated osteoinductive agent polynucleotides and polypeptides provided herein. Typical osteoinductive formulations comprise isolated osteoinductive agent at concentrations of from about 0.1 mg/ml to 100 mg/ml, preferably 1-10 mg/ml, at a pH of about 3 to 8.

In one embodiment of the invention, isolated osteoinductive agents include one or more polynucleotides or polypeptides of members of the family of Bone Morphogenetic Proteins ("BMPs"). BMPs are a class of proteins thought to have osteoinductive or growth-promoting activities on endogenous bone tissue. Known members of the BMP family include, but are not limited to, BMPs-1, BMP-2, BMP-3, BMP-4, BMP-5, BMP-6, BMP-7, BMP-8, BMP-9, BMP-10, BMP-11, BMP-12, BMP-13, BMP-15, BMP-16, BMP-17, and BMP-18.
BMPs useful as isolated osteoinductive agents include, but are not limited to, the following BMPs:
BMP-1 polynucleotides and polypeptides corresponding to SEQ ID NO:1, SEQ ID NO:2, SEQ ID NO:3 and SEQ ID NO:4, as well as mature BMP-1 polypeptides and polynucleotides encoding the same;
BMP-2 polynucleotides and polypeptides corresponding to SEQ ID NO:5 and SEQ ID NO:6, as well as mature BMP-2 polypeptides and polynucleotides encoding the same;
BMP-3 polynucleotides and polypeptides corresponding to SEQ ID NO:7 and SEQ ID NO:8, as well as mature BMP-3 polypeptides and polynucleotides encoding the same;
BMP-4 polynucleotides and polypeptides corresponding to SEQ ID NO:9 and SEQ ID NO: 10, as well as mature BMP-4 polypeptides and polynucleotides encoding the same;
BMP-5 polynucleotides and polypeptides corresponding to SEQ ID NO:11, SEQ ID NO: 12, SEQ ID NO:13 and SEQ ID NO: 14, as well as mature BMP-5 polypeptides and polynucleotides encoding the same;
BMP-6 polynucleotides and polypeptides corresponding to SEQ ID NO:15, SEQ ID NO: 16, SEQ ID NO:17 and SEQ ID NO:18, as well as mature BMP-6 polypeptides and polynucleotides encoding the same;
BMP-7 polynucleotides and polypeptides corresponding to SEQ ID NO:19, SEQ ID NO:20, SEQ ID NO:21 and SEQ ID NO:22, as well as mature BMP-7 polypeptides and polynucleotides encoding the same;
BMP-8 polynucleotides and polypeptides corresponding to SEQ ID NO:23, SEQ ID NO:24, SEQ ID NO:25 and SEQ ID NO:26, as well as mature BMP-8 polypeptides and polynucleotides encoding the same;
BMP-9 polynucleotides and polypeptides corresponding to SEQ ID NO:27 and SEQ ID NO:28, as well as mature BMP-9 polypeptides and polynucleotides encoding the same;
BMP-10 polynucleotides and polypeptides corresponding to SEQ ID NO:29, SEQ ID NO:30, SEQ ID NO:31 and SEQ ID NO:32, as well as mature BMP-10 polypeptides and polynucleotides encoding the same;
BMP-11 polynucleotides and polypeptides corresponding to SEQ ID NO:33 and SEQ ID NO:34, as well as mature BMP-11 polypeptides and polynucleotides encoding the same;
BMP-12 polynucleotides and polypeptides corresponding to SEQ ID NO:35 and SEQ ID NO:36, as well as mature BMP-12 polypeptides and polynucleotides encoding the same;
BMP-13 polynucleotides and polypeptides corresponding to SEQ ID NO:37 and SEQ ID NO:38, as well as mature BMP-13 polypeptides and polynucleotides encoding the same;
BMP-15 polynucleotides and polypeptides corresponding to SEQ ID NO:39, SEQ ID NO:40, SEQ ID NO:41 and SEQ ID NO:42, as well as mature BMP-15 polypeptides and polynucleotides encoding the same;
BMP-16 polynucleotides and polypeptides corresponding to SEQ ID NO:43, SEQ ID NO:44, SEQ ID NO:45 and SEQ ID NO:46, as well as mature BMP-16 polypeptides and polynucleotides encoding the same;
BMP-17 polynucleotides and polypeptides corresponding to SEQ ID NO:47 and SEQ ID NO:48, as well as mature BMP-17 polypeptides and polynucleotides encoding the same; and
BMP-18 polynucleotides and polypeptides corresponding to SEQ ID NO:49 and SEQ ID NO:50, as well as mature BMP-18 polypeptides and polynucleotides encoding the same.

BMPs utilized as osteoinductive agents of the invention comprise, or alternatively consist of, one or more of BMP-1; BMP-2; BMP-3; BMP-4; BMP-5; BMP-6; BMP-7; BMP-8; BMP-9; BMP-10; BMP-11; BMP-12; BMP-13; BMP-15; BMP-16; BMP-17; and BMP-18; as well as any combination of one or more of these BMPs, including full length BMPs or fragments thereof, or combinations thereof, either as polypeptides or polynucleotides encoding said polypeptide fragments of all of the recited BMPs. The isolated BMP osteoinductive agents may be administered as polynucleotides, polypeptides, or combinations of both.

In a particularly preferred embodiment of the invention, isolated osteoinductive agents comprise, or alternatively consist of, BMP-2 polynucleotides or polypeptides or mature fragments of the same.

In another embodiment of the invention, isolated osteoinductive agents include osteoclastogenesis inhibitors to inhibit bone resorption of the bone tissue surrounding the site of implantation of the implant by osteoclasts.

Osteoclast and Osteoclastogenesis inhibitors include, but are not limited to, Osteoprotegerin polynucleotides and polypeptides corresponding to SEQ ID NO:51, SEQ ID NO:52, SEQ ID NO:53 and SEQ ID NO:54, as well as mature Osteoprotegerin polypeptides and polynucleotides encoding the same. Osteoprotegerin is a member of the TNF-receptor superfamily and is an osteoblast-secreted decoy receptor that functions as a negative regulator of bone resorption. This protein specifically binds to its ligand, osteoprotegerin ligand (TNFSF11/OPGL), both of which are key extracellular regulators of osteoclast development.

Osteoclastogenesis inhibitors further include, but are not limited to, chemical compounds such as bisphosphonate, 5-lipoxygenase inhibitors such as those described in U.S. Patent Nos. 5,534,524 and 6,455,541 , heterocyclic compounds such as those described in U.S. Patent No. 5,658,935, 2,4-dioxoimidazolidine and imidazolidine derivative compounds such as those described in U.S. Patent Nos. 5,397,796 and 5,554,594, sulfonamide derivatives such as those described in U.S. Patent No. 6,313,119, and acylguanidine compounds such as those described in U.S. Patent No. 6,492,356.

In another embodiment of the invention, isolated osteoinductive agents include one or more polynucleotides or polypeptides of members of the family of Connective Tissue Growth Factors ("CTGFs"). CTGFs are a class of proteins thought to have growth-promoting activities on connective tissues. Known members of the CTGF family include, but are not limited to, CTGF-1, CTGF-2, and CTGF-4.

CTGFs useful as isolated osteoinductive agents include, but are not limited to, the following CTGFs:
CTGF-1 polynucleotides and polypeptides corresponding to SEQ ID NO:55, SEQ ID NO:56, SEQ ID NO:57 and SEQ ID NO:58, as well as mature CTGF-1 polypeptides and polynucleotides encoding the same.
CTGF-2 polynucleotides and polypeptides corresponding to SEQ ID NO:59 and SEQ ID NO:60, as well as mature CTGF-2 polypeptides and polynucleotides encoding the same.

CTGF-4 polynucleotides and polypeptides corresponding to SEQ ID NO:61 and SEQ ID NO:62, as well as mature CTGF-4 polypeptides and polynucleotides encoding the same.

In another embodiment of the invention, isolated osteoinductive agents include one or more polynucleotides or polypeptides of members of the family of Vascular Endothelial Growth Factors ("VEGFs"). VEGFs are a class of proteins thought to have growth-promoting activities on vascular tissues. Known members of the VEGF family include, but are not limited to, VEGF-A, VEGF-B, VEGF-C, VEGF-D and VEGF-E.

VEGFs useful as isolated osteoinductive agents include, but are not limited to, the following VEGFs:
VEGF-A polynucleotides and polypeptides corresponding to SEQ ID NO:63 and SEQ ID NO:64, as well as mature VEGF-A polypeptides and polynucleotides encoding the same.
VEGF-B polynucleotides and polypeptides corresponding to SEQ ID NO:65 and SEQ ID NO:66, as well as mature VEGF-B polypeptides and polynucleotides encoding the same.
VEGF-C polynucleotides and polypeptides corresponding to SEQ ID NO:67 and SEQ ID NO:68, as well as mature VEGF-C polypeptides and polynucleotides encoding the same.
VEGF-D polynucleotides and polypeptides corresponding to SEQ ID NO:69 and SEQ ID NO:70, as well as mature VEGF-D polypeptides and polynucleotides encoding the same.
VEGF-E polynucleotides and polypeptides corresponding to SEQ ID NO:71 and SEQ ID NO:72, as well as mature VEGF-E polypeptides and polynucleotides encoding the same.

In another embodiment of the invention, isolated osteoinductive agents include one or more polynucleotides or polypeptides of Transforming Growth Factor-beta genes ("TGF-βs"). TGF-βs are a class of proteins thought to have growth-promoting activities on a range of tissues, including connective tissues. Known members of the TGF-β family include, but are not limited to, TGF-β-1, TGF-β-2, and TGF-β-3.

TGF-βs useful as isolated osteoinductive agents include, but are not limited to, the following TGF-βs:
TGF-β-1 polynucleotides and polypeptides corresponding to SEQ ID NO:73, SEQ ID NO:74, SEQ ID NO:75 and SEQ ID NO:76, as well as mature TGF-β-1 polypeptides and polynucleotides encoding the same.
TGF-β-2 polynucleotides and polypeptides corresponding to SEQ ID NO:77, SEQ ID NO:78, SEQ ID NO:79 and SEQ ID NO:80, as well as mature TGF-β-2 polypeptides and polynucleotides encoding the same.
TGF-β-3 polynucleotides and polypeptides corresponding to SEQ ID NO:81 and SEQ ID NO:82, as well as mature TGF-β-3 polypeptides and polynucleotides encoding the same.

In another embodiment of the invention, isolated osteoinductive agents include polynucleotides and polypeptides promoting bone adhesion, such as Periostin polynucleotides and polypeptides that are thought to function as adhesion molecules in bone formation.

Bone adhesion promoters include, but are not limited to, Periostin polynucleotides and polypeptides corresponding to SEQ ID NO:83 and SEQ ID NO:84, as well as mature Periostin polypeptides and polynucleotides encoding the same.

In another embodiment of the invention, isolated osteoinductive agents include one or more members of any one of Bone Morphogenetic Proteins (BMPs), Connective Tissue Growth Factors (CTGFs), Vascular Endothelial Growth Factors (VEGFs), Osteoprotegerin or any of the other osteoclastogenesis inhibitors, Periostin, and Transforming Growth Factor-betas (TGF-βs), and any combination of these osteoinductive agents.

Polymers utilized to produce polymeric wraps include any polymers capable of forming a biologically acceptable and biodegradable film. Polymeric wraps of the invention comprise biodegradable polymer layers, wherein the biodegradable polymer layers optionally contain osteoinductive formulations within the polymer layers. In one embodiment of the invention, multi-layer polymeric wraps are produced by synthesizing at least two individual polymer layers comprising one or more osteoinductive agents as a component of osteoinductive formulations. The at least two individual polymer layers are then combined by, for example, laying one of the polymer layers above the one or more other layers to produce a multi-layer polymeric wrap. Optionally, biodegradable cements or other bio-compatible adhesives are used in small quantities as adhesives between the at least two individual polymer layers.

In another embodiment of the invention, the osteoinductive formulations are applied to the surface of the biodegradable polymer layers prior to the addition of the next polymer layer to the multi-layer polymeric wrap. In this embodiment, the osteoinductive formulations are contained as a liquid layer between the at least two individual polymer layers of the polymeric wrap. Upon degradation of the one or more polymer layers, osteoinductive formulations are released from the polymeric wrap and are available in bioactive form.

The polymeric wrap may also be created by producing the polymer layers in liquid form (prepolymers) and admixing osteoinductive formulations, applying the prepolymer liquids to a preformed mold, and curing or fixing the liquid prepolymers to create a preformed polymer wrap that is preformed to fit the exterior dimensions of an orthopaedic device. In this embodiment, the preformed polymeric wrap is placed around the exterior dimensions of the orthopaedic device prior to or concurrent with implantation into a patient.

In an alternative embodiment of this invention, a preformed multi-layer polymeric wrap is generated by producing each of the individual biodegradable polymer layers as preformed layers designed to be placed around the exterior dimensions of the orthopaedic device prior to implantation into a patient. In this alternative embodiment, osteoinductive formulations are applied as liquid formulations between the individual pre-formed polymer layers. Osteoinductive formulations are liberated in bioactive form from the multi-layer polymeric wrap based on the degradation rates of the individual biodegradable exterior polymer layers of the multi-layer polymeric wrap *in vivo.*

The present invention also relates to vectors containing the osteoinductive polynucleotides of the present invention, host cells, and the production of osteoinductive polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged *in vitro* using an appropriate packaging cell line and then transduced into host cells. Useful vectors include, but are not limited to, plasmids, bacteriophage, insect and animal cell vectors, retroviruses, cosmids, and other single and double-stranded viruses.

The polynucleotide insert should be operatively linked to an appropriate promoter, such as the phage lambda PL promoter, the *E. coli* lac, trp, phoA and tac promoters, the SV40 early and late promoters and promoters of retroviral LTRs, to name a few. Other suitable promoters will be known to the skilled artisan. The expression constructs will further contain sites for transcription initiation, termination; origin of replication sequence, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

The expression construct may further contain sequences such as enhancer sequences, efficient RNA processing signals such as splicing and polyadenylation signals, sequences that enhance translation efficiency, and sequences that enhance protein secretion.

Expression systems and methods of producing osteoinductive agents, such as recombinant proteins or protein fragments, are well known in the art. For example, methods of producing recombinant proteins or fragments thereof using bacterial, insect or mammalian expression systems are well known in the art. (*See,* e.g., Molecular Biotechnology: Principles and Applications of Recombinant DNA, B. R. Glick and J. Pasternak, and M. M. Bendig, Genetic Engineering, 7, pp. 91-127 (1988), for a discussion of recombinant protein production).

The expression vectors will preferably include at least one selectable marker. Such markers include dihydrofolate reductase, G418 or neomycin

## Claims

1. A multi-layer polymeric wrap designed to cover an orthopaedic device, the multi-layer polymeric wrap comprising at least two biodegradable polymer sheets wherein the at least two biodegradable polymer sheets are associated with one another, and wherein the multi-layer polymeric wrap further comprises at least one osteoinductive formulation; wherein the at least one osteoinductive formulation either is contained within at least one of the biodegradable polymer sheets or is contained between at least two of the biodegradable polymer sheets.

2. The multi-layer polymeric wrap of claim 1, wherein the osteoinductive formulation further comprises one or more osteoinductive agents.

3. The multi-layer polymeric wrap of claim 2, wherein the one or more osteoinductive agents comprise BMP-2 polynucleotides or polypeptides or mature fragments of same.

4. The multi-layer polymeric wrap of claim 2, wherein the one or more osteoinductive agents are selected from the group consisting of BMP-1, BMP-2, BMP-3, BMP-4, BMP-5, BMP-6, BMP-7, BMP-8, BMP-9, BMP-10, BMP-11, BMP-12, BMP-13, BMP-15, BMP-16, BMP-17, BMP-18, and any combination thereof, including full length BMPs or fragments thereof, and combinations thereof, either as polypeptides or polynucleotides encoding said polypeptide fragments of all of said BMPs.

5. The multi-layer polymeric wrap of claim 2, wherein the one or more osteoinductive agents are selected from the group consisting of CTGF-1, CTGF-2, CGTF-3, CTGF-4, and any combination thereof

6. The multi-layer polymeric wrap of claim 2, wherein the one or more osteoinductive agents are selected from the group consisting of VEGF-A, VEGF-B, VEGF-C, VEGF-D, VEGF-E, and any combination thereof.

7. The multi-layer polymeric wrap of claim 2, wherein the one or more osteoinductive agents are selected from osteoclastogenesis inhibitors.

8. The multi-layer polymeric wrap of claim 2, wherein the one or more osteoinductive agents is osteoprotegerin polynucleotides or polypeptides.

9. The multi-layer polymeric wrap of claim 2, wherein the one or more osteoinductive agents are selected from the group consisting of TGF-β-1, TGF-β-2, TGF-β-3, and any combination thereof.

10. The multi-layer polymeric wrap of claim 2, wherein the one or more osteoinductive agents are selected from polynucleotides and polypeptides promoting bone adhesion.

11. The multi-layer polymeric wrap of claim 2, wherein the one or more osteoinductive agents is selected from the group consisting of one or more BMPs, one or more VEGFs, one or more CTGFs, osteoprotegerin or other osteoclastogenesis inhibitors, periostin, one or more TGF-βs, and any combination thereof.

12. The multi-layer polymeric wrap of claim 2, wherein the one or more osteoinductive agents are provided as therapeutic polynucleotides.

13. The multi-layer polymeric wrap of claim 2, wherein the one or more osteoinductive agents are provided as therapeutic polypeptides.

14. The multi-layer polymeric wrap of claim 13, wherein the therapeutic polypeptides are administered as mature polypeptides.

15. The multi-layer polymeric wrap of claim 1, wherein the osteoinductive formulation comprises a sustained-release formulation.

16. The multi-layer polymeric wrap of claim 2, wherein the osteoinductive formulation further comprises one or more components selected from one or more antibiotics; one or more carriers; ; demineralized bone matrix; bone marrow aspirate; bone marrow concentrate; one or more immunosuppressives; one or more agents that enhance isotonicity and chemical stability; and any combination of one or more, including all, of the said components.

17. The multi-layer polymeric wrap of claim 16, wherein the carrier is collagen.

18. The multi-layer polymeric wrap of claim 16, wherein the carrier is collagen in combination with glycosaminoglycan.

19. The multi-layer polymeric wrap of claim 17, wherein the collagen is recombinantly produced collagen.

20. The multi-layer polymeric wrap of claim 1, wherein the wrap is applied to an orthopaedic device prior to, or concurrently with, implantation of the device.

21. The multi-layer polymeric wrap of claim 1, wherein the wrap is provided as a pre-formed composition that covers the exterior dimensions of an orthopaedic device.

22. An orthopaedic device covered with the multi-layer polymeric wrap of claim 1.

23. The multi-layer polymeric wrap of claim 1, wherein the at least two biodegradable polymer sheets each comprise at least one osteoinductive formulation comprising at least one osteoinductive agent.

24. The multi-layer polymeric wrap of claim 23, wherein the at least two biodegradable polymer sheets comprise at least one different osteoinductive agent(s) within the osteoinductive formulation.

25. The multi-layer polymeric wrap of claim 23, wherein the at least two biodegradable polymer sheets comprise at least one osteoinductive agent(s) at a different concentration within the osteoinductive formulation.

26. The multi-layer polymeric wrap of claim 1, wherein the layers are formulated to have different degradation characteristics.

27. The multi-layer polymeric wrap of claim 26, wherein the biodegradation resistance of the polymer is increased by introducing or increasing the percentage of biostable polymers contained within the layers.

28. The multi-layer polymeric wrap of claim 27, wherein the biostable polymers are selected from silicones, polyesters, vinyl homopolymers and copolymers, acrylate homopolymers and copolymers, polyethers, and cellulosics.

29. The multi-layer polymeric wrap of claim 1, wherein biodegradable sustained release polymers useful in the at least two biodegradable polymer sheets are selected from polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malic acid), poly(amino acids), polyvinylpyrrolidone, polyethylene glycol, polyhydroxycellulose, chitin, chitosan, poly(L-lactic acid), poly(lactide-co-glycolide), poly(hydroxybutyrate-co-valerate), and copolymers, terpolymers, or combinations or mixtures thereof.

## Patentansprüche

1. Mehrschichtige Polymerhülle, die zum Umhüllen einer orthopädischen Vorrichtung bestimmt ist, wobei die mehrschichtige Polymerhülle mindestens zwei biologisch abbaubare Polymerfolien umfasst, worin die mindestens zwei biologisch abbaubaren Polymerfolien miteinander assoziiert sind, und worin die mehrschichtige Polymerhülle ferner mindestens eine osteoinduktive Formulierung umfasst; worin die mindestens eine osteoinduktive Formulierung entweder in mindestens einer der biologisch abbaubaren Polymerfolien enthalten ist oder zwischen mindestens zwei biologisch abbaubaren Polymerfolien enthalten ist.

2. Mehrschichtige Polymerhülle nach Anspruch 1, worin die osteoinduktive Formulierung ferner einen oder mehr osteoinduktive(n) Wirkstoff(e) umfasst.

3. Mehrschichtige Polymerhülle nach Anspruch 2, worin der eine oder mehr osteoinduktive Wirkstoff(e) BMP-2-Polynukleotide oder -Polypeptide oder mature Fragmente von diesen umfasst/umfassen.

4. Mehrschichtige Polymerhülle nach Anspruch 2, worin der eine oder mehr osteoinduktive Wirkstoff(e) aus der Gruppe ausgewählt ist/sind, die aus BMP-1, BMP-2, BMP-3, BMP-4, BMP-5, BMP-6, BMP-7, BMP-8, BMP-9, BMP-10, BMP-11, BMP-12, BMP-13, BMP-15, BMP-16, BMP-17, BMP-18 und jedweder Kombination davon besteht, einschließlich BMPs der vollen Länge oder Fragmente davon, und Kombinationen davon, entweder als Polypeptide oder Polynukleotide, die für die Polypeptidfragmente von allen diesen BMP kodieren.

5. Mehrschichtige Polymerhülle nach Anspruch 2, worin der eine oder mehr osteoinduktive Wirkstoff(e) aus der Gruppe ausgewählt ist/sind, die aus CTGF-1, CTGF-2, CGTF-3, CTGF-4 und jedweder Kombination davon besteht.

6. Mehrschichtige Polymerhülle nach Anspruch 2, worin der eine oder mehr osteoinduktive Wirkstoff(e) aus der Gruppe ausgewählt ist/sind, die aus VEGF-A, VEGF-B, VEGF-C, VEGF-D, VEGF-E und jedweder Kombination davon besteht.

7. Mehrschichtige Polymerhülle nach Anspruch 2, worin der eine oder mehr osteoinduktive Wirkstoff(e) aus Osteoklastogenese-Inhibitoren ausgewählt ist/sind.

8. Mehrschichtige Polymerhülle nach Anspruch 2, worin der eine oder mehr osteoinduktive Wirkstoff(e) für Osteoprotegerin-Polynukleotide oder -Polypeptide steht/stehen.

9. Mehrschichtige Polymerhülle nach Anspruch 2, worin der eine oder mehr osteoinduktive Wirkstoff(e) aus der Gruppe ausgewählt ist/sind, die aus TGF-β-1, TGF-β-2, TGF-β-3 und jeder Kombination davon besteht.

10. Mehrschichtige Polymerhülle nach Anspruch 2, worin der eine oder mehr osteoinduktive Wirkstoff(e) aus Polynukleotiden und Polypeptiden ausgewählt ist/sind, welche die Knochenadhäsion fördern.

11. Mehrschichtige Polymerhülle nach Anspruch 2, worin der eine oder mehr osteoinduktive Wirkstoff(e) aus der Gruppe ausgewählt ist/sind, die aus einem oder mehr BMPs, einem oder mehr VEGFs, einem oder mehr CTGFs, Osteoprotegerin oder anderen Osteoklastogenese-Inhibitoren, Periostin, einem oder mehr TGF-β und jedweder Kombination davon besteht.

12. Mehrschichtige Polymerhülle nach Anspruch 2, worin der eine oder mehr osteoinduktive Wirkstoff(e) als therapeutische Polynukleotide bereitgestellt wird/werden.

13. Mehrschichtige Polymerhülle nach Anspruch 2, worin der eine oder mehr osteoinduktive Wirkstoff(e) als therapeutische Polypeptide bereitgestellt wird/werden.

14. Mehrschichtige Polymerhülle nach Anspruch 13, worin die therapeutischen Polypeptide als mature Polypeptide verabreicht werden.

15. Mehrschichtige Polymerhülle nach Anspruch 1, worin die osteoinduktive Formulierung eine Formulierung zur hinhaltenden Freisetzung umfasst.

16. Mehrschichtige Polymerhülle nach Anspruch 2, worin die osteoinduktive Formulierung ferner eine oder mehr Komponente(n) umfasst, die aus Folgendem ausgewählt ist/sind: einem Antibiotikum oder mehr Antibiotika; einem oder mehr Träger(n); einer demineralisierten Knochenmatrix; einem Knochenmarkaspirat; einem Knochemarkkonzentrat; einem Immunsuppressivum oder mehr Immunsuppressiva; einem oder mehr Wirkstoff(en), welche(r) die Isotonität und chemische Stabilität fördert/fördern; und jedwede Kombination von einer oder mehr, einschließlich aller dieser Komponenten.

17. Mehrschichtige Polymerhülle nach Anspruch 16, worin der Träger für Kollagen steht.

18. Mehrschichtige Polymerhülle nach Anspruch 16, worin der Träger Kollagen in Kombination mit Glycosaminoglykan darstellt.

19. Mehrschichtige Polymerhülle nach Anspruch 17, worin das Kollagen rekombinant produziertes Kollagen darstellt.

20. Mehrschichtige Polymerhülle nach Anspruch 1, worin die Hülle an einer orthopädischen Vorrichtung vor, oder gleichzeitig, mit der Implantation der Vorrichtung angewendet wird.

21. Mehrschichtige Polymerhülle nach Anspruch 1, worin die Hülle als eine vorgeformte Zusammensetzung bereitgestellt wird, welche die Außenabmessungen einer orthopädischen Vorrichtung umhüllt.

22. Orthopädische Vorrichtung, die mit der mehrschichtigen Polymerhülle nach Anspruch 1 umhüllt ist.

23. Mehrschichtige Polymerhülle nach Anspruch 1, worin die mindestens zwei biologisch abbaubaren Polymerfolien jeweils mindestens eine osteoinduktive Formulierung umfassen, die mindestens einen osteoinduktiven Wirkstoff umfasst.

24. Mehrschichtige Polymerhülle nach Anspruch 23, worin die mindestens zwei biologisch abbaubaren Polymerfolien mindestens (einen) unterschiedliche(n) osteoinduktive(n) Wirkstoff(e) in der osteoinduktiven Formulierung umfassen.

25. Mehrschichtige Polymerhülle nach Anspruch 23, worin die mindestens zwei biologisch abbaubaren Polymerfolien mindestens (einen) osteoinduktive(n) Wirkstoff(e) in einer unterschiedlichen Konzentration in der osteoinduktiven Formulierung umfassen.

26. Mehrschichtige Polymerhülle nach Anspruch 1, worin die Schichten dergestalt formuliert sind, dass sie unterschiedliche Abbaumerkmale aufweisen.

27. Mehrschichtige Polymerhülle nach Anspruch 26, worin die Resistenz des Polymers gegen einen biologischen Abbau durch Einführung oder Erhöhung des prozentualen Anteils von in den Schichten enthaltenen biostabilen Polymeren gesteigert wird.

28. Mehrschichtige Polymerhülle nach Anspruch 27, worin die biostabilen Polymere aus Silikonen, Polyestern, Vinylhomopolymeren und -copolymeren, Acrylathomopolymeren und -copolymeren, Polyethern und Celluloserzeugnissen ausgewählt sind.

29. Mehrschichtige Polymerhülle nach Anspruch 1, worin biologisch abbaubare Polymere mit hinhaltender Freisetzung, die in den mindestens zwei biologisch abbaubaren Polymerfolien nützlich sind, aus Folgendem ausgewählt sind: Polylactiden, Polyglykoliden, Polycaprolaktonen, Polyanhydriden, Polyamiden, Polyurethanen, Polyesteramiden, Polyorthoestern, Polydioxanonen, Polyacetalen, Polyketalen, Polycarbonaten, Polyorthocarbonaten, Polyphosphazenen, Polyhydroxybutyraten, Polyhydroxyvaleraten, Polyalkylenoxalaten, Polyalkylensuccinaten, Poly(malinsäure), Poly(aminosäuren), Polyvinylpyrrolidon, Polyethylenglykol, Polyhydroxycellulose, Chitin, Chitosan, Poly(L-milchsäure), Poly(lactid-co-glykolid), Poly(hydroxybutyrat-co-valerat) und Copolymeren, Terpolymeren oder Kombinationen oder Gemischen davon.

## Revendications

1. Enveloppe polymère multi-couches conçu pour recouvrir un dispositif orthopédique, l'enveloppe polymère multi-couches comprenant au moins deux feuilles polymères biodégradables, où les au moins deux feuilles polymères biodégradables sont associées l'une à l'autre, et où l'enveloppe polymère multi-couches comprend en outre au moins une formulation ostéo-inductrice ; où la au moins une formulation ostéo-inductrice est contenue au sein d'au moins l'une des feuilles polymères biodégradables ou bien est contenue entre au moins deux des feuilles polymères biodégradables.

2. Enveloppe polymère multi-couches selon la revendication 1, dans laquelle la formulation ostéo-inductrice comprend en outre un ou plusieurs agents ostéo-inducteurs.

3. Enveloppe polymère multi-couches selon la revendication 2, dans laquelle les un ou plusieurs agents ostéo-inducteurs comprennent des polynucléotides BMP-2 ou des polypeptides ou des fragments matures de ceux-ci.

4. Enveloppe polymère multi-couches selon la revendication 2, dans laquelle les un ou plusieurs agents ostéo-inducteurs sont choisis parmi le groupe constitué par BMP-1, BMP-2, BMP-3, BMP-4, BMP-5, BMP-6, BMP-7, BMP-8, BMP-9, BMP-10, BMP-11, BMP-12, BMP-13, BMP-15, BMP-16, BMP-17, BMP-18, et une association quelconque de ceux-ci, y compris des BMP pleine longueur ou des fragments de ceux-ci, et des associations de ceux-ci, comme polypeptides ou bien comme polynucléotides codant pour lesdits fragments polypeptidiques de l'ensemble desdits BMP.

5. Enveloppe polymère multi-couches selon la revendication 2, dans laquelle les un ou plusieurs agents ostéo-inducteurs sont choisis parmi le groupe constitué par CTGF-1, CTGF-2, CGTF-3, CTGF-4, et une combinaison quelconque de ceux-ci.

6. Enveloppe polymère multi-couches selon la revendication 2, dans laquelle les un ou plusieurs agents ostéo-inducteurs sont choisis parmi le groupe constitué par VEGF-A, VEGF-B, VEGF-C, VEGF-D, VEGF-E, et une combinaison quelconque de ceux-ci.

7. Enveloppe polymère multi-couches selon la revendication 2, dans laquelle les un ou plusieurs agents ostéo-inducteurs sont choisis parmi des inhibiteurs d'ostéoclastogenèse.

8. Enveloppe polymère multi-couches selon la revendication 2, dans laquelle les un ou plusieurs agents ostéo-inducteurs sont des polynucléotides ou des polypeptides d'ostéoprotégérine.

9. Enveloppe polymère multi-couches selon la revendication 2, dans laquelle les un ou plusieurs agents ostéo-inducteurs sont choisis parmi le groupe constitué par TGF-β-1, TGF-β-2, TGF-β-3, et une combinaison quelconque de ceux-ci.

10. Enveloppe polymère multi-couches selon la revendication 2, dans laquelle les un ou plusieurs agents ostéo-inducteurs sont choisis parmi des polynucléotides et des polypeptides favorisant l'adhésion osseuse.

11. Enveloppe polymère multi-couches selon la revendication 2, dans laquelle les un ou plusieurs agents ostéo-inducteurs sont choisis parmi le groupe constitué par un ou plusieurs BMP, un ou plusieurs VEGF, un ou plusieurs CTGF, l'ostéoprotégérine ou d'autres inhibiteurs d'ostéoclastogenèse, la périostine, un ou plusieurs TGF-β, et une combinaison quelconque de ceux-ci.

12. Enveloppe polymère multi-couches selon la revendication 2, dans laquelle les un ou plusieurs agents ostéo-inducteurs sont fournis sous forme de polynucléotides thérapeutiques.

13. Enveloppe polymère multi-couches selon la revendication 2, dans laquelle les un ou plusieurs agents ostéo-inducteurs sont fournis sous forme de polypeptides thérapeutiques.

14. Enveloppe polymère multi-couches selon la revendication 13, dans laquelle les polypeptides thérapeutiques sont administrés sous forme de polypeptides matures.

15. Enveloppe polymère multi-couches selon la revendication 1, dans laquelle la formulation ostéo-inductrice comprend une formulation à libération prolongée.

16. Enveloppe polymère multi-couches selon la revendication 2, dans laquelle la formulation ostéo-inductrice comprend en outre un ou plusieurs composants choisis parmi un ou plusieurs antibiotiques ; un ou plusieurs véhicules ; une matrice osseuse déminéralisée ; un aspirat de moelle osseuse ; un concentrat de moelle osseuse ; un ou plusieurs immunosuppresseurs ; un ou plusieurs agents qui améliorent l'isotonicité et la stabilité chimique ; et une association quelconque d'un ou plusieurs, y compris l'ensemble, desdits composants.

17. Enveloppe polymère multi-couches selon la revendication 16, dans laquelle le véhicule est le collagène.

18. Enveloppe polymère multi-couches selon la revendication 16, dans laquelle le véhicule est le collagène en association avec du glycosaminoglycane.

19. Enveloppe polymère multi-couches selon la revendication 17, dans laquelle le collagène est du collagène produit de manière recombinée.

20. Enveloppe polymère multi-couches selon la revendication 1, dans laquelle l'enveloppe est appliquée à un dispositif orthopédique préalablement à, ou conjointement avec, l'implantation du dispositif.

21. Enveloppe polymère multi-couches selon la revendication 1, dans laquelle l'enveloppe est fournie sous forme de composition préformée qui recouvre les dimensions extérieures d'un dispositif orthopédique.

22. Dispositif orthopédique recouvert de l'enveloppe polymère multi-couches selon la revendication 1.

23. Enveloppe polymère multi-couches selon la revendication 1, dans laquelle les au moins deux feuilles polymères biodégradables comprennent chacune au moins une formulation ostéo-inductrice comprenant au moins un agent ostéo-inducteur.

24. Enveloppe polymère multi-couches selon la revendication 23, dans laquelle les au moins deux feuilles polymères biodégradables comprennent au moins un ou plusieurs agents ostéo-inducteurs différents au sein de la formulation ostéo-inductrice.

25. Enveloppe polymère multi-couches selon la revendication 23, dans laquelle les au moins deux feuilles polymères biodégradables comprennent au moins un ou plusieurs agents ostéo-inducteurs en une concentration différente au sein de la formulation ostéo-inductrice.

26. Enveloppe polymère multi-couches selon la revendication 1, dans laquelle les couches sont formulées afin de posséder différentes caractéristiques de dégradation.

27. Enveloppe polymère multi-couches selon la revendication 26, dans laquelle la résistance à la biodégradation du polymère est augmentée par l'introduction ou l'augmentation du pourcentage de polymères biostables contenus au sein des couches.

28. Enveloppe polymère multi-couches selon la revendication 27, dans laquelle les polymères biostables sont choisis parmi les silicones, les polyesters, les homopolymères et copolymères vinyliques, les homopolymères et copolymères d'acrylate, les polyéthers et les dérivés cellulosiques.

29. Enveloppe polymère multi-couches selon la revendication 1, dans laquelle les polymères biodégradables à libération prolongée utiles dans les au moins deux feuilles polymères biodégradables sont choisis parmi les polylactides, les polyglycolides, les polycaprolactones, les polyanhydrides, les polyamides, les polyuréthanes, les polyestéramides, les polyorthoesters, les polydioxanones, les polyacétals, les polycétals, les polycarbonates, les polyorthocarbonates, les polyphosphazènes, les polyhydroxybutyrates, les polyhydroxyvalérates, les oxalates de polyalkylène, les succinates de polyalkylène, le poly(acide malique), les poly(acides aminés), la polyvinylpyrrolidone, le polyéthylène glycol, la polyhydroxycellulose, la chitine, le chitosane, le poly(acide L-lactique), le poly(lactide-co-glycolide), le poly(hydroxybutyrate-co-valérate), et les copolymères, terpolymères, ou associations ou mélanges de ceux-ci.
